# EUROPEAN PATENT SPECIFICATION

(11) **EP 4 444 269 B1**
(45) Date of publication and mention of the grant of the patent: **28.10.2026**
(21) Application number: 22826422.2
(22) Date of filing: 06.12.2022
(51) Int. Cl.: A61K 9/00, A61K 9/16, A61K 9/70, A61K 47/34, A61K 31/401

(54) **DANEGAPTIDE FORMULATION FOR APPLICATOIN IN THE EYE**
DIANGAPTID-FORMULIERUNG FÜR ANWENDUNGEN IM AUGE
FORMULATION DE DANEGAPTIDE POUR APPLICATION DANS L'OEIL

(30) Priority: 06.12.2021 US 202163286348 P
(43) Date of publication of application: 16.10.2024
(73) Proprietor: Breye Therapeutics ApS, 2930 Rungsted Kyst (DK)
(72) Inventor: MOURITZEN, Ulrik, 2960 Rungsted Kyst (DK)
(74) Representative: Gill Jennings & Every LLP
(86) International application number: PCT/IB2022/061849
(87) International publication number: WO 2023/105417

(56) References cited:
- WO-A1-2018/202865
- US-A- 4 135 514
- US-A1- 2011 034 448
- US-A1- 2016 177 298
- RIPAL GAUDANA ET AL: "Recent Perspectives in Ocular Drug Delivery", PHARMACEUTICAL RESEARCH, KLUWER ACADEMIC PUBLISHERS-PLENUM PUBLISHERS, NL, vol. 26, no. 5, 29 August 2008 (2008-08-29), pages 1197 - 1216, XP019686073, ISSN: 1573-904X
- ALMOSHARI YOSIF: "Osmotic Pump Drug Delivery Systems-A Comprehensive Review", PHARMACEUTICALS, vol. 15, no. 11, 18 November 2022 (2022-11-18), pages 1430, XP093018006, DOI: 10.3390/ph15111430
- KIM JURY ET AL: "The Evaluation of Osmotic Pump as Glaucoma Drug Delivery System in Normal Dogs", PAKISTAN VETERINARY JOURNAL, vol. 35, no. 2, 1 January 2015 (2015-01-01), PK, pages 239 - 241, XP093018009, ISSN: 0253-8318, Retrieved from the Internet <URL:http://www.pvj.com.pk/pdf-files/35_2/239-241.pdf> [retrieved on 20230126]

## Description

### Technical field

The present disclosure relates to a slow-release or extended-release formulation of danegaptide for sustained drug delivery.

### Background

In human physiology, intercellular communication is known to be essential for homeostasis, proliferation, differentiation and structural tissue integrity. Such communication is believed to be facilitated by gap junctions. These specialized cell-cell structures are a critical means for coupling cells and permitting "cross-talk" between cells. This cross-talk between gap junctions is referred to as "gap junctional intercellular communication" (GJIC). Generally, gap junctions are specialized regions of the cell membrane that contain clusters of hundreds to thousands of densely packed channels that directly connect the cytoplasm of two adjacent cells. The gap junction channels are composed of two hemichannels, or connexons, provided by each of two neighbouring cells. Each connexon, in turn, is made up of six proteins called connexins. These specialized transmembrane, gap junction proteins can interact with other cell-cell adhesion and cell-matrix adhesion complexes, and these gap junction proteins also are operative in intracellular communication and modulating cellular adhesions in specialized tissues, such as the blood-retinal barrier and specialized cellular structures in the eye.

For example, WO 2018/202865 A1 describes the use of gap junction modulators in the treatment or prevention of diabetic eye diseases, such as diabetic retinopathy and diabetic macular edema, by inhibiting both apoptosis of retinal endothelial cells and pericyte loss. Additionally, US 2016/0177298 A1 describes compositions comprising gap junction modulators and their use in treating ocular and other disorders. US 2011/0034448 is said to describe drug delivery devices which are suitable for administrating therapeutic agents to a subject.

### Summary

It can be advantageous to provide eye implantable, drug delivery systems, such as intraocular implants, and methods of using such systems that are capable of releasing a therapeutic agent, such as danegaptide at a sustained or controlled rate for extended periods of time and in therapeutic effective amounts with few or no negative side effects, in particular for treatment of diabetic retinopathy and diabetic macular edema along with possibly other pathological conditions in or disease of the eye.

*In vitro* and *in vivo* pre-clinical studies suggest that the optimal concentration of Danegaptide in the target tissue, such as the retina is 50 - 100 nM when the focus is to maintain Gap-Junction coupling under stressed conditions.

An example of the dose response curve for danegaptide to maintain retinal endothelial cell coupling *in vitro* is published in Kim et al. 2018. Data show that the optimal protection from high-glucose induced cell-cell uncoupling was achieved at concentrations of 50 - 100 nM danegaptide. The coupling of cells, such as homophilic adhesion between endothelial cells and heterophilic adhesion between endothelial cells and pericytes in the eye of humans and animals is known to be mediated in part via the Gap Junction protein, Connexin 43 (Cx43) and danegaptide has been shown in selected experimental models to maintain Cx43 dependent Gap Junctions despite cellular stress including high glucose induced stress and diabetic conditions.

Data generated in an extensive toxicology program show that there are no toxicity concerns when high concentration levels of danegaptide are administered either locally in selected tissues or systemically in humans and animal models. However, the protective effects of danegaptide from stress-induced cell-cell uncoupling or cellular loss may be diminished or lost at higher concentrations of danegaptide.

Another function of danegaptide appears to be to modulate closure of hemichannels (HC) and to keep HC closed under stressed conditions. The dose response for danegaptide to facilitate closure of HC is different than the dose response curve for maintaining Gap-Junctions and optimal therapeutic dose and local tissue concentration of danegaptide needed to be an effective treatment for patients with diabetic retinopathy and diabetic macular edema remains to be determined.

The present disclosure provides an intraocular implant comprising a compound of formula (I), or a pharmaceutically acceptable salt thereof, wherein the intraocular implant is configured to enable optimal cell protection - both ensuring maintenance of Gap-Junction couplings and closure of HC in selected conditions. This is done by maintaining an effective concentration of compound at 50 nM or more.

In a first aspect of the present disclosure, a biodegradable intraocular implant comprising a hydrophilic compound of formula (I) is provided, 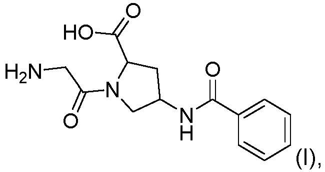 or a pharmaceutically acceptable salt thereof;
wherein the compound is associated with a slow-release or extended-release biodegradable polymer matrix;
wherein the biodegradable polymer matrix comprises a biodegradable polymer selected from the group consisting of a polylactic acid (PLA) polymer, a polyglycolic acid (PGA) polymer, polylactide-co-glycolide (PLGA) polymer, and copolymers thereof;
wherein the pharmaceutically acceptable salt is selected from the group consisting of: a hydrochloride salt, a maleic acid salt, an acetic acid salt, a tartaric acid salt, and a pamoic acid salt; and
wherein the biodegradable polymer matrix comprises a mixture of two or more biodegradable polymers comprising a first biodegradable polymer and a different second biodegradable polymer.

In some embodiments, the disclosure provides a biodegradable intraocular implant comprising:
a compound of formula (I), 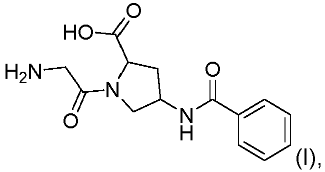
or a pharmaceutically acceptable salt thereof; and an extended-release or sustained release biodegradable polymer matrix. In one embodiment, the compound is:

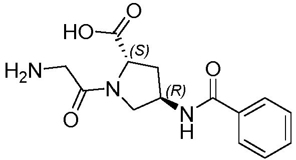

In one embodiment, the compound is dispersed within the biodegradable polymer matrix. In one embodiment, the matrix comprises a mixture of a first biodegradable polymer of polylactic acid, and a different second biodegradable polymer of polylactic acid. In one embodiment, the first biodegradable polymer is selected from the group consisting of polylactic acid, polyglycolic acid, polylactide-co-glycolide, and copolymers thereof. In one embodiment, the first biodegradable polymer has an inherent viscosity of from about 0.1 dl/g to about 0.7 dl/g. In one embodiment, the first biodegradable polymer has an inherent viscosity of from about 0.7 dl/g to about 1 dl/g. In one embodiment, the second biodegradable polymer has an inherent viscosity of from about 0.1 dl/g to about 0.7 dl/g. In one embodiment, the second biodegradable polymer has an inherent viscosity of from about 0.7 dl/g to about 1 dl/g. In one embodiment, the first biodegradable polymer and the second biodegradable polymer have substantially similar inherent viscosities. In one embodiment, the inherent viscosity of the first biodegradable polymer is higher than the inherent viscosity of the second biodegradable polymer. In one embodiment, the inherent viscosity of the first biodegradable polymer is lower than the inherent viscosity of the second biodegradable polymer. In one embodiment, the matrix comprises at least one PLGA polymer selected from the group consisting of: Poly(D,L-lactide-co-glycolide) lactide:glycolide (50:50) with an average molecular weight (avg. MW) of 30,000-60,000 g/mol; Poly(D,L-lactide-co-glycolide) lactide:glycolide 50:50 with avg. MW of 45,000 g/mol; Poly(D,L-lactide-co-glycolide) lactide:glycolide 50:50, acid and hydroxy terminated with avg. MW of 25,000 g/mol; Poly(D,L-lactide-co-glycolide) lactide:glycolide (50:50), ester terminated with avg. MW of 100,000 g/mol; Poly(D,L-Lactide-co-Glycolide) lactide:glycolide 50:50, ester terminated with avg. MW of 7,000-17,000 g/mol; Poly(D,L-lactide-co-glycolide) acid terminated with avg. MW of 7,000-17,000 g/mol; Poly(D,L-lactide-co-glycolide) lactide:glycolide 50:50, ester terminated with avg. MW of 24,000-38,000 g/mol; Poly(D,L-lactide-co-glycolide) acid terminated, lactide:glycolide 50:50 with avg. MW of 24,000-38,000 g/mol; Poly(D,L-lactide-co-glycolide) lactide:glycolide 50:50, ester terminated with avg. MW of 38,000-54,000 g/mol; Poly(D,L-lactide-co-glycolide) acid terminated, lactide:glycolide 50:50 with avg. MW of 38,000-54,000 g/mol; and Poly(D,L-lactide-co-glycolide) ester terminated with avg. MW of 54,000-69,000 g/mol. In one embodiment, the matrix comprises at least one PLGA polymer selected from the group consisting of: Poly(D,L-lactide-co-glycolide) lactide:glycolide 65:35 with avg. MW of 40,000-75,000 g/mol; Poly(L-lactide-co-glycolide) lactide:glycolide 65:35, viscosity 0.6 dL/g; and Poly(D,L-lactide-co-glycolide) acid terminated with avg. MW of 24,000-38,000 g/mol. In one embodiment, the matrix comprises at least one PLGA polymer selected from the group consisting of: Poly(D,L-lactide-co-glycolide) lactide:glycolide (75:25) with avg. MW of 66,000-107,000 g/mol; Poly(D,L-lactide-co-glycolide) acid terminated lactide:glycolide 75:25 with avg. MW of 6,000-10,000 g/mol; Poly(D,L-lactide-co-glycolide) ester terminated, lactide:glycolide 75:25; Poly(D,L-lactide-co-glycolide) ester terminated, lactide:glycolide 75:25 with avg. MW of 4,000-15,000 g/mol; Poly(D,L-lactide-co-glycolide) acid terminated, lactide:glycolide 75:25 with avg. MW of 4,000-15,000 g/mol; Poly(D,L-lactide-co-glycolide) ester terminated, Lactide: Glycolide 75:25 with avg. MW of 66,000 - 107,000 g/mol; Poly(D,L-lactide-co-glycolide) acid terminated (RG 753 H) with avg. MW of 66,000 -107,000 g/mol; Poly(D,L-lactide-co-glycolide) ester terminated (RG 755 S); Poly(D,L-lactide-co-glycolide) ester terminated, lactide:glycolide 75:25 with avg. MW of 76,000-115,000 g/mol; and Poly(D,L-lactide-co-glycolide) ester terminated, Lactide: Glycolide 75:25 (RG 757 S). In one embodiment, the matrix comprises at least one PLGA polymer selected from the group consisting of: Poly(D,L-lactide-co-glycolide) ester terminated with avg. MW of 50,000-75,000 g/mol; Poly(D,L-lactide-co-glycolide)-COOH, lactide:glycolide 85:15 with avg. MW of 17,000 g/mol; Poly(D,L-lactide-co-glycolide) ester terminated lactide:glycolide 80:20 with avg. MW of 200,000; Poly(L-lactide-co-glycolide) ester terminated, lactide:glycolide 85:15 (LG 855 S); Poly(L-lactide-co-glycolide) ester terminated, lactide:glycolide 85:15 (LG 857 S); and Poly(D,L-lactide-co-glycolide) ester terminated, lactide:glycolide 85:15 (RG 858 S) with avg. MW of 190,000-240,000 g/mol. In one embodiment, the matrix comprises at least one PLA polymer selected from the group consisting of: Poly(D,L-lactide), Acid end group (R 202 H); Poly(D,L-lactide), Ester end group (R 202 S); Poly(D,L-lactide), Acid end group (R 203 H); Poly(D,L-lactide), Ester end group (R 203 S); and Poly(D,L-lactide), Ester end group (R 205 H). In one embodiment, the implant comprises from about 2% to about 35 w/w% of the compound. In one embodiment, the implant comprises from about 2 to about 25 w/w% of the compound, and the biodegradable polymer matrix comprises a combination of two different polylactide polymers. In one embodiment, the combination of two different polylactide polymers comprises two different PLGAs, two different PLAs, or a PLGA and a PLA. In one embodiment, the matrix comprises a mixture of biodegradable polymers, and at least one of the biodegradable polymers is a polylactide having a molecular weight of from about 6 kDa to about 120 kDa. In one embodiment, the matrix comprises a second polymer of a polylactide having a molecular weight of from about 6 kDa to about 82 kDa. In one embodiment, the implant comprises from about 1 µg to about 50 µg of the compound or a pharmaceutically acceptable salt thereof. In one embodiment, the implant comprises from about 50 µg to about 75 µg of the compound or a pharmaceutically acceptable salt thereof. In one embodiment, the implant comprises from about 75 µg to about 150 µg of the compound or a pharmaceutically acceptable salt thereof. In one embodiment, the implant comprises from about 5 µg to about 30 µg of the compound or a pharmaceutically acceptable salt thereof. In one embodiment, the implant comprises about 5 µg, about 6 µg, about 7 µg, about 8 µg, about 9 µg, about 10 µg, about 11 µg, or about 12 µg of the compound or a pharmaceutically acceptable salt thereof. In one embodiment, the implant comprises about 13 µg, about 14 µg, about 15 µg, about 16 µg, about 17 µg, about 18 µg, about 19 µg, or about 20 µg of the compound or a pharmaceutically acceptable salt thereof. In one embodiment, the implant comprises about 21 µg, about 22 µg, about 23 µg, or about 24 µg of the compound or a pharmaceutically acceptable salt thereof. In one embodiment, the implant comprises about 25 µg, about 26 µg, about 27 µg, about 28 µg, about 29 µg, about 30 µg, about 31 µg, about 32 µg, about 33 µg, about 34 µg, or about 35 µg of the compound or a pharmaceutically acceptable salt thereof. In one embodiment, the implant comprises about 36 µg, about 37 µg, about 38 µg, about 39 µg, about 40 µg, about 41 µg, about 42 µg, about 43 µg, about 44 µg, or about 45 µg of the compound or a pharmaceutically acceptable salt thereof. In one embodiment, the implant is configured to be placed in the vitreous of the eye or other posterior segments of the eye. In one embodiment, the compound is released at a rate effective to sustain release of the compound for more than one week from the time the implant is placed in the vitreous of the eye. In one embodiment, the compound is released at a rate effective to sustain release of the compound for more than one month from the time the implant is placed in the vitreous of the eye or other posterior segments of the eye. In one embodiment, upon placement in the vitreous of the eye or other posterior segments of the eye, the implant maintains a concentration of at least 50 nM of the compound in the retinal tissue. In one embodiment, upon placement in the vitreous of the eye or other posterior segments of the eye, the implant maintains a concentration of from 50 nM to 125 nM of the compound in the retinal tissue once a steady state equilibrium has been reached. In one embodiment, upon placement in the vitreous of the eye or other posterior segments of the eye, the implant is configured to release from about 0.1 µg per day to about 2 µg per day of the compound or a pharmaceutically acceptable salt thereof. In one embodiment, upon placement in the vitreous of the eye or other posterior segments of the eye, the implant is configured to provide an initial burst release of the compound or a pharmaceutically acceptable salt thereof followed by a lower steady state concentration of the compound or a pharmaceutically acceptable salt thereof. In one embodiment, the initial burst release provides a concentration of from about 10 to 50 ng/mL of the compound or a pharmaceutically acceptable salt thereof in the ocular compartment. In one embodiment, the lower steady state concentration of the compound or a pharmaceutically acceptable salt thereof is from about 50 nM to about 125 nM steady state concentration. In one embodiment, the initial burst release provides a dose of from about 150 to about 300 ng of the compound or a pharmaceutically acceptable salt thereof over two days from the time the implant is placed in the vitreous of the eye. In one embodiment, upon placement the vitreous of the eye or other posterior segments of the eye, the implant is configured to release the compound or a pharmaceutically acceptable salt thereof for at a period of time of least one month to three months. In one embodiment, the implant comprises between about 11% and 29% w/w of the compound or a pharmaceutically acceptable salt thereof, between 0% and about 7.5% w/w of a PEG polymer, between about 15% and about 25% w/w of R203S or a substantially similar polymer, between about 10% and about 20% w/w of R202H or a substantially similar polymer, and between about 35% and about 45% of RG752S or a substantially similar polymer. In one embodiment, the implant comprises between about 11% and about 29% w/w of the compound or a pharmaceutically acceptable salt thereof, between 0% and about 7.5% w/w of a PEG polymer, between about 25% and about 35% w/w of R202S or a substantially similar polymer, and between about 45% and about 55% of RG752S or a substantially similar polymer. In one embodiment, the implant consists of between about 11% and about 29% w/w of the compound or a pharmaceutically acceptable salt thereof, between 0% and about 7.5% w/w of PEG3350 or a substantially similar polymer, between about 15% and about 25% w/w of R203S or a substantially similar polymer, between about 10% and about 20% w/w of R202H or a substantially similar polymer, and between about 35% and about 45% of RG752S or a substantially similar polymer. In one embodiment, the implant consists of about 15% w/w of the compound or a pharmaceutically acceptable salt thereof, between 0% and about 7.5% w/w of PEG3350 or a substantially similar polymer, between about 15% and about 25% w/w of R203S or a substantially similar polymer, between about 10% and about 20% w/w of R202H or a substantially similar polymer, and between about 35% and about 45% of RG752S or a substantially similar polymer. In one embodiment, the implant consists of about 16% w/w of the compound or a pharmaceutically acceptable salt thereof, between 0% and about 7.5% w/w of PEG3350 or a substantially similar polymer, between about 15% and about 25% w/w of R203S or a substantially similar polymer, between about 10% and about 20% w/w of R202H or a substantially similar polymer, and between about 35% and about 45% of RG752S or a substantially similar polymer. In one embodiment, the implant consists of about 17% w/w of the compound or a pharmaceutically acceptable salt thereof, between 0% and about 7.5% w/w of PEG3350 or a substantially similar polymer, between about 15% and about 25% w/w of R203S or a substantially similar polymer, between about 10% and about 20% w/w of R202H or a substantially similar polymer, and between about 35% and about 45% of RG752S or a substantially similar polymer. In one embodiment, the implant consists of about 18% w/w of the compound or a pharmaceutically acceptable salt thereof, between 0% and about 7.5% w/w of PEG3350 or a substantially similar polymer, between about 15% and about 25% w/w of R203S or a substantially similar polymer, between about 10% and about 20% w/w of R202H or a substantially similar polymer, and between about 35% and about 45% of RG752S or a substantially similar polymer. In one embodiment, the implant consists of about 19% w/w of the compound or a pharmaceutically acceptable salt thereof, between 0% and about 7.5% w/w of PEG3350 or a substantially similar polymer, between about 15% and about 25% w/w of R203S or a substantially similar polymer, between about 10% and about 20% w/w of R202H or a substantially similar polymer, and between about 35% and about 45% of RG752S or a substantially similar polymer. In one embodiment, the implant consists of about 20% w/w of the compound or a pharmaceutically acceptable salt thereof, between 0% and about 7.5% w/w of PEG3350 or a substantially similar polymer, between about 15% and about 25% w/w of R203S or a substantially similar polymer, between about 10% and about 20% w/w of R202H or a substantially similar polymer, and between about 35% and about 45% of RG752S or a substantially similar polymer. In one embodiment, the implant consists of about 21% w/w of the compound or a pharmaceutically acceptable salt thereof, between 0% and about 7.5% w/w of PEG3350 or a substantially similar polymer, between about 15% and about 25% w/w of R203S or a substantially similar polymer, between about 10% and about 20% w/w of R202H or a substantially similar polymer, and between about 35% and about 45% of RG752S or a substantially similar polymer. In one embodiment, the implant consists of about 22% w/w of the compound or a pharmaceutically acceptable salt thereof, between 0% and about 7.5% w/w of PEG3350 or a substantially similar polymer, between about 15% and about 25% w/w of R203S or a substantially similar polymer, between about 10% and about 20% w/w of R202H or a substantially similar polymer, and between about 35% and about 45% of RG752S or a substantially similar polymer. In one embodiment, the implant consists of about 23% w/w of the compound or a pharmaceutically acceptable salt thereof, between 0% and about 7.5% w/w of PEG3350 or a substantially similar polymer, between about 15% and about 25% w/w of R203S or a substantially similar polymer, between about 10% and about 20% w/w of R202H or a substantially similar polymer, and between about 35% and about 45% of RG752S or a substantially similar polymer. In one embodiment, the implant consists of about 24% w/w of the compound or a pharmaceutically acceptable salt thereof, between 0% and about 7.5% w/w of PEG3350 or a substantially similar polymer, between about 15% and about 25% w/w of R203S or a substantially similar polymer, between about 10% and about 20% w/w of R202H or a substantially similar polymer, and between about 35% and about 45% of RG752S or a substantially similar polymer. In one embodiment, the implant consists of about 25% w/w of the compound or a pharmaceutically acceptable salt thereof, between 0% and about 7.5% w/w of PEG3350 or a substantially similar polymer, between about 15% and about 25% w/w of R203S or a substantially similar polymer, between about 10% and about 20% w/w of R202H or a substantially similar polymer, and between about 35% and about 45% of RG752S or a substantially similar polymer. In one embodiment, the implant consists of between about 11% and about 29% w/w of the compound or a pharmaceutically acceptable salt thereof, between about 0% and about 7.5% w/w of PEG3350 or a substantially similar polymer, between about 25% and about 35% w/w of R202S or a substantially similar polymer, and between about 45% and about 55% of RG752S or a substantially similar polymer. In one embodiment, the implant consists of about 15% w/w of the compound or a pharmaceutically acceptable salt thereof, between about 0% and about 7.5% w/w of PEG3350 or a substantially similar polymer, between about 25% and about 35% w/w of R202S or a substantially similar polymer, and between about 45% and about 55% of RG752S or a substantially similar polymer. In one embodiment, the implant consists of about 16% w/w of the compound or a pharmaceutically acceptable salt thereof, between about 0% and about 7.5% w/w of PEG3350 or a substantially similar polymer, between about 25% and about 35% w/w of R202S or a substantially similar polymer, and between about 45% and about 55% of RG752S or a substantially similar polymer. In one embodiment, the implant consists of about 17% w/w of the compound or a pharmaceutically acceptable salt thereof, between about 0% and about 7.5% w/w of PEG3350 or a substantially similar polymer, between about 25% and about 35% w/w of R202S or a substantially similar polymer, and between about 45% and about 55% of RG752S or a substantially similar polymer. In one embodiment, the implant consists of about 18% w/w of the compound or a pharmaceutically acceptable salt thereof, between about 0% and about 7.5% w/w of PEG3350 or a substantially similar polymer, between about 25% and about 35% w/w of R202S or a substantially similar polymer, and between about 45% and about 55% of RG752S or a substantially similar polymer. In one embodiment, the implant consists of about 19% w/w of the compound or a pharmaceutically acceptable salt thereof, between about 0% and about 7.5% w/w of PEG3350 or a substantially similar polymer, between about 25% and about 35% w/w of R202S or a substantially similar polymer, and between about 45% and about 55% of RG752S or a substantially similar polymer. In one embodiment, the implant consists of about 20% w/w of the compound or a pharmaceutically acceptable salt thereof, between about 0% and about 7.5% w/w of PEG3350 or a substantially similar polymer, between about 25% and about 35% w/w of R202S or a substantially similar polymer, and between about 45% and about 55% of RG752S or a substantially similar polymer. In one embodiment, the implant consists of about 21% w/w of the compound or a pharmaceutically acceptable salt thereof, between about 0% and about 7.5% w/w of PEG3350 or a substantially similar polymer, between about 25% and about 35% w/w of R202S or a substantially similar polymer, and between about 45% and about 55% of RG752S or a substantially similar polymer. In one embodiment, the implant consists of about 22% w/w of the compound or a pharmaceutically acceptable salt thereof, between about 0% and about 7.5% w/w of PEG3350 or a substantially similar polymer, between about 25% and about 35% w/w of R202S or a substantially similar polymer, and between about 45% and about 55% of RG752S or a substantially similar polymer. In one embodiment, the implant consists of about 23% w/w of the compound or a pharmaceutically acceptable salt thereof, between about 0% and about 7.5% w/w of PEG3350 or a substantially similar polymer, between about 25% and about 35% w/w of R202S or a substantially similar polymer, and between about 45% and about 55% of RG752S or a substantially similar polymer. In one embodiment, the implant consists of about 24% w/w of the compound or a pharmaceutically acceptable salt thereof, between about 0% and about 7.5% w/w of PEG3350 or a substantially similar polymer, between about 25% and about 35% w/w of R202S or a substantially similar polymer, and between about 45% and about 55% of RG752S or a substantially similar polymer. In one embodiment, the implant consists of about 25% w/w of the compound or a pharmaceutically acceptable salt thereof, between about 0% and about 7.5% w/w of PEG3350 or a substantially similar polymer, between about 25% and about 35% w/w of R202S or a substantially similar polymer, and between about 45% and about 55% of RG752S or a substantially similar polymer. The disclosure also provides a method of treating diabetic retinopathy in a subject in need thereof, comprising administering to the subject a biodegradable intraocular implant described herein.

### Brief Description of the Figures

**FIG. 1A****-** **FIG. 1C** are photographs of the extruded rods of formulation 1 at 14 days, 42 days, and 83 days.
**FIG. 2** is a graph illustrating the dissolution of formulation 1 over time.
**FIG. 3A****-** **FIG. 3C** are photographs of the extruded rods of formulation 2 at 14 days, 42 days, and 83 days.
**FIG. 4** is a graph illustrating the dissolution of formulation 2 over time.
**FIG. 5A****-** **FIG. 5C** are photographs of the extruded rods of formulation 3 at 14 days, 42 days, and 83 days.
**FIG. 6** is a graph illustrating the dissolution of formulation 3 over time.

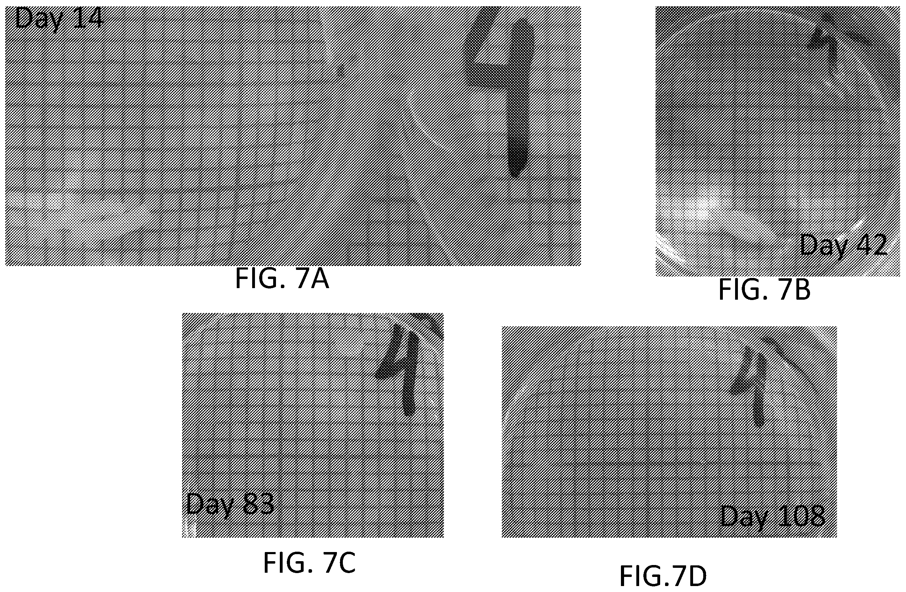

**FIG. 7A****-** **FIG. 7C** are photographs of the extruded rods of formulation 4 at 14 days, 42 days, 83 days, and 108 days.
**FIG. 8** is a graph illustrating the dissolution of formulation 4 over time.

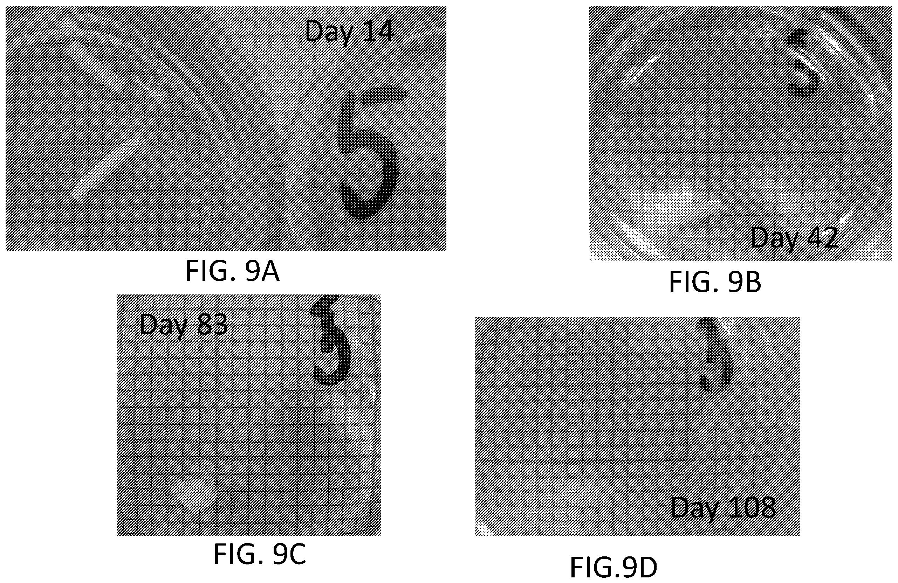

**FIG. 9A****-** **FIG. 9C** are photographs of the extruded rods of formulation 5 at 14 days, 42 days, 83 days, and 108 days.
**FIG. 10** is a graph illustrating the dissolution of formulation 5 over time.

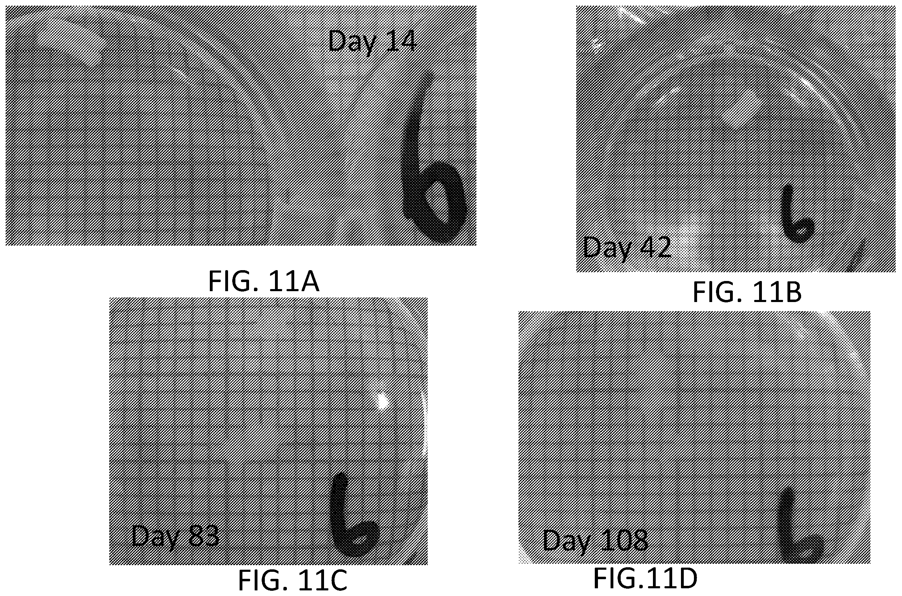

**FIG. 11A****-** **FIG. 11C** are photographs of the extruded rods of formulation 6 at 14 days, 42 days, 83 days, and 108 days.
**FIG. 12** is a graph illustrating the dissolution of formulation 6 over time.
**FIG. 13** is a graph illustrating the dissolution of formulations 1- 6 over time.

### Detailed description

Diabetic retinopathy is the leading cause of vision loss and blindness among working- age adults. Retinal vascular cell loss and vascular leakage are characteristic hallmarks of early diabetic retinopathy. It is known that accelerated retinal vascular cell loss is, at least in part, due to high glucose-induced cellular uncoupling and apoptosis. A gap junction modulator, Danegaptide (2S,4R)-1 -(2-aminoacetyl)-4-benzoylamino- pyrrolidine-2-carboxylic acid (J. Med. Chem. 52(4): 908-91 1, 2009), has been shown to prevent cell-cell GJ uncoupling under high glucose condition, to prevent high glucose-induced apoptosis in retinal vascular cells, and to reduce vascular leakage.

Biocompatible implants for placement in the eye have been disclosed in a number of patents, such as U. S. Pat. Nos. 4,521,210; 4,853,224; 4,997,652; 5,164,188; 5,443,505; 5,501,856; 5,766,242; 5,824,072; 5,869,079; 6,074,661; 6,331,313; 6,369,116; and 6,699,493. The function of such biocompatible implants can be modified depending on several factors to provide an optimal pharmacological effect including a beneficial and targeted pharmacokinetic profile of a drug associated with such biocompatible implants. Extended release of a therapeutic agent through the use of one or more intraocular implants may improve treatment of undesirable ocular conditions. The implants comprise a pharmaceutically acceptable polymeric composition and are formulated to release one or more pharmaceutically active agents, such as danegaptide, over an extended period of time. The implants are effective to provide a therapeutically effective dosage of the agent or agents directly to a region or segment of the eye to treat or prevent one or more undesirable ocular conditions. Thus, with a single administration, a relevant concentration of a therapeutic agent will be made available at the site where they are needed and will be maintained for an extended period of time, rather than subjecting the patient to repeated eye injections or, in the case of self-administered drops, ineffective treatment with only limited bursts of exposure to the active agent or agents.

As such, an intraocular implant in accordance with the disclosure herein comprises a therapeutic component and a drug release sustaining component associated with the therapeutic component. In accordance with the present disclosure, the therapeutic component or compound comprises and/or consists essentially of danegaptide or a pharmaceutically acceptable salt, based or thereof.

### Definitions

For the purposes of this description, the following terms as defined in this section are used, unless the context of the word indicates a different meaning.

As used herein, an "intraocular implant" refers to a device, element or compound that is structured, sized, or otherwise configured to be placed in an eye. Implant can include microspheres and rods like structures. "Implantation" includes "injection". Intraocular implants are designed to be and generally found to be biocompatible with physiological conditions of an eye, and do not cause significant adverse side effects. Intraocular implants may be placed in an eye without disrupting vision of the eye. Intraocular implants have been developed which can release drug loads or pharmaceutical agents over various time periods. These implants, which when inserted into an eye, such as the vitreous of an eye, provide therapeutic levels of a compound as defined herein for extended periods of time (e. g., for about 1 week or more).

As used herein, "associated with" means mixed with, dispersed within, coupled to, covering, or surrounding.

As used herein, an "ocular region" or "ocular site" refers generally to any area of the eyeball, including the anterior and posterior segment of the eye, and which generally includes, but is not limited to, any functional (e. g., for vision) or structural tissues found in the eyeball, or tissues or cellular layers that partly or completely line the interior or exterior of the eyeball. Specific examples of areas of the eyeball in an ocular region include the anterior chamber, the posterior chamber, the vitreous cavity, the choroid, the suprachoroidal space, the conjunctiva, the subconjunctival space, the episcleral space, the intracorneal space, the epicorneal space, the sclera, the pars plana, surgically induced avascular regions, the macula, and the retina.

The term "biodegradable polymer" refers to a polymer or polymers which degrade *in vivo,* and wherein erosion of the polymer or polymers over time occurs concurrent with or subsequent to release of a therapeutic agent. Specifically, hydrogels such as methylcellulose which act to release drug through polymer swelling are specifically excluded from the term "biodegradable polymer". The terms "biodegradable" and "bioerodible" are equivalent and are used interchangeably herein. A biodegradable polymer may be a homopolymer, a copolymer, or a polymer comprising more than two different polymeric units. The term "pharmaceutically acceptable acid salts" of the compound of the present disclosure are those formed from acids which form non-toxic addition salts containing pharmaceutically acceptable anions, such as the hydrochloride, hydrobromide, pamoate, hydroiodide, sulfate, or bisulfate, phosphate or acid phosphate, acetate, maleate, fumarate, oxalate, lactate, pamoate, tartrate, citrate, gluconate, saccharate and p-toluene sulphonate salts.

The term "steady state concentration" as used herein refers to an equilibrium level or concentration of danegaptide obtained at the end of a certain number of administrations (e.g. 1 to about 5). Steady state is achieved when the concentration or level of the drug will remain substantially constant if the dose and the frequency of administrations remain substantially constant.

When ranges are used herein to describe, for example, physical or chemical properties such as molecular weight or chemical formulae, or amounts or concentrations, all combinations and subcombinations of ranges and specific embodiments therein are intended to be included. Use of the term "about" when referring to a number or a numerical range means that the number or numerical range referred to is an approximation within experimental variability (or within statistical experimental error), and thus the number or numerical range may vary. The variation is typically from 0% to 15%, or from 0% to 10%, or from 0% to 5% of the stated number or numerical range. The term "including" (and related terms such as "comprise" or "comprises" or "having" or "including") includes those embodiments such as, for example, an embodiment of any composition of matter, method or process that "consist of" or "consist essentially of' the described features.

### Danegaptide

In one embodiment, the implant is provided with a compound of formula (I), 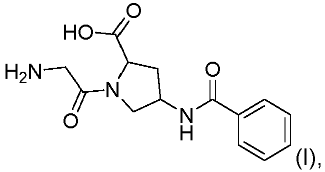 or a pharmaceutically acceptable salt thereof.

In particular embodiments, the compound is danegaptide, 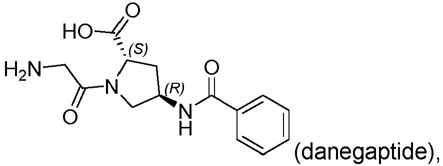 or a pharmaceutically acceptable salt thereof.

In one embodiment, the implant is provided, wherein the pharmaceutically acceptable salt is selected from the group consisting of: a hydrochloride salt, a maleic acid salt, an acetic acid salt, a tartaric acid salt, and a pamoic acid salt.

The pharmaceutical compound may be in a particulate or powder form and entrapped by the biodegradable polymer matrix. Usually, compound particles will have an effective average size less than 3000 nanometres. In certain implants, the particles may have an effective average particle size an order of magnitude smaller than 3000 nanometres. For example, the particles may have an effective average particle size of less than 500 nanometres. In additional implants, the particles may have an effective average particle size of less than 400 nanometres, and in still further embodiments, a size less than 200 nanometres.

The compound of the implant is preferably from about 1% to 90% by weight of the implant. More preferably, the compound is from about 2% to about 30% by weight of the implant. In a preferred embodiment, the compound comprises about 3% by weight of the implant. In another embodiment, the compound comprises about 12% by weight of the implant.

In one embodiment, the implant is provided, wherein the implant comprises from 5 µg to 800 µg of the compound provided herein, such as from 5 to 20 µg, such as from 20 to 35 µg, such as from 35 to 50 µg, such as from 50 to 65 µg, such as from 65 to 80 µg, such as from 80 to 95 µg, such as from 95 to 110 µg, such as from 110 to 125 µg, such as from 125 to 140 µg, such as from 140 to 155 µg, such as from 155 to 170 µg, such as from 170 to 185 µg, such as from 185 to 200 µg, such as from 200 to 215 µg, such as from 215 to 230 µg, such as from 230 to 245 µg, such as from 245 to 260 µg, such as from 260 to 275 µg, such as from 275 to 290 µg, such as from 290 to 305 µg, such as from 305 to 320 µg, such as from 320 to 335 µg, such as from 335 to 350 µg, such as from 350 to 365 µg, such as from 365 to 380 µg, such as from 380 to 395 µg, such as from 395 to 410 µg, such as from 410 to 425 µg, such as from 425 to 440 µg, such as from 440 to 455 µg, such as from 455 to 470 µg, such as from 470 to 485 µg, such as from 485 to 500 µg, such as from 500 to 515 µg, such as from 515 to 530 µg, such as from 530 to 545 µg, such as from 545 to 560 µg, such as from 560 to 575 µg, such as from 575 to 590 µg, such as from 590 to 605 µg, such as from 605 to 620 µg, such as from 620 to 635 µg, such as from 635 to 650 µg, such as from 650 to 665 µg, such as from 665 to 680 µg, such as from 680 to 695 µg, such as from 695 to 710 µg, such as from 710 to 725 µg, such as from 725 to 740 µg, such as from 740 to 755 µg, such as from 755 to 770 µg, such as from 770 to 785 µg, such as from 785 to 800 µg. In one embodiment, the implant is provided, wherein the implant comprises from about 5 µg to about 800 µg of the compound provided herein, such as from about 5 to about 20 µg, such as from about 20 to about 35 µg, such as from about 35 to about 50 µg, such as from about 50 to about 65 µg, such as from about 65 to about 80 µg, such as from about 80 to about 95 µg, such as from about 95 to about 110 µg, such as from about 110 to about 125 µg, such as from about 125 to about 140 µg, such as from about 140 to about 155 µg, such as from about 155 to about 170 µg, such as from about 170 to about 185 µg, such as from about 185 to about 200 µg, such as from about 200 to about 215 µg, such as from about 215 to about 230 µg, such as from about 230 to about 245 µg, such as from about 245 to about 260 µg, such as from about 260 to about 275 µg, such as from about 275 to about 290 µg, such as from about 290 to about 305 µg, such as from about 305 to about 320 µg, such as from about 320 to about 335 µg, such as from about 335 to about 350 µg, such as from about 350 to about 365 µg, such as from about 365 to about 380 µg, such as from about 380 to about 395 µg, such as from about 395 to about 410 µg, such as from about 410 to about 425 µg, such as from about 425 to about 440 µg, such as from about 440 to about 455 µg, such as from about 455 to about 470 µg, such as from about 470 to about 485 µg, such as from about 485 to about 500 µg, such as from about 500 to about 515 µg, such as from about 515 to about 530 µg, such as from about 530 to about 545 µg, such as from about 545 to about 560 µg, such as from about 560 to about 575 µg, such as from about 575 to about 590 µg, such as from about 590 to about 605 µg, such as from about 605 to about 620 µg, such as from about 620 to about 635 µg, such as from about 635 to about 650 µg, such as from about 650 to about 665 µg, such as from about 665 to about 680 µg, such as from about 680 to about 695 µg, such as from about 695 to about 710 µg, such as from about 710 to about 725 µg, such as from about 725 to about 740 µg, such as from about 740 to about 755 µg, such as from about 755 to about 770 µg, such as from about 770 to about 785 µg, such as from about 785 to about 800 µg.

In one embodiment, the implant is provided, wherein the implant comprises from 4 µg to 50 µg of the compound provided herein, such as from 4 to 5 µg, such as from 5 to 6 µg, such as from 6 to 7 µg, such as from 7 to 8 µg, such as from 8 to 9 µg, such as from 9 to 10 µg, such as from 10 to 11 µg, such as from 11 to 12 µg, such as from 12 to 13 µg, such as from 13 to 14 µg, such as from 14 to 15 µg, such as from 15 to 16 µg, such as from 16 to 17 µg, such as from 17 to 18 µg, such as from 18 to 19 µg, such as from 19 to 20 µg, such as from 20 to 21 µg, such as from 21 to 22 µg, such as from 22 to 23 µg, such as from 23 to 24 µg, such as from 24 to 25 µg, such as from 25 to 26 µg, such as from 26 to 28 µg, such as from 28 to 29 µg, such as from 29 to 30 µg, such as from 30 to 31 µg, such as from 31 to 32 µg, such as from 32 to 33 µg, such as from 33 to 34 µg, such as from 34 to 35 µg, such as from 35 to 36 µg, such as from 36 to 37 µg, such as from 37 to 38 µg, such as from 38 to 39 µg, such as from 39 to 40 µg, such as from 40 to 41 µg, such as from 41 to 42 µg, such as from 42 to 43 µg, such as from 43 to 44 µg, such as from 44 to 45 µg, such as from 45 to 46 µg, such as from 46 to 47 µg, such as from 47 to 48 µg, such as from 48 to 49 µg, such as from 49 to 50 µg.

In one embodiment, the implant is provided, wherein the implant comprises from 4 µg to 41 µg of the compound provided herein, such as 5 µg, such as from 4 to 6 µg, such as from 4.5 to 5.5 µg, such as from 4.6 to 5.4 µg, such as from 4.7 to 5.3 µg, such as from 4.8 to 5.2 µg, such as from 4.9 to 5.1 µg, such as 6 µg, such as from 5 to 7 µg, such as from 5.5 to 6.5 µg, such as from 5.6 to 6.4 µg, such as from 5.7 to 6.3 µg, such as from 5.8 to 6.2 µg, such as from 5.9 to 6.1 µg, such as 7 µg, such as from 6 to 8 µg, such as from 6.5 to 7.5 µg, such as from 6.6 to 7.4 µg, such as from 6.7 to 7.3 µg, such as from 6.8 to 7.2 µg, such as from 6.9 to 7.1 µg, such as 8 µg, such as from 7 to 9 µg, such as from 7.5 to 8.5 µg, such as from 7.6 to 8.4 µg, such as from 7.7 to 8.3 µg, such as from 7.8 to 8.2 µg, such as from 7.9 to 8.1 µg, such as 9 µg, such as from 8 to 10 µg, such as from 8.5 to 9.5 µg, such as from 8.6 to 9.4 µg, such as from 8.7 to 9.3 µg, such as from 8.8 to 9.2 µg, such as from 8.9 to 9.1 µg, such as 10 µg, such as from 9 to 11 µg, such as from 9.5 to 10.5 µg, such as from 9.6 to 10.4 µg, such as from 9.7 to 10.3 µg, such as from 9.8 to 10.2 µg, such as from 9.9 to 10.1 µg, such as 11 µg, such as from 10 to 12 µg, such as from 10.5 to 11.5 µg, such as from 10.6 to 11.4 µg, such as from 10.7 to 11.3 µg, such as from 10.8 to 11.2 µg, such as from 10.9 to 11.1 µg, such as 12 µg, such as from 11 to 13 µg, such as from 11.5 to 12.5 µg, such as from 11.6 to 12.4 µg, such as from 11.7 to 12.3 µg, such as from 11.8 to 12.2 µg, such as from 11.9 to 12.1 µg, such as 13 µg, such as from 12 to 14 µg, such as from 12.5 to 13.5 µg, such as from 12.6 to 13.4 µg, such as from 12.7 to 13.3 µg, such as from 12.8 to 13.2 µg, such as from 12.9 to 13.1 µg, such as 14 µg, such as from 13 to 15 µg, such as from 13.5 to 14.5 µg, such as from 13.6 to 14.4 µg, such as from 13.7 to 14.3 µg, such as from 13.8 to 14.2 µg, such as from 13.9 to 14.1 µg, such as 15 µg, such as from 14 to 16 µg, such as from 14.5 to 15.5 µg, such as from 14.6 to 15.4 µg, such as from 14.7 to 15.3 µg, such as from 14.8 to 15.2 µg, such as from 14.9 to 15.1 µg, such as 16 µg, such as from 15 to 17 µg, such as from 15.5 to 16.5 µg, such as from 15.6 to 16.4 µg, such as from 15.7 to 16.3 µg, such as from 15.8 to 16.2 µg, such as from 15.9 to 16.1 µg, such as 17 µg, such as from 16 to 18 µg, such as from 16.5 to 17.5 µg, such as from 16.6 to 17.4 µg, such as from 16.7 to 17.3 µg, such as from 16.8 to 17.2 µg, such as from 16.9 to 17.1 µg, such as 18 µg, such as from 17 to 19 µg, such as from 17.5 to 18.5 µg, such as from 17.6 to 18.4 µg, such as from 17.7 to 18.3 µg, such as from 17.8 to 18.2 µg, such as from 17.9 to 18.1 µg, such as 19 µg, such as from 18 to 20 µg, such as from 18.5 to 19.5 µg, such as from 18.6 to 19.4 µg, such as from 18.7 to 19.3 µg, such as from 18.8 to 19.2 µg, such as from 18.9 to 19.1 µg, such as 20 µg, such as from 19 to 21 µg, such as from 19.5 to 20.5 µg, such as from 19.6 to 20.4 µg, such as from 19.7 to 20.3 µg, such as from 19.8 to 20.2 µg, such as from 19.9 to 20.1 µg, such as 21 µg, such as from 20 to 22 µg, such as from 20.5 to 21.5 µg, such as from 20.6 to 21.4 µg, such as from 20.7 to 21.3 µg, such as from 20.8 to 21.2 µg, such as from 20.9 to 21.1 µg, such as 22 µg, such as from 21 to 23 µg, such as from 21.5 to 22.5 µg, such as from 21.6 to 22.4 µg, such as from 21.7 to 22.3 µg, such as from 21.8 to 22.2 µg, such as from 21.9 to 22.1 µg, such as 23 µg, such as from 22 to 24 µg, such as from 22.5 to 23.5 µg, such as from 22.6 to 23.4 µg, such as from 22.7 to 23.3 µg, such as from 22.8 to 23.2 µg, such as from 22.9 to 23.1 µg, such as 24 µg, such as from 23 to 25 µg, such as from 23.5 to 24.5 µg, such as from 23.6 to 24.4 µg, such as from 23.7 to 24.3 µg, such as from 23.8 to 24.2 µg, such as from 23.9 to 24.1 µg, such as 25 µg, such as from 24 to 26 µg, such as from 24.5 to 25.5 µg, such as from 24.6 to 25.4 µg, such as from 24.7 to 25.3 µg, such as from 24.8 to 25.2 µg, such as from 24.9 to 25.1 µg, such as 26 µg, such as from 25 to 27 µg, such as from 25.5 to 26.5 µg, such as from 25.6 to 26.4 µg, such as from 25.7 to 26.3 µg, such as from 25.8 to 26.2 µg, such as from 25.9 to 26.1 µg, such as 27 µg, such as from 26 to 28 µg, such as from 26.5 to 27.5 µg, such as from 26.6 to 27.4 µg, such as from 26.7 to 27.3 µg, such as from 26.8 to 27.2 µg, such as from 26.9 to 27.1 µg, such as 28 µg, such as from 27 to 29 µg, such as from 28.5 to 29.5 µg, such as from 28.6 to 29.4 µg, such as from 28.7 to 29.3 µg, such as from 28.8 to 29.2 µg, such as from 28.9 to 29.1 µg, such as 29 µg, such as from 28 to 30 µg, such as from 28.5 to 29.5 µg, such as from 28.6 to 29.4 µg, such as from 28.7 to 29.3 µg, such as from 28.8 to 29.2 µg, such as from 28.9 to 29.1 µg, such as 30 µg, such as from 29 to 31 µg, such as from 29.5 to 30.5 µg, such as from 29.6 to 30.4 µg, such as from 29.7 to 30.3 µg, such as from 29.8 to 30.2 µg, such as from 29.9 to 30.1 µg, such as 31 µg, such as from 30 to 32 µg, such as from 30.5 to 31.5 µg, such as from 30.6 to 31.4 µg, such as from 30.7 to 31.3 µg, such as from 30.8 to 31.2 µg, such as from 30.9 to 31.1 µg, such as 32 µg, such as from 31 to 33 µg, such as from 31.5 to 32.5 µg, such as from 31.6 to 32.4 µg, such as from 31.7 to 32.3 µg, such as from 31.8 to 32.2 µg, such as from 31.9 to 32.1 µg, such as 33 µg, such as from 32 to 34 µg, such as from 32.5 to 33.5 µg, such as from 32.6 to 33.4 µg, such as from 32.7 to 33.3 µg, such as from 32.8 to 33.2 µg, such as from 32.9 to 33.1 µg, such as 34 µg, such as from 33 to 35 µg, such as from 33.5 to 34.5 µg, such as from 33.6 to 34.4 µg, such as from 33.7 to 34.3 µg, such as from 33.8 to 34.2 µg, such as from 33.9 to 34.1 µg, such as 35 µg, such as from 34 to 36 µg, such as from 34.5 to 35.5 µg, such as from 34.6 to 35.4 µg, such as from 34.7 to 35.3 µg, such as from 34.8 to 35.2 µg, such as from 34.9 to 35.1 µg, such as 36 µg, such as from 35 to 37 µg, such as from 35.5 to 36.5 µg, such as from 35.6 to 36.4 µg, such as from 35.7 to 36.3 µg, such as from 35.8 to 36.2 µg, such as from 35.9 to 36.1 µg, such as 37 µg, such as from 36 to 38 µg, such as from 36.5 to 37.5 µg, such as from 36.6 to 37.4 µg, such as from 36.7 to 37.3 µg, such as from 36.8 to 37.2 µg, such as from 36.9 to 37.1 µg, such as 38 µg, such as from 37 to 39 µg, such as from 37.5 to 38.5 µg, such as from 37.6 to 38.4 µg, such as from 37.7 to 38.3 µg, such as from 37.8 to 38.2 µg, such as from 37.9 to 38.1 µg, such as 39 µg, such as from 38 to 40 µg, such as from 38.5 to 39.5 µg, such as from 38.6 to 39.4 µg, such as from 38.7 to 39.3 µg, such as from 38.8 to 39.2 µg, such as from 38.9 to 39.1 µg, such as 40 µg, such as from 39 to 41 µg, such as from 39.5 to 40.5 µg, such as from 39.6 to 40.4 µg, such as from 39.7 to 40.3 µg, such as from 39.8 to 40.2 µg, such as from 39.9 to 40.1 µg.

In one embodiment, the implant is provided, wherein the implant comprises 8 µg of the compound provided herein. In one embodiment, the implant is provided, wherein the implant comprises 32 µg of the compound provided herein.

In one embodiment, the implant is provided, wherein the implant comprises about 8 µg of the compound provided herein. In one embodiment, the implant is provided, wherein the implant comprises about 32 µg of the compound provided herein.

### Biodegradable polymer matrix

Suitable polymeric materials or compositions for use in the implant include those materials which are compatible that is biocompatible, with the eye so as to cause no substantial interference with the functioning or physiology of the eye. Such materials preferably are at least partially and more preferably completely biodegradable or bioerodible.

Examples of useful polymeric materials include such materials derived from and/or including organic esters and organic ethers, which when degraded result in physiologically acceptable degradation products, including the monomers. Also, polymeric materials derived from and/or including, anhydrides, amides, orthoesters and the like, by themselves or in combination with other monomers, may also find use. The polymeric materials may be addition or condensation polymers, advantageously condensation polymers. The polymeric materials may be cross-linked or non-cross-linked, for example not more than lightly cross-linked, such as less than 5%, or less than 1 % of the polymeric material being cross-linked. For the most part, besides carbon and hydrogen, the polymers will include at least one of oxygen and nitrogen, advantageously oxygen.

The oxygen may be present as oxy, e. g. hydroxy or ether, carbonyl, e. g. non-oxocarbonyl, such as carboxylic acid ester, and the like. The nitrogen may be present as amide, cyano and amino. The polymers set forth in Heller, Biodegradable Polymers in Controlled Drug Delivery, In: CRC Critical Reviews in Therapeutic Drug Carrier Systems, Vol. 1, CRC Press, Boca Raton, FL 1987, pp 39-90, which describes encapsulation for controlled drug delivery, may find use in the present implants.

In one embodiment, the implant is provided, wherein the compound is dispersed within the biodegradable polymer matrix.

In one embodiment, the implant is provided, wherein the matrix comprises a polylactic acid (PLA) polymer, a polyglycolic acid (PGA) polymer, polylactide-co-glycolide (PLGA) polymer, or copolymers thereof.

In one embodiment, the implant is provided, wherein the matrix comprises a mixture of a first biodegradable polymer of polylactic acid, and a different second biodegradable polymer of polylactic acid.

In one embodiment, the implant is provided, wherein the first biodegradable polymer is selected from the group consisting of polylactic acid, polyglycolic acid, polylactide-co-glycolide, and copolymers thereof

In one embodiment, the implant is provided, wherein the second biodegradable polymer is selected from the group consisting of decafluorobutane, poly(isobutylene), poly(hexemethylene adipamide), poly propylene, polyethylene and polyethylene glycol.

In one embodiment, the implant comprises polymers of hydroxyaliphatic carboxylic acids, either homopolymers or copolymers, and polysaccharides. In one embodiment, the implant comprises polymers of D-lactic acid, L-lactic acid, racemic lactic acid, glycolic acid, polycaprolactone, and combinations thereof.

Generally, by employing polymers of L-lactate or D-lactate, a slowly eroding polymer or polymeric material is achieved, while erosion is substantially enhanced with polymers of lactate racemate.

Among the useful polysaccharides are, without limitation, calcium alginate, and functionalized celluloses, particularly carboxymethylcellulose esters characterized by being water insoluble, a molecular weight of from about 5 kDa to about 500 kDa, for example.

Other polymers of interest include, without limitation, polyvinyl alcohol, polyesters, polyethers, polyethylene glycols and combinations thereof which are biocompatible and may be biodegradable and/or bioerodible.

### Preferred effects and extended release

Some preferred characteristics of the polymers or polymeric materials for use in the implant disclosed herein may include biocompatibility, compatibility with the compound of formula (I), ease of use of the polymer in making the extended-release intraocular implant, a half-life in the physiological environment of at least about 6 hours, preferably greater than about one day, not significantly increasing the viscosity of the vitreous, and water insolubility.

In particular, the implant provides effective treatment and reversal of diabetic retinopathy associated lesions or other pathologies in the eye following administration of the implant every month or less frequently.

In a preferred embodiment, the implant provides effective treatment and reversal of lesions or other pathologies in the eye when administered every 3 to 6 months. In some embodiments, the implant provides effective treatment and reversal of lesions or other pathologies in the eye when administered about every month. In some embodiments, the implant provides effective treatment and reversal of lesions or other pathologies in the eye when administered about every 2 months. In some embodiments, the implant provides effective treatment and reversal of lesions or other pathologies in the eye when administered about every 3 months. In some embodiments, the implant provides effective treatment and reversal of lesions or other pathologies in the eye when administered about every 4 months. In some embodiments, the implant provides effective treatment and reversal of lesions or other pathologies in the eye when administered about every 5 months. In some embodiments, the implant provides effective treatment and reversal of lesions or other pathologies in the eye when administered about every 6 months. In some embodiments, the implant provides effective treatment and reversal of lesions or other pathologies in the eye when administered about every 7 months. In some embodiments, the implant provides effective treatment and reversal of lesions or other pathologies in the eye when administered about every 8 months. In some embodiments, the implant provides effective treatment and reversal of lesions or other pathologies in the eye when administered about every 9 months. In some embodiments, the implant provides effective treatment and reversal of lesions or other pathologies in the eye when administered about every 10 months. In some embodiments, the implant provides effective treatment and reversal of lesions or other pathologies in the eye when administered about every 11 months. In some embodiments, the implant provides effective treatment and reversal of lesions or other pathologies in the eye when administered about every 12 months.

The biodegradable polymeric materials which are included to form the matrix are desirably subject to enzymatic or hydrolytic instability. Water soluble polymers may be cross-linked with hydrolytic or biodegradable unstable cross-links to provide useful water insoluble polymers. The degree of stability can be varied widely, depending upon the choice of monomer, whether a homopolymer or copolymer is employed, employing mixtures of polymers, and whether the polymer includes terminal acid groups.

The biodegradation of the polymer and hence the extended-release profile of the implant may be controlled by the relative average molecular weight of the polymeric composition employed in the implant. Different molecular weights of the same or different polymeric compositions may be included in the implant to modulate the release profile. In certain implants, the relative average molecular weight of the polymer will range from about 9 to about 64 kDa, usually from about 10 to about 54 kDa, and more usually from about 12 to about 45 kDa.

In one embodiment, the implant is provided, wherein the matrix comprises a mixture of biodegradable polymers, at least one of the biodegradable polymers is a polylactide having a molecular weight of from 6 to 120 kDa, such as from 6 to 10 kDa, such as from 10 to 14 kDa, such as from 14 to 18 kDa, such as from 18 to 22 kDa, such as from 22 to 26 kDa, such as from 26 to 30 kDa, such as from 30 to 34 kDa, such as from 34 to 38 kDa, such as from 38 to 42 kDa, such as from 42 to 46 kDa, such as from 46 to 50 kDa, such as from 50 to 54 kDa, such as from 54 to 58 kDa, such as from 58 to 62 kDa, such as from 62 to 66 kDa, such as from 66 to 70 kDa, such as from 70 to 74 kDa, such as from 74 to 78 kDa, such as from 78 to 82 kDa, such as from 82 to 86 kDa, such as from 86 to 100 kDa, such as from 100 to 104 kDa, such as from 104 to 108 kDa, such as from 108 to 112 kDa, such as from 112 to 116 kDa, such as from 116 to 112 kDa, for example from 62 to 66 kDa, for example 64 kDa.

In one embodiment, the implant is provided, wherein the matrix comprises a second polymer of a polylactide having a molecular weight of from 6 to 82 kDa, such as from 6 to 10 kDa, such as from 10 to 14 kDa, such as from 14 to 18 kDa, such as from 18 to 22 kDa, such as from 22 to 26 kDa, such as from 26 to 30 kDa, such as from 30 to 34 kDa, such as from 34 to 38 kDa, such as from 38 to 42 kDa, such as from 42 to 46 kDa, such as from 46 to 50 kDa, such as from 50 to 54 kDa, such as from 54 to 58 kDa, such as from 58 to 62 kDa, such as from 62 to 66 kDa, such as from 66 to 70 kDa, such as from 70 to 74 kDa, such as from 74 to 78 kDa, such as from 78 to 82 kDa.

The biodegradation of PLGA and hence the extended-release profile of the implant may be controlled by using mixture of ester terminated 50/50 PLGA copolymer and acid terminated 50/50 PLGA copolymer.

In some implants, copolymers of glycolic acid and lactic acid are used, where the rate of biodegradation is controlled by the ratio of glycolic acid to lactic acid. The most rapidly degraded copolymer has roughly equal amounts of glycolic acid and lactic acid. Homopolymers, or copolymers having ratios other than equal, are more resistant to degradation. The ratio of glycolic acid to lactic acid will also affect the brittleness of the implant, where a more flexible implant is desirable for larger geometries. The % of polylactic acid in the polylactic acid polyglycolic acid (PLGA) copolymer can be 0-100%, preferably about 15-85%, more preferably about 35-65%. In some implants of the present disclosure, a 50/50 PLGA copolymer is used.

The biodegradable polymer matrix of the intraocular implant may comprise a mixture of two or more biodegradable polymers. For example, the implant may comprise a mixture of a first biodegradable polymer and a different second biodegradable polymer. One or more of the biodegradable polymers may have terminal acid groups.

Release of a drug from an erodible polymer is the consequence of several mechanisms or combinations of mechanisms. Some of these mechanisms include desorption from the implants surface, dissolution, diffusion through porous channels of the hydrated polymer and erosion. Erosion can be bulk or surface or combination of both. As discussed herein, the matrix of the intraocular implant may release the compound at a rate effective to sustain release and maintain an amount of the compound for more than one week after implantation into an eye. In certain implants, therapeutic amounts of compound are released for no more than about 30-35 days after implantation. For example, an implant may comprise danegaptide, and the matrix of the implant degrades at a rate effective to sustain release of a therapeutically effective amount of danegaptide for about one month or more after being placed in an eye. As another example, the implant may comprise danegaptide, and the matrix releases drug at a rate effective to sustain release and maintain a therapeutically effective amount of danegaptide for more than forty days, such as for about six months.

In one embodiment, the implant is provided, wherein the matrix releases the compound at a rate effective to sustain release of the compound for more than one week from the time the implant is placed in the vitreous of the eye. In one embodiment, the implant is provided, wherein the matrix is configured to be placed in the vitreous of the eye.

In one embodiment, the implant is provided, wherein the matrix releases the compound at a rate effective to sustain release of the compound for more than one month from the time the implant is placed in the vitreous of the eye.

One example of the biodegradable intraocular implant comprises a compound of formula (I) associated with a biodegradable polymer matrix, which comprises a mixture of different biodegradable polymers. At least one of the biodegradable polymers is a polylactide having a molecular weight of 63.3 kDa.

A second biodegradable polymer is a polylactide having a molecular weight of 14 kDa. Such a mixture is effective in sustaining release of a therapeutically effective amount of the compound for a time period greater than about one month from the time the implant is placed in an eye.

Another example of a biodegradable intraocular implant comprises a compound as disclosed herein associated with a biodegradable polymer matrix, which comprises a mixture of different biodegradable polymers, each biodegradable polymer having an inherent viscosity from 0.16 dl/g to 1.0 dl/g. For example, one of the biodegradable polymers may have an inherent viscosity of 0.3 dl/g. A second biodegradable polymer may have an inherent viscosity of about 1.0 dl/g. The inherent viscosities identified above may be determined in 0.1 % chloroform at 25 °C.

In one embodiment, the implant is provided, wherein the first biodegradable polymer has an inherent viscosity of from 0.2 dl/g to 1.0 dl/g, such as from 0.2 to 0.3 dl/g, such as from 0.3 to 0.4 dl/g, such as from 0.4 to 0.5 dl/g, such as from 0.5 to 0.6 dl/g, such as from 0.6 to 0.7 dl/g, such as from 0.7 to 0.8 dl/g, such as from 0.8 to 0.9 dl/g, such as from 0.9 to 1.0 dl/g, for example about 0.3 dl/g. In one embodiment, the implant is provided, wherein the first biodegradable polymer has an inherent viscosity of from about 0.1 dl/g to about 0.2 dl/g, from about 0.2 dl/g to about 0.3 dl/g, from about 0.3 dl/g to about 0.4 dl/g, from about 0.4 dl/g to about 0.5 dl/g, from about 0.5 dl/g to about 0.6 dl/g, from about 0.6 dl/g to about 0.7 dl/g, from about 0.7 dl/g to about 0.8 dl/g, from about 0.8 dl/g to about 0.9 dl/g, from about 0.9 dl/g to about 1.0 dl/g, or from about 1.0 dl/g to about 1.1 dl/g In some embodiments, the implant is provided, wherein the first biodegradable polymer has an inherent viscosity of about 0.1 dl/g, about 0.2 dl/g, about 0.3 dl/g, about 0.4 dl/g, about 0.5 dl/g, about 0.6 dl/g, about 0.7 dl/g, about 0.8 dl/g, about 0.9 dl/g, about 1.0 dl/g, or about 1.1 dl/g. In one embodiment, the implant is provided, wherein the first biodegradable polymer has a molecular weight of from about 10 kDa to about 18 kDa, or from about 18 kDa to about 24 kDa. In some embodiments, the implant is provided, wherein the first biodegradable polymer has a molecular weight of about 5 kDa, about 6 kDa, about 7 kDa, about 8 kDa, about 9 kDa, about 10 kDa, about 11 kDa, about 12 kDa, about 13 kDa, about 14 kDa, about 15 kDa, about 16 kDa, about 17 kDa, about 18 kDa, about 19 kDa, about 20 kDa, about 21 kDa, about 22 kDa, about 23 kDa, about 24 kDa, about 25 kDa, about 26 kDa, about 27 kDa, about 28 kDa, about 29 kDa, about 30 kDa, about 31 kDa, about 32 kDa, about 33 kDa, about 34 kDa, or about 35 kDa. In one embodiment, the implant is provided, wherein the first biodegradable polymer is acid terminated. In one embodiment, the implant is provided, wherein the first biodegradable polymer is ester terminated.

In one embodiment, the implant is provided, wherein the second biodegradable polymer has an inherent viscosity of from 0.6 dl/g to 6.0 dl/g, such as from 0.6 to 0.8 dl/g, such as from 0.8 to 1.0 dl/g, such as from 1.0 to 1.2 dl/g, such as from 1.2 to 1.4 dl/g, such as from 1.4 to 1.6 dl/g, such as from 1.6 to 1.8 dl/g, such as from 1.8 to 2.0 dl/g, such as from 2.0 to 2.2 dl/g, such as from 2.2 to 2.4 dl/g, such as from 2.4 to 2.6 dl/g, such as from 2.6 to 2.8 dl/g, such as from 2.8 to 3.0 dl/g, such as from 3.0 to 3.2 dl/g, such as from 3.2 to 3.4 dl/g, such as from 3.4 to 3.6 dl/g, such as from 3.6 to 3.8 dl/g, such as from 3.8 to 4.0 dl/g, such as from 4.0 to 4.2 dl/g, such as from 4.2 to 4.4 dl/g, such as from 4.4 to 4.6 dl/g, such as from 4.6 to 4.8 dl/g, such as from 4.8 to 5.0 dl/g, such as from 5.0 to 5.2 dl/g, such as from 5.2 to 5.4 dl/g, such as from 5.4 to 5.6 dl/g, such as from 5.6 to 5.8 dl/g, such as from 5.8 to 6.0 dl/g, for example about 1.0 dl/g. In one embodiment, the implant is provided, wherein the second biodegradable polymer has an inherent viscosity of from about 0.1 dl/g to about 0.2 dl/g, from about 0.2 dl/g to about 0.3 dl/g, from about 0.3 dl/g to about 0.4 dl/g, from about 0.4 dl/g to about 0.5 dl/g, from about 0.5 dl/g to about 0.6 dl/g, from about 0.6 dl/g to about 0.7 dl/g, from about 0.7 dl/g to about 0.8 dl/g, from about 0.8 dl/g to about 0.9 dl/g, from about 0.9 dl/g to about 1.0 dl/g, or from about 1.0 dl/g to about 1.1 dl/g In some embodiments, the implant is provided, wherein the second biodegradable polymer has an inherent viscosity of about 0.1 dl/g, about 0.2 dl/g, about 0.3 dl/g, about 0.4 dl/g, about 0.5 dl/g, about 0.6 dl/g, about 0.7 dl/g, about 0.8 dl/g, about 0.9 dl/g, about 1.0 dl/g, or about 1.1 dl/g. In one embodiment, the implant is provided, wherein the second biodegradable polymer has a molecular weight of from about 4 kDa to about 15 kDa, from about 7 kDa to about 17 kDa, from about 17 kDa to about 24 kDa, from about 24 kDa to about 38 kDa, from about 38 kDa to about 54 kDa, from about 54 kDa to about 69 kDa. In some embodiments, the implant is provided, wherein the second biodegradable polymer has a molecular weight of about 5 kD, about 6 kDa, about 7 kDa, about 8 kDa, about 9 kDa, about 10 kDa, about 11 kDa, about 12 kDa, about 13 kDa, about 14 kDa, about 15 kDa, about 16 kDa, about 17 kDa, about 18 kDa, about 19 kDa, about 20 kDa, about 21 kDa, about 22 kDa, about 23 kDa, about 24 kDa, about 25 kDa, about 26 kDa, about 27 kDa, about 28 kDa, about 29 kDa, about 30 kDa, about 31 kDa, about 32 kDa, about 33 kDa, about 34 kDa, about 35 kDa, about 36 kDa, about 37 kDa, about 38 kDa, about 39 kDa, about 40 kDa, about 41 kDa, about 42 kDa, about 43 kDa, about 44 kDa, about 45 kDa, about 46 kDa, about 47 kDa, about 48 kDa, about 49 kDa, about 50 kDa, about 51 kDa, about 52 kDa, about 53 kDa, about 54 kDa, about 55 kDa, about 56 kDa, about 57 kDa, about 58 kDa, about 59 kDa, about 60 kDa, about 61 kDa, about 62 kDa, about 63 kDa, about 64 kDa, about 65 kDa, about 66 kDa, about 67 kDa, about 68 kDa, about 69 kDa, about 70 kDa, about 71 kDa, about 72 kDa, about 73 kDa, about 74 kDa, or about 75 kDa. In one embodiment, the implant is provided, wherein the second biodegradable polymer is acid terminated. In one embodiment, the implant is provided, wherein the second biodegradable polymer is ester terminated.

In one embodiment, the implant comprises the compound associated with a combination of two different polylactide polymers. The compound is present in about 20% by weight of the implant. One polylactide polymer has a molecular weight of about 14 kDa and an inherent viscosity of about 0.3 dl/g, and the other polylactide polymer has a molecular weight of about 63.3 kDa and an inherent viscosity of about 1.0 dl/g. The two polylactide polymers are present in the implant in a 1:1 ratio. Such an implant provides for release of the compound for more than two months *in vitro,* as described herein. The implant is provided in the form of a rod or a filament produced by an extrusion process. In one embodiment, the implant is provided, wherein the compound is present in about 1% by weight of the implant, about 2% by weight of the implant, about 3% by weight of the implant, about 4% by weight of the implant, about 5% by weight of the implant, about 6% by weight of the implant, about 7% by weight of the implant, about 8% by weight of the implant, about 9% by weight of the implant, or about 10% by weight of the implant. In one embodiment, the implant is provided, wherein the compound is present in about 11% by weight of the implant, about 12% by weight of the implant, about 13% by weight of the implant, about 14% by weight of the implant, about 15% by weight of the implant, about 16% by weight of the implant, about 17% by weight of the implant, about 18% by weight of the implant, about 19% by weight of the implant, about 20% by weight of the implant, about 21% by weight of the implant, about 22% by weight of the implant, about 23% by weight of the implant, about 24% by weight of the implant, about 25% by weight of the implant, about 26% by weight of the implant, about 27% by weight of the implant, about 28% by weight of the implant, or about 29% by weight of the implant. In one embodiment, the implant is provided, wherein the compound is present in more than 10% by weight of the implant. In one embodiment, the implant is provided, wherein the compound is present in less than 30% by weight of the implant.

The release of the compound from the intraocular implant comprising a biodegradable polymer matrix may include an initial burst of release followed by a gradual increase in the amount of the compound released, or the release may include an initial delay in release of the compound followed by an increase in release. When the implant is substantially completely degraded, the percent of the compound that has been released is about one hundred.

In order to decrease the amount of initial burst a low soluble salt of the compound may be selected. In order to decrease the lag phase time for slow degrading polymer mixtures, a hydrophilic biocompatible polymer such as polyethylene glycol may be added.

The implants disclosed herein do not completely release, or release 100% of the compound, until after at least one week of being placed in an eye.

It may be desirable to provide a relatively constant rate of release of the compound from the implant over the life of the implant. For example, it may be desirable for the compound to be released in amounts from about 0.01 µg to 2 µg per day for the life of the implant. However, the release rate may change to either increase or decrease depending on the formulation of the biodegradable polymer matrix. In addition, the
release profile of the compound may include one or more linear portions and/or one or more non-linear portions. Preferably, the release rate is greater than zero once the implant has begun to degrade or erode. In one embodiment, the implant is provided, wherein the implant maintains a concentration of at least 50 nM of the compound in the retinal tissue. In one embodiment, the implant is provided, wherein the implant maintains a concentration of from 50 nM to 125 nM of the compound in the retinal tissue once a steady state equilibrium has been reached.

In one embodiment, the implant is provided, wherein the implant is configured to release from 0.1 µg per day to 2 µg per day of the compound as defined herein, such as from 0.1 to 0.2 µg per day, such as from 0.2 to 0.3 µg per day, such as from 0.3 to 0.4 µg per day, such as from 0.4 to 0.5 µg per day, such as from 0.5 to 0.6 µg per day, such as from 0.6 to 0.7 µg per day, such as from 0.7 to 0.8 µg per day, such as from 0.8 to 0.9 µg per day, such as from 0.9 to 1.0 µg per day, such as from 1.0 to 1.1 µg per day, such as from 1.1 to 1.2 µg per day, such as from 1.2 to 1.3 µg per day, such as from 1.3 to 1.4 µg per day, such as from 1.4 to 1.5 µg per day, such as from 1.5 to 1.6 µg per day, such as from 1.6 to 1.7 µg per day, such as from 1.7 to 1.8 µg per day, such as from 1.8 to 1.9 µg per day, such as from 1.9 to 2.0 µg per day. In one embodiment, the implant is provided, wherein the implant is configured to have an initial cumulative release of the compound as defined herein, of about 1% upon implantation, about 2% upon implantation, about 3% upon implantation, about 4% upon implantation, or about 5% upon implantation. As described herein, "upon implantation" refers to a period of time of about 1 day or less. In some embodiments, "upon implantation" refers to a period of time of about 1 hour or less, a period of time of about 2 hours or less, a period of time of about 3 hours or less, a period of time of about 4 hours or less, a period of time of about 5 hours or less, a period of time of about 6 hours or less, a period of time of about 7 hours or less, a period of time of about 8 hours or less, a period of time of about 9 hours or less, a period of time of about 10 hours or less, a period of time of about 11 hours or less, a period of time of about 12 hours or less, a period of time of about 13 hours or less, a period of time of about 14 hours or less, a period of time of about 15 hours or less, a period of time of about 16 hours or less, a period of time of about 17 hours or less, a period of time of about 18 hours or less, a period of time of about 19 hours or less, a period of time of about 20 hours or less, a period of time of about 21 hours or less, a period of time of about 22 hours or less, or a period of time of about 23 hours or less, or a period of time of about 24 hours or less. In one embodiment, the implant is provided, wherein the implant is configured to have a cumulative release of the compound as defined herein, of from about 5% to about 15% between implantation and day 20 after implantation. In one embodiment, the implant is provided, wherein the implant is configured to have a cumulative release of the compound as defined herein, of from about 10% to about 20% between implantation and day 20 after implantation. In one embodiment, the implant is provided, wherein the implant is configured to have a cumulative release of the compound as defined herein, of from about 10% to about 20% between day 20 after implantation and day 40 after implantation. In one embodiment, the implant is provided, wherein the implant is configured to have a cumulative release of the compound as defined herein, of from about 20% to about 30% between day 20 after implantation and day 40 after implantation. In one embodiment, the implant is provided, wherein the implant is configured to have a cumulative release of the compound as defined herein, of from about 10% to about 25% between day 40 after implantation and day 60 after implantation. In one embodiment, the implant is provided, wherein the implant is configured to have a cumulative release of the compound as defined herein, of from about 25% to about 60% between day 40 after implantation and day 60 after implantation. In one embodiment, the implant is provided, wherein the implant is configured to have a cumulative release of the compound as defined herein, of from about 20% to about 60% between day 60 after implantation and day 80 after implantation. In one embodiment, the implant is provided, wherein the implant is configured to have a cumulative release of the compound as defined herein, of from about 60% to about 75% between day 60 after implantation and day 80 after implantation. In one embodiment, the implant is provided, wherein the implant is configured to have a cumulative release of the compound as defined herein, of from about 60% to about 70% between day 80 after implantation and day 100 after implantation. In one embodiment, the implant is provided, wherein the implant is configured to have a cumulative release of the compound as defined herein, of from about 60% to about 75% between day 80 after implantation and day 100 after implantation. In one embodiment, the implant is provided, wherein the implant is configured to have a cumulative release of the compound as defined herein, of from about 75% to 100% between day 90 after implantation and day 100 after implantation. In one embodiment, the implant is provided, wherein the implant is configured to have a cumulative release of the compound as defined herein, of from about 75% to 100% between day 100 after implantation and day 110 after implantation. In one embodiment, the implant is provided, wherein the implant is configured to have a cumulative release of the compound as defined herein, of from about 75% to 100% between day 110 after implantation and day 120 after implantation. In one embodiment, the implant is provided, wherein the implant is configured to have a cumulative release of the compound as defined herein, of from about 80% to 100% between day 90 after implantation and day 100 after implantation. In one embodiment, the implant is provided, wherein the implant is configured to have a cumulative release of the compound as defined herein, of from about 80% to 100% between day 100 after implantation and day 110 after implantation. In one embodiment, the implant is provided, wherein the implant is configured to have a cumulative release of the compound as defined herein, of from about 80% to 100% between day 110 after implantation and day 120 after implantation. In one embodiment, the implant is provided, wherein the implant is configured to have a cumulative release of the compound as defined herein, of from about 90% to 100% between day 90 after implantation and day 100 after implantation. In one embodiment, the implant is provided, wherein the implant is configured to have a cumulative release of the compound as defined herein, of from about 90% to 100% between day 100 after implantation and day 110 after implantation. In one embodiment, the implant is provided, wherein the implant is configured to have a cumulative release of the compound as defined herein, of from about 90% to 100% between day 110 after implantation and day 120 after implantation. In one embodiment, the implant is provided, wherein the implant is configured to have a cumulative release of the compound as defined herein, of from about 5% to between 75% to 100% between day 1 after implantation and day 90 after implantation, wherein the rate of release is substantially linear between day 1 after implantation and day 90 after implantation. In one embodiment, the implant is provided, wherein the implant is configured to have a cumulative release of the compound as defined herein, of from about 5% to between 75% to 100% between day 1 after implantation and day 90 after implantation, wherein the rate of release is substantially linear between day 1 after implantation and day 100 after implantation. In one embodiment, the implant is provided, wherein the implant is configured to have a cumulative release of the compound as defined herein, of from about 5% to between 75% to 100% between day 1 after implantation and day 90 after implantation, wherein the rate of release is substantially linear between day 1 after implantation and day 110 after implantation. In one embodiment, the implant is provided, wherein the implant is configured to have a cumulative release of the compound as defined herein, of from about 5% to between 75% to 100% between day 1 after implantation and day 90 after implantation, wherein the rate of release is substantially linear between day 1 after implantation and day 120 after implantation. In one embodiment, the implant is provided, wherein the implant is configured to have a constant release rate of the compound as defined herein amounting to 0.2 - 0.5 µg per day after implantation and lasting up to 4 months. In one embodiment, the implant is provided, wherein the implant is configured to have a constant release rate of the compound as defined herein amounting to 0.5 - 1.0 µg per day after implantation and lasting up to 4 months. In one embodiment, the implant is provided, wherein the implant is configured to have a constant release rate of the compound as defined herein amounting to 1.0 - 2.0 µg per day after implantation and lasting up to 4 months. In one embodiment, the implant is provided, wherein the implant is configured to have a constant release rate of the compound as defined herein amounting to 2.0 - 3.0 µg per day after implantation and lasting up to 4 months. In one embodiment, the implant is provided, wherein the implant is configured to have a constant release rate of the compound as defined herein amounting to 3.0 - 4.0 µg per day after implantation and lasting up to 4 months. In any embodiment described herein, the implant is provided, wherein the implant is configured to have a substantially linear rate of release of the compound as defined herein, during any period of time selected from between day 1 after implantation and day 20 after implantation, between day 1 after implantation and day 40 after implantation, between day 1 after implantation and day 60 after implantation, between day 1 after implantation and day 80 after implantation, between day 1 after implantation and day 90 after implantation, between day 1 after implantation and day 100 after implantation, between day 1 after implantation and day 110 after implantation, and between day 1 after implantation and day 120 after implantation. In any embodiment described herein, the implant is provided, wherein the implant is configured to have a substantially linear rate of release of the compound as defined herein, during any period of time selected from between day 20 after implantation and day 40 after implantation, between day 20 after implantation and day 60 after implantation, between day 20 after implantation and day 80 after implantation, between day 20 after implantation and day 90 after implantation, between day 20 after implantation and day 100 after implantation, between day 20 after implantation and day 110 after implantation, and between day 20 after implantation and day 120 after implantation. In any embodiment described herein, the implant is provided, wherein the implant is configured to have a substantially linear rate of release of the compound as defined herein, during any period of time selected from between day 40 after implantation and day 60 after implantation, between day 40 after implantation and day 80 after implantation, between day 40 after implantation and day 90 after implantation, between day 40 after implantation and day 100 after implantation, between day 40 after implantation and day 110 after implantation, and between day 40 after implantation and day 120 after implantation. In any embodiment described herein, the implant is provided, wherein the implant is configured to have a substantially linear rate of release of the compound as defined herein, during any period of time selected from between day 60 after implantation and day 80 after implantation, between day 60 after implantation and day 90 after implantation, between day 60 after implantation and day 100 after implantation, between day 60 after implantation and day 110 after implantation, and between day 60 after implantation and day 120 after implantation. In any embodiment described herein, the implant is provided, wherein the implant is configured to have a substantially linear rate of release of the compound as defined herein, during any period of time selected from between day 80 after implantation and day 90 after implantation, between day 80 after implantation and day 100 after implantation, between day 80 after implantation and day 110 after implantation, and between day 80 after implantation and day 120 after implantation. In any embodiment described herein, the implant is provided, wherein the implant is configured to have a substantially linear rate of release of the compound as defined herein, during any period of time selected from between day 90 after implantation and day 100 after implantation, between day 90 after implantation and day 110 after implantation, and between day 90 after implantation and day 120 after implantation. In any embodiment described herein, the implant is provided, wherein the implant is configured to have a substantially linear rate of release of the compound as defined herein, during any period of time selected from between day 100 after implantation and day 110 after implantation, between day 100 after implantation and day 120 after implantation, and between day 110 after implantation and day 120 after implantation.

Implants containing more than 30% by weight compound often produce a "burst' of compound release upon immersion in a specific release medium such as PBS. On the other hand, for implants containing less than 20% by weight compound, the release of the compound defined herein is sometimes delayed resulting in an undesirable lag period between the time the implant is placed in release medium and the time significant quantities of compound begin appearing in the medium. The implant of the present disclosure is configured to provide a close to constant release rate of compound over time. A limited burst or loading dose which is released within the first days after implant insertion can be considered beneficial in terms of loading up the ocular tissues with the compound of formula (I). Hereafter, a constant release rate is preferred.

In one embodiment, the implant is provided, wherein the implant is configured to provide an initial burst release of the compound followed by a lower steady state concentration of the compound, such as from 50 nM to 125 nM steady state concentration. In one embodiment, the implant is provided, wherein the initial burst release provides a concentration of from 10 to 50 ng/mL of the compound, such as 30 ng/mL in the ocular compartment.

In one embodiment, the implant is provided, wherein the initial burst release provides a dose of from 150 to 300 ng of the compound over two days from the time the implant is placed in the vitreous of the eye.

In one embodiment, the implant is provided, wherein the implant is configured to release the compound for at least one month, such as at least two months, such as at least three months.

In one embodiment, the implant is provided, wherein the implant comprises from 2 to 50 w/w% of the compound as defined herein, such as from 2 to 3 w/w%, such as from 3 to 4 w/w%, such as from 4 to 5 w/w%, such as from 5 to 6 w/w%, such as from 6 to 7 w/w%, such as from 7 to 8 w/w%, such as from 8 to 9 w/w%, such as from 9 to 10 w/w%, such as from 10 to 11 w/w%, such as from 11 to 12 w/w%, such as from 12 to 13 w/w%, such as from 13 to 14 w/w%, such as from 14 to 15 w/w%, such as from 15 to 16 w/w%, such as from 16 to 17 w/w%, such as from 17 to 18 w/w%, such as from 18 to 19 w/w%, such as from 19 to 20 w/w%, such as from 20 to 21 w/w%, such as from 21 to 22 w/w%, such as from 22 to 23 w/w%, such as from 23 to 24 w/w%, such as from 24 to 25 w/w%, such as from 25 to 26 w/w%, such as from 26 to 27 w/w%, such as from 27 to 28 w/w%, such as from 28 to 29 w/w%, such as from 29 to 30 w/w%, such as from 30 to 31 w/w%, such as from 31 to 32 w/w%, such as from 32 to 33 w/w%, such as from 33 to 34 w/w%, such as from 34 to 35 w/w%, such as from 35 to 36 w/w%, such as from 36 to 37 w/w%, such as from 37 to 38 w/w%, such as from 38 to 39 w/w%, such as from 39 to 40 w/w%, such as from 40 to 41 w/w%, such as from 41 to 42 w/w%, such as from 42 to 43 w/w%, such as from 43 to 44 w/w%, such as from 44 to 45 w/w%, such as from 45 to 46 w/w%, such as from 46 to 47 w/w%, such as from 47 to 48 w/w%, such as from 48 to 49 w/w%, such as from 49 to 50 w/w%, for example from 2 to 25 w/w%.

In one embodiment, the implant is provided, wherein the implant comprises from 2 to 15 w/w% of the compound, and the biodegradable polymer matrix comprises a combination of two different polylactide polymers, such as two different PLGA's or two different PLA's.

### Form of the implants

The implants may be monolithic, i.e., having the active agent or agents homogenously distributed through the polymeric matrix, or encapsulated, where a reservoir of active agent is encapsulated by the polymeric matrix. In one embodiment, a monolithic implant is preferred over encapsulated forms. In one embodiment, the implant is provided, wherein the implant is a rod or microsphere shaped implant.

However, the greater control afforded by the encapsulated, reservoir-type implant may be of benefit in some circumstances, where the therapeutic level of the compound falls within a narrow window. In addition, the therapeutic component, including the compound, may be distributed in a non-homogenous pattern in the matrix. For example, the implant may include a portion that has a greater concentration of the compound relative to a second portion of the implant.

The intraocular implants disclosed herein can have a size of between 5 µm (µ or microns) and 2 mm, or between 10 µm and 1 mm for administration with a needle, greater than 1 mm, or greater than 2 mm, such as 3 mm or up to 10 mm, for administration by surgical implantation. The vitreous chamber in humans is able to accommodate relatively large implants of varying geometries, having lengths of, for example, from 1 to 10 mm. The implant may be a cylindrical pellet (e.g., a rod) with dimensions of about 2 mm x 0.75 mm diameter. Or the implant may be a cylindrical pellet with a length of 7 mm to 10 mm, and a diameter of 0.75 mm to 1.5 mm.

In one embodiment, the implant is provided, wherein the rod shaped implant has a diameter of from 0.1 mm to 5 mm, such as from 0.1 to 0.3 mm, such as from 0.3 to 0.5 mm, such as from 0.5 to 0.7 mm, such as from 0.7 to 0.9 mm, such as from 0.9 to 1.1 mm, such as from 1.1 to 1.3 mm, such as from 1.3 to 1.5 mm, such as 1.5 to 1.7 mm, such as from 1.7 to 1.9 mm, such as from 1.9 to 2.1 mm, such as from 2.1 to 2.3 mm, such as from 2.3 to 2.5 mm, such as from 2.5 to 2.7 mm, such as from 2.7 to 2.9 mm, such as from 2.9 to 3.1 mm, such as from 3.1 to 3.3 mm, such as from 3.3 to 3.5 mm, such as from 3.5 to 3.7 mm, such as from 3.7 to 3.9 mm, such as from 3.9 to 4.1 mm, such as from 4.1 to 4.3 mm, such as from 4.3 to 4.5 mm, such as from 4.5 to 4.7 mm, such as from 4.7 to 4.9 mm, such as from 4.9 to 5.0 mm.

In one embodiment, the implant is provided, wherein the rod shaped implant has a length of from 1 mm to 10 mm, such as from 1 to 2 mm, such as from 2 to 3 mm, such as from 3 to 4 mm, such as from 4 to 5 mm, such as from 5 to 6 mm, such as from 6 to 7, such as from 7 to 8 mm, such as from 8 to 9 mm, such as from 9 to 10 mm.

In one embodiment, the implant is provided, wherein the rod shaped implant has a diameter of from 0.2 mm to 0.8 mm, and a length of from 2 mm to 8 mm.

The implants may also be at least somewhat flexible so as to facilitate both insertion of the implant in the eye, such as in the vitreous, and accommodation of the implant. The total weight of the implant is usually 100-5000 µg, for example about 250-1000 µg. For example, an implant may be about 500 µg, or about 1000 µg. For non-human individuals, the dimensions and total weight of the implant(s) may be larger or smaller, depending on the type of individual. For example, humans have a vitreous volume of approximately 3.8 ml, compared with approximately 30 ml for horses, and approximately 60-100 ml for elephants. An implant sized for use in a human may be scaled up or down accordingly for other animals, for example, about 8 times larger for an implant for a horse, or about, for example, 26 times larger for an implant for an elephant.

Thus, implants can be prepared where the centre may be of one material and the surface may have one or more layers of the same or a different composition, where the layers may be cross-linked, or of a different molecular weight, different density or porosity, or the like. For example, where it is desirable to quickly release an initial bolus of compound, the centre may be a polylactide coated with a polylactatide- polyglycolide copolymer, so as to enhance the rate of initial degradation.

Alternatively, the centre may be polyvinyl alcohol coated with polylactide, so that upon degradation of the polylactide exterior the centre would dissolve and be rapidly washed out of the eye.

The implants may be of any geometry including fibres, sheets, films, microspheres, spheres, circular discs, plaques and the like. The upper limit for the implant size will be determined by factors such as toleration for the implant, size limitations on insertion, ease of handling, etc. Where sheets or films are employed, the sheets or films will be in the range of at least about 0.5 mm x 0.5 mm, usually about 3-10 mm x 5-10 mm with a thickness of about 0.1-1.0 mm for ease of handling. Where fibres are employed, the fibre diameter will generally be in the range of about 0.05 to 3 mm and the fibre length will generally be in the range of about 0.5-10 mm. Spheres may be in the range of 0.5 µm to 4 mm in diameter, with comparable volumes for other shaped particles.

The implant may be in the form of microspheres which may comprise or consist of a biodegradable polymer matrix, the compound of formula (I) or a pharmaceutically acceptable salt thereof, and optionally a pharmaceutically acceptable excipient. The biodegradable polymer matrix can comprise or consist of a poly(D,L-lactide), a poly(D,L-lactide-co-glycolide), a poly(ortho ester), a poly(phosphazine), a poly(phosphate ester), a polycaprolactone, a polyethylene glycol, a naturally occurring polymer, or any combination thereof. Useful naturally occurring polymers include gelatin and collagen. Some microspheres may comprise both a poly(D,L-lactide) and a poly(D,L-lactide-co-glycolide). Other polymers of interest include without limitation polyvinyl alcohol, polyanhydrides, polyamines, polyesteramides, polydioxanones, polyacetals, polyketals, polycarbonates, polyphosphesters, polyethers, polyesters, polybutylene, terephthalate, polyorthocarbonates, polyphosphazenes, succinates, poly(malic acid), poly(amino acids), polyvinylpyrrolidone, polysaccharides, copolymers, terpolymers, and combinations thereof that are biocompatible and may be biodegradable.

Suitable biodegradable polymers for use in the implant, such as the microspheres include those which are biocompatible, with the eye so as to cause no substantial interference with the functioning or physiology of the eye. Such materials preferably are at least partially and more preferably substantially completely biodegradable or bioerodible. Useful polyethylene glycols have a molecular weight of about 300 to about 40,000. Specific examples of polyethylene glycols that may be included in a microsphere formulation include polyethylene glycol 3350 (PEG 3350), PEG 4400, and PEG 8000. In other instances, a polyethylene glycol with a molecular weight of about 20,000 (PEG 20K) may be used. In one embodiment, the implant is provided, wherein a PEG is present in about 1% by weight of the implant, about 2% by weight of the implant, about 3% by weight of the implant, about 4% by weight of the implant, about 5% by weight of the implant, about 6% by weight of the implant, about 7% by weight of the implant, about 8% by weight of the implant, about 9% by weight of the implant, or about 10% by weight of the implant. In one embodiment, the implant is provided, wherein a PEG is present in about 11% by weight of the implant, about 12% by weight of the implant, about 13% by weight of the implant, about 14% by weight of the implant, about 15% by weight of the implant, about 16% by weight of the implant, about 17% by weight of the implant, about 18% by weight of the implant, about 19% by weight of the implant, or about 20% by weight of the implant.

Microspheres of the present disclosure can have a diameter between about 25 µm and about 150 µm. A plurality of microspheres can have diameters within the range between 25 µm and 150 µm or within a narrower range such as 40 µm and 50 µm or 75 and 100 µm. The average diameter, of a plurality of microspheres can be between 40 and 80 µm, preferably between 50 and 70 µm. The average diameter of a plurality of microspheres of the disclosure can be 40 µm, 45 µm, 50 µm, 55 µm, 60 µm, 65 µm, 70 µm, 75 µm, 80 µm or any number in between.

The size and form of the implant can also be used to control the rate of release, period of treatment, and drug concentration at the site of implantation.

Larger implants will deliver a proportionately larger dose, but depending on the surface to mass ratio, may have a slower release rate. The particular size and geometry of the implant are chosen to suit the site of implantation.

The proportions of compound, polymer, and any other modifiers may be empirically determined by formulating several implants with varying proportions. A USP approved method for dissolution or release test can be used to measure the rate of release (USP 23; NF 18 (1995) pp. 1790-1798). For example, using the infinite sink method, a weighed sample of the implant is added to a measured volume of a solution containing 0.9% NaCl in water, where the solution volume will be such that the compound concentration after release is less than 5% of saturation. The mixture is maintained at 37 °C and stirred slowly to maintain the implants in suspension. The appearance of the dissolved drug as a function of time may be followed by various methods known in the art, such as spectrophotometrically, HPLC, mass spectroscopy, etc. until the absorbance becomes constant or until greater than 90% of the drug has been released.

In some embodiments, the implant may be delivered directly via a needle injection, surgical placement, or injection into a portal like device that is surgically implanted in the eye of a subject. A suitable device has a reservoir chamber coupled to a porous structure for controlled release of a therapeutic agent in the vitreous of the eye after the system is placed or inserted into the eye. The implant of the disclosure comprising a compound of formula (I), or a pharmaceutically acceptable salt thereof, is placed in the reservoir before delivery, and the controlled release of the therapeutic agent and any formulation agents from the reservoir through the porous structure increases the residence time of the therapeutic agent in the vitreous.

### Additives

The intraocular implants disclosed herein may include effective amounts of buffering agents, preservatives and the like.

Suitable water soluble buffering agents include, without limitation, alkali and alkaline earth carbonates, phosphates, bicarbonates, citrates, borates, acetates, succinates and the like, such as sodium phosphate, citrate, borate, acetate, bicarbonate, carbonate and the like. These agents advantageously present in amounts sufficient to maintain a pH of the system of between about 2 to about 9 and more preferably about 4 to about 8. As such the buffering agent may be as much as about 5% by weight of the total implant. Suitable additives include sodium citrate, mannitol, sodium bisulfite, sodium bisulfate, sodium thiosulfate, ascorbate, benzalkonium chloride, chlorobutanol, thimerosal, phenylmercuric acetate, phenylmercuric borate, phenylmercuric nitrate, parabens, methylparaben, polyvinyl alcohol, benzyl alcohol, phenylethanol and the like and mixtures thereof. These agents may be present in amounts of from 0.001 to about 5% by weight and preferably 0.01 to about 2% by weight. In at least one of the present implants, a Purite preservative is provided in the implant.

Additionally, release modulators such as those described in U. S. Patent No. 5,869,079 may be included in the implants. The amount of release modulator employed will be dependent on the desired release profile, the activity of the modulator, and on the release profile of the compound of formula (I) in the absence of modulator. Electrolytes such as sodium chloride and potassium chloride may also be included in the implant. Where the buffering agent or enhancer is hydrophilic, it may also act as a release accelerator. Hydrophilic additives act to increase the release rates through faster dissolution of the material surrounding the drug particles, which increases the surface area of the compound exposed, thereby increasing the rate of compound bioerosion. Similarly, a hydrophobic buffering agent or enhancer dissolve more slowly, slowing the exposure of drug particles, and thereby slowing the rate of drug bioerosion.

In certain implants, an implant comprising the compound of formula (I) and a biodegradable polymer matrix is able to release or deliver an amount of the compound between 0.025 mg to 0.5 mg for about 3-6 months after implantation into the eye. The implant may be configured as a rod or a wafer. A rod-shaped implant may be derived from filaments extruded from a 720 µm nozzle and cut into for example from 25 microgram to 2000 microgram in size / weight. For example, the rod-shaped implant may be from 25 microgram to 50 microgram in weight, from 50 microgram to 100 microgram in weight, from 100 microgram to 200 microgram in weight, 200 microgram to 300 microgram in weight, 300 microgram to 400 microgram in weight, 400 microgram to 500 microgram in weight, 500 microgram to 600 microgram in weight, 600 microgram to 700 microgram in weight, 700 microgram to 800 microgram in weight, 800 microgram to 900 microgram in weight, 900 microgram to 1000 microgram in weight, 1000 microgram to 1100 microgram in weight, 1100 microgram to 1200 microgram in weight, 1200 microgram to 1300 microgram in weight, 1300 microgram to 1400 microgram in weight, 1400 microgram to 1500 microgram in weight, 1500 microgram to 1600 microgram in weight, 1600 microgram to 1700 microgram in weight, 1700 microgram to 1800 microgram in weight, 1800 microgram to 1900 microgram in weight, or 1900 microgram to 2000 microgram in weight. A wafer-shaped implant may be a circular disc having a diameter of about 2.5 mm, a thickness of about 0.127 mm, and a weight of about 1 mg.

The proposed 3-month release formulations may be sterile, and bioerodible in the form of a rod, a wafer or a microsphere containing danegaptide tartrate within a PLA matrix or POE matrix.

### Terminal sterilization

In order to use a polymeric material, including a drug delivery system, such as an intraocular implant in the body of a person or mammal, the polymeric material must be sterile. Sterilization can be carried out by chemical treatment (such as by ethylene oxide gas), heat treatment, filtration, irradiation, or other methods. The method of sterilization is chosen considering factors such as the polymeric materials used, the identity of active agents used, and the particular use of the polymeric material in a human or animal body. The implant according to the present disclosure is in one embodiment provided by sterile manufacturing using pre-sterilized ingredients. In this case, terminal sterilization is not required.

During irradiation, microspheres, microparticles, and microcapsules tend to agglomerate and aggregate, altering i.a. the drug release properties of the materials. In the case of polymeric materials comprising particles, the diffusion and degradation properties of the particles are dependent on surface area to volume relationships, which are affected by aggregation. As such, surface area changes encountered with aggregation will cause significant variability in drug release and particle degradation profile. In addition, the chemical and/or physical changes effected by gamma irradiation may affect the diffusion and degradation properties of polymeric materials in other ways. In particular, gamma irradiation tends to increase the drug release from an implant.

Some examples of radiation which may be used to sterilize the implant include gamma radiation, alpha radiation, beta radiation, microwave radiation, and ultraviolet radiation. In the preferred embodiment, the implant is sterilized by gamma irradiation. For example, the sterilization is by gamma irradiation at a dose of about 1.5 to about 4.0 mRad. In one example, implants may be sterilized by a gamma radiation dose of 25 to 40 kGy.

### Preparation of the implants

Various techniques may be employed to produce the implants described herein. Useful techniques include, but are not necessarily limited to, solvent evaporation methods, phase separation methods, interfacial methods, molding methods, cast molding, injection molding methods, extrusion methods, co-extrusion methods, carver press method, die cutting methods, heat compression, combinations thereof and the like.

Extrusion methods may be used to avoid the need for solvents in manufacturing. When using extrusion methods, the polymer and drug are chosen so as to be stable at the temperatures required for manufacturing, usually at least about 85 degrees Celsius. Extrusion methods use temperatures of 25 degrees C to 185 degrees C, more preferably 65 degrees C to 130 degrees C. An implant may be produced by bringing the temperature to 60 degrees C to 185 degrees C for mixing the polymer and the compound of formula (I), such as 130 degrees C, for a time period of 0 to 1 hour, 0 to 30 minutes, or 5-15 minutes. For example, a time period may be 10 minutes, preferably 0 to 5 min. The implants are then extruded at a temperature of 60 degrees C to 130 degrees C, such as 75 degrees C.

In addition, the implant may be coextruded so that a coating is formed over a core region during the manufacture of the implant.

Compression methods may be used to make the implants, and typically yield implants with faster release rates than extrusion methods. Compression methods may use pressures of 50-150 psi, more preferably 70-80 psi, even more preferably 76 psi, and use temperatures of 0 degrees C to 115 degrees C, more preferably 25 degrees C.

In one embodiment, the implant of the present disclosure can be prepared by 3D printing using techniques known in the art.

### Methods for applying the implants

The implants disclosed herein may be inserted into the eye, for example the vitreous chamber of the eye, by a variety of methods, including placement by forceps or by trocar following making a 2-3 mm incision in the sclera.

The method of placement may influence the compound release kinetics. For example, delivering the implant with a trocar may result in placement of the implant deeper within the vitreous than placement by forceps, which may result in the implant being closer to the edge of the vitreous. The location of the implant may influence the concentration gradients of therapeutic component or drug surrounding the element, and thus influence the release rates (e. g., an element placed closer to the edge of the vitreous may result in a slower release rate).

### Exemplary formulations

The dissolution profile depends on several parameters of the formulation. Non-limiting examples of parameters of influence are the salt form, the weight% of drug associated with the polymer matrix, the nature of the polymers, and the inherent viscosity. The parameters provided herein are based on the ingredients prior to any sterilization, such as terminal sterilization by e.g., gamma radiation.

The reference codes "Ref" provided in the following tables refer to polymers of the Resomer^{®} type from Evonik. The polymers provided in the following tables can be obtained from Evonik but the polymers can also be obtained from other sources.

PLGA copolymers of the present disclosure are for example **50:50** (lactide:glycolide) polymers according to table 1.

**Table 1. List of PLGAs with 50:50 ratio between lactide and glycolide.**

| **50:50 PLGA's** | | |
|---|---|---|
| **Ref** | **Name** | **Average molecular weight (g/mol)** |
| | Poly(D,L-lactide-co-glycolide) lactide:glycolide (50:50) | 30,000-60,000 |
| | Poly(D,L-lactide-co-glycolide) lactide:glycolide 50:50 | 45,000 |
| | Poly(D,L-lactide-co-glycolide) lactide:glycolide 50:50, acid and hydroxy terminated | 25,000 |
| | Poly(D,L-lactide-co-glycolide) lactide:glycolide (50:50), ester terminated | 100,000 |
| RG 502 | Poly(D,L-lactide-co-glycolide) lactide:glycolide 50:50, ester terminated | 7,000-17,000 |
| RG 502 H | Poly(D,L-lactide-co-glycolide) acid terminated | 7,000-17,000 |
| RG 503 | Poly(D,L-lactide-co-glycolide) lactide:glycolide 50:50, ester terminated | 24,000-38,000 |
| RG 503 H | Poly(D,L-lactide-co-glycolide) acid terminated, lactide:glycolide 50:50 | 24,000-38,000 |
| RG 504 | Poly(D,L-lactide-co-glycolide) lactide:glycolide 50:50, ester terminated | 38,000-54,000 |
| RG 504 H | Poly(D,L-lactide-co-glycolide) acid terminated, lactide:glycolide 50:50 | 38,000-54,000 |
| RG 505 | Poly(D,L-lactide-co-glycolide) ester terminated 50:50 | 54,000-69,000 |
| Condensate | Poly(D,L-lactide-co-glycolide) 50:50 | 700 - 900 |
| RG 50:50 | | |
| Mn 800 | | |
| Condensate | Poly(D,L-lactide-co-glycolide) 50:50 | 2000 - 2500 |
| RG 50:50 | | |
| Mn 2300 | | |

PLGA copolymers of the present disclosure are for example **65:35** (lactide:glycolide) polymers according to table 2.

**Table 2. List of PLGAs with 65:35 ratio between lactide and glycolide.**

| **65:35 PLGA's** | | |
|---|---|---|
| **Ref** | **Name** | **Average molecular weight (g/mol)** |
| | Poly(D,L-lactide-co-glycolide) lactide:glycolide 65:35 | 40,000-75,000 |
| | Poly(L-lactide-co-glycolide) lactide:glycolide 65:35, viscosity 0.6 dL/g | |
| RG 653 H | Poly(D,L-lactide-co-glycolide) acid terminated | 24,000-38,000 |

PLGA copolymers of the present disclosure are for example **75:25** (lactide:glycolide) polymers according to table 3.

**Table 3. List of PLGAs with 75:25 ratio between lactide and glycolide.**

| **75:25 PLGA's** | | |
|---|---|---|
| **Ref** | **Name** | **Average molecular weight (g/mol)** |
| | Poly(D,L-lactide-co-glycolide) lactide:glycolide (75:25) | 66,000-107,000 |
| | Poly(D,L-lactide-co-glycolide) acid terminated lactide:glycolide 75:25 | 6,000-10,000 |
| RG 750 S | Poly(D,L-lactide-co-glycolide) ester terminated, lactide:glycolide 75:25 | |
| RG 752 S | Poly(D,L-lactide-co-glycolide) ester terminated, lactide:glycolide 75:25 | 4,000-15,000 |
| RG 752 H | Poly(D,L-lactide-co-glycolide) acid terminated, lactide:glycolide 75:25 | 4,000-15,000 |
| RG 753 S | Poly(D,L-lactide-co-glycolide) ester terminated, lactide: glycolide 75:25 | 66,000 -107,000 |
| RG 753 H | Poly(D,L-lactide-co-glycolide) acid terminated | 66,000 -107,000 |
| RG 755 S | Poly(D,L-lactide-co-glycolide) ester terminated | |
| RG 756 S | Poly(D,L-lactide-co-glycolide) ester terminated, lactide:glycolide 75:25 | 76,000-115,000 |
| RG 757 S | Poly(D,L-lactide-co-glycolide) ester terminated, lactide: glycolide 75:25 | |

PLGA copolymers of the present disclosure are for example **85:15** (Lactide:glycolide) polymers according to table 4.

**Table 4. List of PLGAs with 85:15 ratio between lactide and glycolide.**

| **85:15 PLGA's** | | |
|---|---|---|
| **Ref** | **Name** | **Average molecular weight (g/mol)** |
| | Poly(D,L-lactide-co-glycolide) ester terminated 85:15 | 50,000-75,000 |
| | Poly(D,L-lactide-co-glycolide)-COOH, lactide:glycolide 85:15 | 17,000 |
| LG 855 S | Poly(L-lactide-co-glycolide) ester terminated, lactide:glycolide 85:15 | |
| LG 857 S | Poly(L-lactide-co-glycolide) ester terminated, lactide:glycolide 85:15 | |
| RG 858 S | Poly(D,L-lactide-co-glycolide) ester terminated, lactide:glycolide 85:15 | 190,000-240,000 |

PLGA copolymers of the present disclosure are for example **5:95, 10:90, 20:80,** or **82:18** (Lactide:glycolide) polymers according to table 5.

**Table 5. List of PLGAs with 5:95, 10:90, 20:80, or 82:18 ratio between lactide and glycolide.**

| **5:95, 10:90, 20:80, or 82:18PLGA's** | | |
|---|---|---|
| **Ref** | **Name** | **Average molecular weight (g/mol)** |
| | Poly(L-lactide-co-glycolide) L-lactide:glycolide 5:95, viscosity ≥ 1.1 dL/g | |
| | Poly(L-lactide-co-glycolide) 10:90, viscosity 1.7 dL/g | |
| | Poly(L-lactide-co-glycolide) lactide:glycolide 20:80, viscosity 1.6 dL/g | |
| | Poly(D,L-lactide-co-glycolide) ester terminated lactide:glycolide 80:20 | 200,000 |
| LG 824 S | Poly(L-lactide-co-glycolide) ester terminated, lactide: glycolide 82:18 | |

PLA polymers of the present disclosure are for example polylactide polymers according to table 6.

**Table 6. List of PLAs with molecular weights between 10,000 and 28,000 g/mol.**

| **PLA's** | | |
|---|---|---|
| **Ref** | **Name** | **Inherent viscosity (dL/g)** |
| R 202 H | Poly(D,L-lactide), Acid end group | 0.2 |
| R 202 S | Poly(D,L-lactide), Ester end group | 0.2 |
| R 203 H | Poly(D,L-lactide), Acid end group | 0.3 |
| R 203 S | Poly(D,L-lactide), Ester end group | 0.3 |
| R 205 H | Poly(D,L-lactide), Ester end group | 0.6-0.7 |

The biodegradable intraocular implant according to the present disclosure comprises an extended-release biodegradable polymer matrix. The extended-release biodegradable polymer matrix may comprise any combination of polymers included herein, and in particular the polymers and copolymers included in tables 1 to 6.

In one embodiment, the biodegradable polymer matrix may comprise a first biodegradable polymer of polylactic acid, and a different second biodegradable polymer of polylactic acid, wherein the first and second biodegradable polymers of polylactic acid are independently selected from the polymers according to tables 1 to 6.

**Table 7: List of exemplary formulations.**

| **ID** | **Composition** | **w/w%** | **Rod Proportions** |
|---|---|---|---|
| 1 | Danegaptide Hydrochloride | 5 | Diameter 0.3 mm |
| | RG 502 | 95 | Length 3 mm |
| 2 | Danegaptide Hydrochloride | 5 | Diameter 0.3 mm |
| | RG 502H | 95 | Length 3 mm |
| 3 | Danegaptide Hydrochloride | 10 | Diameter 0.3 mm |
| | RG 502H+RG 502 50/50 | 90 | Length 3 mm |
| 4 | Danegaptide Maleate | 5 | Diameter 0.37 mm |
| | RG 502 | 95 | Length 3.5 mm |
| 5 | Danegaptide Pamoate | 5 | Diameter 0.37 mm |
| | RG 502 | 95 | Length 3.5 mm |
| 6 | Danegaptide Hydrochloride | 10 | Diameter 0.37 mm |
| | RG 503 | 90 | Length 3.5 mm |
| 7 | Danegaptide Pamoate | 10 | Diameter 0.37 mm |
| | RG 503H | 90 | Length 3.5 mm |
| 8 | Danegaptide Hydrochloride | 5 | Diameter 0.45 mm |
| | RG 503+RG752H 80/20 | 95 | Length 6 mm |
| 9 | Danegaptide Hydrochloride | 5 | Diameter 0.45 mm |
| | RG 503+R 203S 80/20 | 95 | Length 6 mm |
| 10 | Danegaptide Hydrochloride | 5 | Diameter 0.45 mm |
| | RG 503+RG752S 80/20 | 95 | Length 6 mm |
| 11 | Danegaptide Hydrochloride | 5 | Diameter 0.45 mm |
| | R 202H+RG752S 80/20 | 95 | Length 6 mm |
| 12 | Danegaptide Hydrochloride | 10 | Diameter 0.45 mm |
| | RG 503+RG752S 20/80 | 90 | Length 6 mm |
| 13 | Danegaptide Hydrochloride | 10 | Diameter 0.45 mm |
| | R202H+RG752S 20/80 | 90 | Length 6 mm |
| 14 | Danegaptide Tartrate | 10 | Diameter 0.45 mm |
| | RG 503+RG752S 20/80 | 90 | Length 6 mm |
| 15 | Danegaptide Hydrochloride | 10 | Diameter 0.45 mm |
| | RG 503+RG753S 20/80 | 90 | Length 6 mm |
| 16 | Danegaptide Pamoate | 15 | Diameter 0.45 mm |
| | RG 503+RG753S 20/80 | 85 | Length 6 mm |
| 17 | Danegaptide Pamoate | 15 | Diameter 0.45 mm |
| | R202H+RG753S 20/80 | 85 | Length 6 mm |
| 18 | Danegaptide Hydrochloride | 15 | Diameter 0.45 mm |
| | RG 653H | 85 | Length 6 mm |
| 19 | Danegaptide Tartrate | 15 | Diameter 0.45 mm |
| | RG 653H | 85 | Length 6 mm |
| 20 | Danegaptide Hydrochloride | 20 | Diameter 0.45 mm |
| | RG 753H | 80 | Length 6 mm |
| 21 | Danegaptide Hydrochloride | 20 | Diameter 0.45 mm |
| | RG 753S | 80 | Length 6 mm |
| 22 | Danegaptide Pamoate | 20 | Diameter 0.45 mm |
| | RG 753S | 80 | Length 6 mm |
| 23 | Danegaptide Pamoate | 20 | Diameter 0.45 mm |
| | RG 756S | 80 | Length 6 mm |
| 24 | Danegaptide Pamoate | 20 | Diameter 0.45 mm |
| | RG 756S+RG502H 90/10 | 80 | Length 6 mm |
| 25 | Danegaptide Pamoate | 20 | Diameter 0.45 mm |
| | RG 756S+R202H 90/10 | 80 | Length 6 mm |
| 26 | Danegaptide Pamoate | 20 | Diameter 0.45 mm |
| | RG 756S+RG503H 90/10 | 80 | Length 6 mm |
| 27 | Danegaptide Hydrochloride | 30 | Diameter 0.45 mm |
| | LG 855S | 70 | Length 6 mm |
| 28 | Danegaptide Hydrochloride | 30 | Diameter 0.45 mm |
| | LG 857S | 70 | Length 6 mm |
| 29 | Danegaptide Hydrochloride | 30 | Diameter 0.45 mm |
| | LG 858S | 70 | Length 6 mm |
| 30 | Danegaptide Hydrochloride | 30 | Diameter 0.45 mm |
| | LG 855S+RG503 90/10 | 70 | Length 6 mm |
| 31 | Danegaptide Pamoate | 30 | Diameter 0.45 mm |
| | LG 855S+RG503 90/10 | 70 | Length 6 mm |
| 32 | Danegaptide Pamoate | 30 | Diameter 0.45 mm |
| | LG 855S+R202H 90/10 | 70 | Length 6 mm |

The biodegradable intraocular implant is in one embodiment selected from any of the compositions termed ID1-32 from Table 7.

Hence, in one embodiment, the implant comprises danegaptide hydrochloride and RG 502, optionally in 5:95 w/w%.

In one embodiment, the implant comprises danegaptide hydrochloride and RG 502 H, optionally in 5:95 w/w%.

In one embodiment, the implant comprises danegaptide hydrochloride and RG 502H+RG 502 50/50, optionally in 10:90 w/w%.

In one embodiment, the implant comprises danegaptide maleate and RG 502, optionally in 5:95 w/w%.

In one embodiment, the implant comprises danegaptide pamoate and RG 502, optionally in 5:95 w/w%.

In one embodiment, the implant comprises danegaptide hydrochloride and RG 503, optionally in 10:90 w/w%.

In one embodiment, the implant comprises danegaptide pamoate and RG 503 H, optionally in 10:90 w/w%.

In one embodiment, the implant comprises danegaptide hydrochloride and RG 503+RG752H 80/20, optionally in 5:95 w/w%.

In one embodiment, the implant comprises danegaptide hydrochloride and RG 503+R 203S 80/20, optionally in 5:95 w/w%.

In one embodiment, the implant comprises danegaptide hydrochloride and RG 503+RG752S 80/20, optionally in 5:95 w/w%.

In one embodiment, the implant comprises danegaptide hydrochloride and R 202H+RG752S 80/20, optionally in 5:95 w/w%.

In one embodiment, the implant comprises danegaptide hydrochloride and RG 503+RG752S 20/80, optionally in 10:90 w/w%.

In one embodiment, the implant comprises danegaptide hydrochloride and R202H+RG752S 20/80, optionally in 10:90 w/w%.

In one embodiment, the implant comprises danegaptide tartrate and RG 503+RG752S 20/80, optionally in 10:90 w/w%.

In one embodiment, the implant comprises danegaptide hydrochloride and RG 503+RG753S 20/80, optionally in 10:90 w/w%.

In one embodiment, the implant comprises danegaptide pamoate and RG 503+RG753S 20/80, optionally in 15:85 w/w%.

In one embodiment, the implant comprises danegaptide pamoate and R202H+RG753S 20/80, optionally in 15:85 w/w%.

In one embodiment, the implant comprises danegaptide hydrochloride and RG 653H, optionally in 15:85 w/w%.

In one embodiment, the implant comprises danegaptide tartrate and RG 653H, optionally in 15:85 w/w%.

In one embodiment, the implant comprises danegaptide hydrochloride and RG 753H, optionally in 20:80 w/w%.

In one embodiment, the implant comprises danegaptide hydrochloride and RG 753S, optionally in 20:80 w/w%.

In one embodiment, the implant comprises danegaptide pamoate and RG 753S, optionally in 20:80 w/w%.

In one embodiment, the implant comprises danegaptide pamoate and RG 756S, optionally in 20:80 w/w%.

In one embodiment, the implant comprises danegaptide pamoate and RG 756S+RG502H 90/10, optionally in 20:80 w/w%.

In one embodiment, the implant comprises danegaptide pamoate and RG 756S+R202H 90/10, optionally in 20:80 w/w%.

In one embodiment, the implant comprises danegaptide pamoate and RG 756S+RG503H 90/10, optionally in 20:80 w/w%.

In one embodiment, the implant comprises danegaptide hydrochloride and LG 855S, optionally in 30:70 w/w%.

In one embodiment, the implant comprises danegaptide hydrochloride and LG 857S, optionally in 30:70 w/w%.

In one embodiment, the implant comprises danegaptide hydrochloride and LG 858S, optionally in 30:70 w/w%.

In one embodiment, the implant comprises danegaptide hydrochloride and LG 855S+RG503 90/10, optionally in 30:70 w/w%.

In one embodiment, the implant comprises danegaptide pamoate and LG 855S+RG503 90/10, optionally in 30:70 w/w%.

In one embodiment, the implant comprises danegaptide pamoate and LG 855S+R202H 90/10, optionally in 30:70 w/w%.

In one embodiment, the implant consists of between about 11% and about 29% w/w of danegaptide or a pharmaceutically acceptable salt thereof, between 0% and about 7.5% w/w of PEG3350 or a substantially similar polymer, between about 15% and about 25% w/w of R203S or a substantially similar polymer, between about 10% and about 20% w/w of R202H or a substantially similar polymer, and between about 35% and about 45% of RG752S or a substantially similar polymer. In one embodiment, the implant consists of about 15% w/w of danegaptide or a pharmaceutically acceptable salt thereof, between 0% and about 7.5% w/w of PEG3350 or a substantially similar polymer, between about 15% and about 25% w/w of R203S or a substantially similar polymer, between about 10% and about 20% w/w of R202H or a substantially similar polymer, and between about 35% and about 45% of RG752S or a substantially similar polymer. In one embodiment, the implant consists of about 16% w/w of danegaptide or a pharmaceutically acceptable salt thereof, between 0% and about 7.5% w/w of PEG3350 or a substantially similar polymer, between about 15% and about 25% w/w of R203S or a substantially similar polymer, between about 10% and about 20% w/w of R202H or a substantially similar polymer, and between about 35% and about 45% of RG752S or a substantially similar polymer. In one embodiment, the implant consists of about 17% w/w of danegaptide or a pharmaceutically acceptable salt thereof, between 0% and about 7.5% w/w of PEG3350 or a substantially similar polymer, between about 15% and about 25% w/w of R203S or a substantially similar polymer, between about 10% and about 20% w/w of R202H or a substantially similar polymer, and between about 35% and about 45% of RG752S or a substantially similar polymer. In one embodiment, the implant consists of about 18% w/w of danegaptide or a pharmaceutically acceptable salt thereof, between 0% and about 7.5% w/w of PEG3350 or a substantially similar polymer, between about 15% and about 25% w/w of R203S or a substantially similar polymer, between about 10% and about 20% w/w of R202H or a substantially similar polymer, and between about 35% and about 45% of RG752S or a substantially similar polymer. In one embodiment, the implant consists of about 19% w/w of danegaptide or a pharmaceutically acceptable salt thereof, between 0% and about 7.5% w/w of PEG3350 or a substantially similar polymer, between about 15% and about 25% w/w of R203S or a substantially similar polymer, between about 10% and about 20% w/w of R202H or a substantially similar polymer, and between about 35% and about 45% of RG752S or a substantially similar polymer. In one embodiment, the implant consists of about 20% w/w of danegaptide or a pharmaceutically acceptable salt thereof, between 0% and about 7.5% w/w of PEG3350 or a substantially similar polymer, between about 15% and about 25% w/w of R203S or a substantially similar polymer, between about 10% and about 20% w/w of R202H or a substantially similar polymer, and between about 35% and about 45% of RG752S or a substantially similar polymer. In one embodiment, the implant consists of about 21% w/w of danegaptide or a pharmaceutically acceptable salt thereof, between 0% and about 7.5% w/w of PEG3350 or a substantially similar polymer, between about 15% and about 25% w/w of R203S or a substantially similar polymer, between about 10% and about 20% w/w of R202H or a substantially similar polymer, and between about 35% and about 45% of RG752S or a substantially similar polymer. In one embodiment, the implant consists of about 22% w/w of danegaptide or a pharmaceutically acceptable salt thereof, between 0% and about 7.5% w/w of PEG3350 or a substantially similar polymer, between about 15% and about 25% w/w of R203S or a substantially similar polymer, between about 10% and about 20% w/w of R202H or a substantially similar polymer, and between about 35% and about 45% of RG752S or a substantially similar polymer. In one embodiment, the implant consists of about 23% w/w of danegaptide or a pharmaceutically acceptable salt thereof, between 0% and about 7.5% w/w of PEG3350 or a substantially similar polymer, between about 15% and about 25% w/w of R203S or a substantially similar polymer, between about 10% and about 20% w/w of R202H or a substantially similar polymer, and between about 35% and about 45% of RG752S or a substantially similar polymer. In one embodiment, the implant consists of about 24% w/w of danegaptide or a pharmaceutically acceptable salt thereof, between 0% and about 7.5% w/w of PEG3350 or a substantially similar polymer, between about 15% and about 25% w/w of R203S or a substantially similar polymer, between about 10% and about 20% w/w of R202H or a substantially similar polymer, and between about 35% and about 45% of RG752S or a substantially similar polymer. In one embodiment, the implant consists of about 25% w/w of danegaptide or a pharmaceutically acceptable salt thereof, between 0% and about 7.5% w/w of PEG3350 or a substantially similar polymer, between about 15% and about 25% w/w of R203S or a substantially similar polymer, between about 10% and about 20% w/w of R202H or a substantially similar polymer, and between about 35% and about 45% of RG752S or a substantially similar polymer. In one embodiment, the implant consists of between about 11% and about 29% w/w of danegaptide or a pharmaceutically acceptable salt thereof, between about 0% and about 7.5% w/w of PEG3350 or a substantially similar polymer, between about 25% and about 35% w/w of R202S or a substantially similar polymer, and between about 45% and about 55% of RG752S or a substantially similar polymer. In one embodiment, the implant consists of about 15% w/w of danegaptide or a pharmaceutically acceptable salt thereof, between about 0% and about 7.5% w/w of PEG3350 or a substantially similar polymer, between about 25% and about 35% w/w of R202S or a substantially similar polymer, and between about 45% and about 55% of RG752S or a substantially similar polymer. In one embodiment, the implant consists of about 16% w/w of danegaptide or a pharmaceutically acceptable salt thereof, between about 0% and about 7.5% w/w of PEG3350 or a substantially similar polymer, between about 25% and about 35% w/w of R202S or a substantially similar polymer, and between about 45% and about 55% of RG752S or a substantially similar polymer. In one embodiment, the implant consists of about 17% w/w of danegaptide or a pharmaceutically acceptable salt thereof, between about 0% and about 7.5% w/w of PEG3350 or a substantially similar polymer, between about 25% and about 35% w/w of R202S or a substantially similar polymer, and between about 45% and about 55% of RG752S or a substantially similar polymer. In one embodiment, the implant consists of about 18% w/w of danegaptide or a pharmaceutically acceptable salt thereof, between about 0% and about 7.5% w/w of PEG3350 or a substantially similar polymer, between about 25% and about 35% w/w of R202S or a substantially similar polymer, and between about 45% and about 55% of RG752S or a substantially similar polymer. In one embodiment, the implant consists of about 19% w/w of danegaptide or a pharmaceutically acceptable salt thereof, between about 0% and about 7.5% w/w of PEG3350 or a substantially similar polymer, between about 25% and about 35% w/w of R202S or a substantially similar polymer, and between about 45% and about 55% of RG752S or a substantially similar polymer. In one embodiment, the implant consists of about 20% w/w of danegaptide or a pharmaceutically acceptable salt thereof, between about 0% and about 7.5% w/w of PEG3350 or a substantially similar polymer, between about 25% and about 35% w/w of R202S or a substantially similar polymer, and between about 45% and about 55% of RG752S or a substantially similar polymer. In one embodiment, the implant consists of about 21% w/w of danegaptide or a pharmaceutically acceptable salt thereof, between about 0% and about 7.5% w/w of PEG3350 or a substantially similar polymer, between about 25% and about 35% w/w of R202S or a substantially similar polymer, and between about 45% and about 55% of RG752S or a substantially similar polymer. In one embodiment, the implant consists of about 22% w/w of danegaptide or a pharmaceutically acceptable salt thereof, between about 0% and about 7.5% w/w of PEG3350 or a substantially similar polymer, between about 25% and about 35% w/w of R202S or a substantially similar polymer, and between about 45% and about 55% of RG752S or a substantially similar polymer. In one embodiment, the implant consists of about 23% w/w of danegaptide or a pharmaceutically acceptable salt thereof, between about 0% and about 7.5% w/w of PEG3350 or a substantially similar polymer, between about 25% and about 35% w/w of R202S or a substantially similar polymer, and between about 45% and about 55% of RG752S or a substantially similar polymer. In one embodiment, the implant consists of about 24% w/w of danegaptide or a pharmaceutically acceptable salt thereof, between about 0% and about 7.5% w/w of PEG3350 or a substantially similar polymer, between about 25% and about 35% w/w of R202S or a substantially similar polymer, and between about 45% and about 55% of RG752S or a substantially similar polymer. In one embodiment, the implant consists of about 25% w/w of danegaptide or a pharmaceutically acceptable salt thereof, between about 0% and about 7.5% w/w of PEG3350 or a substantially similar polymer, between about 25% and about 35% w/w of R202S or a substantially similar polymer, and between about 45% and about 55% of RG752S or a substantially similar polymer.

In some embodiments, an implant can be optimized according to the following non-limiting examples in Table 8 and Table 9:

| **Table 8** | | | | | | | | | | |
|---|---|---|---|---|---|---|---|---|---|---|
| | | | | | **Test Combinations (% w/w)** | | | | | |
| | | | | | **1** | **2** | **3** | **4** | **5** | **6** |
| **Polymer Type (First Biodegradable Polymer)** | **IV midpoint (dl/g)** | **End type** | | API (base) | 20 | 20 | 20 | 20 | 20 | 20 |
| | | | | PEG3350 | 5 | | 5 | | | 5 |
| Poly (DL-lactide) | 0.2 dl/g | Acid terminated (~H) | R203H | | | | | | | |
| | | | **R202H** | | 15 | | | | | |
| Poly (DL-lactide) | 0.2 dl/g | Ester (~S) | R202S | | | | | | 30 | 30 |
| | | | **R203S** | | 20 | | | | | |
| Poly (DL-lactide) | 0.4 dl/g | Acid terminated | | | | | | | | |
| Poly (DL-lactide) | 0.4 dl/g | | | | | | | | | |
| Poly (DL-lactide) | 0.5 dl/g | | | | | | | | | |
| Poly (DL-lactide) | 0.6 dl/g * | | | | | | | | | |
| | | | | | | | | | | |

| **Polymer Type (Second Biodegradable Polymer)** | **IV midpoint** | **End type** | | | | | | | | |
|---|---|---|---|---|---|---|---|---|---|---|
| | | | | | | | | | | |
| 50/50 DL-lactide/glycolide copolymer | 0.2 dl/g | Acid terminated | RG502H | | | 40 | | | | |
| 50/50 DL-lactide/glycolide copolymer | 0.2 dl/g * | Acid terminated | | | | | | | | |
| 50/50 DL-lactide/glycolide copolymer | 0.2 dl/g | Ester | RG502 | | | 40 | | | | |
| 50/50 DL-lactide/glycolide copolymer | 0.4 dl/g | Acid terminated | RG504H | | | | | | | |
| 50/50 DL-lactide/glycolide copolymer | 0.4 dl/g | | | | | | | | | |
| 50/50 DL-lactide/glycolide copolymer | 0.8 dl/g * | | | | | | | | | |
| 50/50 DL-lactide/glycolide copolymer | 1.0 dl/g | | | | | | | | | |
| 55/45 DL-lactide/glycolide copolymer | 0.5 dl/g | | | | | | | | | |
| 75/25 DL-lactide/glycolide copolymer | 0.16 dl/g | Acid terminated | | | | | | | | |
| 75/25 DL-lactide/glycolide copolymer | 0.2 dl/g | Acid terminated | RH752H | | | | | | | |
| 75/25 DL-lactide/glycolide copolymer | 0.2 dl/g | Ester (~S) | RG752S | | 40 | | | 30 | 50 | |
| 75/25 DL-lactide/glycolide copolymer | 0.4 dl/g | Acid terminated | | | | | | | | |
| 75/25 DL-lactide/glycolide copolymer | 0.4 dl/g | | | | | | | | | |
| 75/25 DL-lactide/glycolide copolymer | 0.7 dl/g * | | | | | | | | | |
| 75/25 DL-lactide/glycolide copolymer | 0.7 dl/g | Ester (~S) | RG755S | | | | 75 | 50 | | 45 |
| 75/25 DL-lactide/Glycolide copolymer | 0.9 dl/g * | Ester (~S) | RG756S | | | | | | | |
| 75/25 DL-lactide/Glycolide copolymer | 0.9 dl/g * | | | | | | | | | |
| 75/25 DL-lactide/glycolide copolymer | 1.0 dl/g * | | | | | | | | | |
| 85/15 DL-lactide/Glycolide copolymer | 0.3 dl/g * | Ester (~S) | RG858S | | | | | | | |
| 85/15 DL-lactide/Glycolide copolymer | 0.5 dl/g * | Acid terminated | | | | | | | | |

| **Table 9** | | | | | | | | | | |
|---|---|---|---|---|---|---|---|---|---|---|
| | | | | | **Test Combinations (% w/w)** | | | | | |
| | | | | | **A** | **B** | **C** | **D** | **E** | **F** |
| **Polymer Type (First Biodegradable Polymer)** | **IV midpoint (dl/g)** | **End type** | | API (base) | 11-29% | 11-29% | 11-29% | 11-29% | 11-29% | 11-29% |
| | | | | PEG | 0-7.5% | 0-7.5% | 0-7.5% | 0-7.5% | 0-7.5% | 0-7.5% |
| Poly (DL-lactide) | 0.1-0.3 dl/g | Acid terminated (^{~}H) | R203H | | | 10-20% | | | | |
| | | | **R202H** | | 10-20% | | | | | |
| Poly (DL-lactide) | 0.1-0.3 dl/g | Ester (~S) | R202S | | | 15-25% | 25-35% | | 25-35% | |
| | | | **R203S** | | 15-25% | | | 25-35% | | 25-35% |

| **Polymer Type (Second Biodegradable Polymer)** | **IV midpoint** | **End type** | | | | | | | | |
|---|---|---|---|---|---|---|---|---|---|---|
| | | | | | | | | | | |
| 75/25 DL-lactide/glycolide copolymer | 0.1-0.3 dl/g | Ester (~S) | RG752S | | 35-45% | 35-45% | 45-55% | 45-55% | 45-55% | 45-55% |

### Examples

### Example 1: Preparation of formulations by Hot Melt Extrusion (HME)

Biodegradable implants are made by combining danegaptide with a biodegradable polymer composition in a mixer equipment. If needed the particle size of drug and polymer can be reduced in mixer such as cryo mill.

The mixed powder blend is the heated to a semi-molten state at specified temperature and time, forming a polymer/drug melt. The melting/mixing can also be performed in such equipped hot melt extruders.

Rods are manufactured by pelletizing the polymer/drug melt using a 9 gauge polytetrafluoroethylene (PTFE) tubing, loading the pellet into the extruder barrel and extruding the material at the specified core extrusion temperature into filaments.

The filaments are then cut to specified length with the extrusion equipment Microspheres are manufactured by dissolving one or more polymers in solvent such as dichloromethane (CH₂Cl₂). Danegaptide is dissolved in a suitable solvent and the polymer solution was mixed with the danegaptide solution. The phase containing danegaptide and polymer are dispersed into polyvinyl alcohol (PVA) dissolved in water, using a Silverson Lab mixer. The organic solvents are extracted and evaporated by increasing the temperature to approximately 42° C and stirred. The microspheres were obtained by filtration, washed, and then freeze-dried.

### Example 2: Dissolution profile of formulations

For each implant 1) the rate of compound released from the implant is measured in vitro over time into a release medium consisting of 0.01 M phosphate buffered saline (PBS), pH 7.4, at 37 °C. (calculated and plotted for replicate implants as the percent average total compound release over time), 2) the degree and extent of implant swelling is measured over time in 0.01 M phosphate buffered saline (PBS), pH 7.4 at 37 °C., and 3) the rate of implant degradation in 0.01 M phosphate buffered saline (PBS), pH 7.4 at 37 °C. The estimated *in vitro* lifetime of an implant is determined for each implant formulation based on the degradation results. For *in vivo* testing the implants are placed in a vial and sterilized by a gamma radiation dose of 25 to 40 kGy.

*In vitro* polymer degradation testing is performed by incubating implants in 0.01 M PBS pH 7.4 in a shaking water bath set at 37° C. For each formulation, 20 implants are incubated in triplicates for a duration of 8 weeks. Samples are taken weekly. The peak molecular weight (MW) is determined using a GPC equipped with an R.I. detector and polystyrene as a standard.

For an implant swelling study, each implant is incubated in approximately 400 uL of PBS (pH 7.4, 0.01 M) in Microwell 96-Well Assay Plates and placed in a Shake N Bake Hybridization Oven set at 37° C. and 50 rpm. Implant images are recorded at 150x magnification at each time point and the lengths and diameters are measured by Keyence Digital Microscope. Images are recorded at initialization, then weekly for the first month, and biweekly thereafter.

### Example 3: Process for Making Danegaptide Implants

The anti-arrhythmic peptide analogue, danegaptide, was incorporated into sustained release polymeric implants made by a high temperature (85 to 155 °C.) melt extrusion process. The implants made comprised from 12 wt % to 25 wt % danegaptide and from 75 wt % to 88 wt % poly(D,L,-lactide-co-glycolide) polymer (a PLGA).

The implants were made at a temperature high enough to melt or soften the PLGAs, yet low enough to avoid substantial loss of danegaptide potency. The solubility parameters of the danegaptide and the PLGA polymer used were similar so that the danegaptide was soluble in the polymer thereby resulting in a solid solution at the temperature used. An extruded implant made from a solid solution of a therapeutic agent and a polymeric carrier can provide a more uniform and reproducible release profile of the therapeutic agent, as compared to an extruded implant where the danegaptide is present as a solid dispersion in the polymeric carrier.

The polymer implants were made by melt extrusion in a piston driven extruder or Daca extruder/microcompounder. The implants are rod-shaped, but can be made in any geometric shape simply by changing the extrusion die.

The polymers were used as received from Boehringer Ingelheim or Evonik and the danegaptide was used as received from Carbogen Amcis (Bubendorf, Switzerland). To make an implant the polymer and danegaptide were combined (see Table 7) in a Retsch ball-mill capsule with a 1/4" stainless steel ball, and then the capsule was placed in the Retsch mill (Type MM200) for 5 min at 20-cycles/min. The capsule was then removed from the mill and the powder blend was stirred with a spatula. The capsule with the powder blend was mixed for 5 minutes on a Turbula mixer. The powder blend was inspected for homogeneity and the mixing procedure is repeated if necessary.

A steel powder funnel and a spatula were used to transfer the powder blend to an extruder barrel mounted in a pneumatic compaction press. A small amount of powder blend was added to the extruder barrel and the powder was compacted with the press set at 50 psi. The powder-blend loaded barrel was placed in the extruder and allowed to equilibrate to a temperature of 145-155° C. The filaments were extruded at 0.0025"/sec through a 720-micron circular die to form the rod-shaped implant. The extruded filaments were smooth and had a consistent diameter. The Implant formulations made are shown in Table 7.

The filaments were cut into rods (approximately 8 mm long) and their drug release over time monitored in phosphate buffered saline pH 7.4.

### Example 4: Extrusion and Compression Processes for Making Danegaptide Implants

Danegaptide is combined with a biodegradable polymer composition in a mortar. The combination is mixed with a shaker set at about 96 RPM for about 15 minutes. The powder blend is scraped off the wall of the mortar and is then remixed for an additional 15 minutes. The mixed powder blend is heated to a semi-molten state at specified temperature for a total of 30 minutes, forming a polymer/drug melt.

Rods are manufactured by pelletizing the polymer/drug melt using a 9 gauge polytetrafluoroethylene (PTFE) tubing, loading the pellet into the barrel and extruding the material at the specified core extrusion temperature into filaments. The filaments are then cut into about 1 mg size implants or drug delivery systems. The rods may have dimensions of about 2 mm long×0.72 mm diameter. Such rod implants weigh between about 900 µg and 1100 µg. Alternative rod sizes have dimensions of 3-6 mm long × 0.37-0.45 mm diameters. Wafers are formed by flattening the polymer melt with a Carver press at a specified temperature and cutting the flattened material into wafers, each weighing about 1 mg. The wafers have a diameter of about 2.5 mm and a thickness of about 0.13 mm. The wafer implants weigh between about 900 µg and 1100 µg.

*In-vitro* release testing is performed by placing each implant into a 24-mL screw cap vial with 10 mL of Phosphate Buffered Saline solution at 37° C. 1 mL aliquots are removed and are replaced with equal volume of fresh medium on day 1, 4, 7, 14, 28, and every two weeks thereafter.

Drug assays are performed by RP-HPLC, applying an Agilent 1100 System equipped with a PDA Detector (detection 225 nm; scan 190-380 nm). A YMC Pack Pro C18, 3.0 µm, 4.6×150 mm column is used for separation, run at 40 °C using gradient elution with a flow rate of 0.8 mL/min and a run time of 45 minutes per sample. The mobile phase A is (935:5:40:20) water:1M ammonium formate:methanol:acetonitrile, and the mobile phase B is (10:5:700:285) water:1M ammonium formate:methanol:acetonitrile. The gradient elution is programmed as 0% B for 15 min followed by a linear gradient from 0 to 67%B over 20 min, remaining for 0.1 min at 67% and finally a gradient back to 0% over 9.9 min to give a total run time of 45 min.

The release rates are determined by calculating the amount of drug being released in a given volume of medium over time and is reported as µg released per day.

Polymers which may be used in the implants can be obtained from Boehringer Ingelheim and Evonik. Examples of polymer include: RG502, RG502H, RG752, R202H, R203 and R206, and Purac PDLG (50/50). RG502 and RG502H are (50:50) poly(D,L-lactide-co-glycolide) with RG502 having an ester end group and RG502H having an acid end group, RG752 is (75:25) poly(D,L-lactide-co-glycolide), R202H is 100% poly(D, L-lactide) with acid end group or terminal acid groups, R203 and R206 are both 100% poly(D, L-lactide). Purac PDLG (50/50) is (50:50) poly(D,L-lactide-co-glycolide). The inherent viscosity of RG502, RG502H, RG752, R202H, R203, R206, and Purac PDLG are 0.2, 0.2, 0.2, 0.2, 0.3, 1.0, and 0.2 dL/g, respectively. The average molecular weight of RG502, RG502H, RG752, R202H, R203, R206, and Purac PDLG are, 11700, 11200, 11200, 6500, 14000, 63300, and 9700 daltons, respectively. The implants made can be suitable for intraocular use to treat an ocular condition.

### Example 5: Melting Process for Screening of Danegaptide Implant Formulations

Biodegradable polymer compositions taken from the list in Table 7 and Example 4, are gently heated until melting point and danegaptide is added under stirring. Concentration of danegaptide can be varied in the range from 5% to 50% dependent on solubility. The different polymer/drug melts are then casted into dies of diameters from 0.5 mm to 2.0 mm and cooled. After cooling the polymer/drug filaments are pressed out of the die and cut into rods of defined length.

The prepared danegaptide/polymer rods are stored at accelerated conditions (40 °C /75 % RH and 60 °C open) and analysed to select most compatible Biodegradable polymer compositions.

*In-vitro* release testing, including compound release profiles from the prepared danegaptide/polymer rods are tested in Phosphate Buffered Saline solution at 37 °C using an appropriate *in vitro* release method, such as an USP 2 method). Sampling time points can be varied after the expected release rate and thereby the total release time.

### Example 6: Danegaptide Formulations

Described below is the dissolution behavior of Danegaptide when formulated in 6 different melt extrusion formulations. The formulations were manufactured with a drug load of about 20%. The formulations were exposed to physiological salt solutions at 37 °C and stored for several months. The media was changed regularly and analyzed for content of Danegaptide. Also, a visual evaluation was done to follow the dissolution process of the extrudate.

The investigated formulations turned out to be very different in their dissolution profiles. Formulations 1 and 5 were found to have a prolonged release over several months, and thus confirmed the capability of the systems to be clinically relevant for the further development as an ophthalmological dosage form containing Danegaptide.

### Introduction

Several delivery strategies are being considered for Danegaptide as a therapy to treat and reverse diabetic retinopathy. As a viable option, a tiny needle formed rod is inserted directly into the eye and delivers the active Danegaptide over prolonged time. In addition to a small needle formed shape, another important requirement for such a product is that the drug product needs to show almost linear delivery to the eye over several months to be feasible for the patient and doctors.

Danegaptide as a HCL salt is water-soluble. To investigate the viability of this drug, a melt extrusion formulation strategy was chosen, and six different formulations based on various viable excipients feasible for the melt extrusion process were manufactured with approximately 20% drug load.

The dissolution process was investigated for each of the 6 manufactured prototypes by inserting the prototypes into a 0.9% NaCl solution and test the dissolution of Danegaptide over time by HPLC. The experiment was conducted over 170 days.

### Materials and Methods

The custom-built extruder allowed for working with small amounts of APIs and polymers, while still being easily scaled to a production scale extruder.

5 g of the Danegaptide blends (Table 10) were fed into a 7.5 mm 16:1 hot melt extruder at a rate of 140 g per hour. The screw speed of the extruder was maintained at 60 RPM for all formulations while the feed zone, mixing zone, die zone and product temperatures as well as the torque and die pressure were varied for each blend as per Table 11. The extrudate was hauled off through a 2 mm die and the diameter of the extrudate was controlled via changes in the haul of speed. The extrudate was inspected and those areas cylindrical in shape, with a continuous surface, no deformation and 0.5 mm + 0.1 mm diameter were cut into implants of 4 mm + 0.4 mm in length.

Except for PEG3350, the excipient code names shown in Table 10 (e.g., "R203S") refer to types of bioabsorbable poly (DL-lactide) or poly(lactide-co-glycolide) (PLG) polymeric excipients, being marketed by Evonik under their registered brand name Resomer^{®}. Other poly(DL-lactide) or poly(lactide-co-glycolide) polymers are known in the art and/or available commercially.

**Table 10. Composition of formulations no. 1 to 6 manufactured at Extruded Pharmaceuticals.**

| **Formulation** | **Composition *** | **Composition **** | **Excipient Ratio ^{#}** |
|---|---|---|---|
| | (w/w) | (w/w) | (w/w) |
| **1** | 20% R203S, | 19.1% R203S | 4 |
| | 40% RG752S, | 38.2% RG752S | 8 |
| | 15% R202H, | 14.3% R202H | 3 |
| | 5% PEG 3350, | 4.8 % PEG 3350 | 1 |
| | 20% Danegaptide | 23.7% Danegaptide (HCl, H₂O) | |
| **2** | 40% RG502, | 38.2% RG502, | 1 |
| | 40% RG502H, | 38.2% RG502H, | 1 |
| | 20% Danegaptide | 23.7% Danegaptide (HCl, H₂O) | |
| **3** | 75% 755S, | 71.5% 755S, | 15 |
| | 5% PEG3350, | 4.8% PEG 3350, | 1 |
| | 20% Danegaptide | 23.7% Danegaptide (HCl, H₂O) | |
| **4** | 30% 752S, | 28.6% 752S, | 3 |
| | 50% 755S, | 47.7% 755S, | 5 |
| | 20% Danegaptide | 23.7% Danegaptide (HCl, H₂O) | |
| **5** | 30% R202S, | 28.6% R202S, | 3 |
| | 50% 752S, | 47.7% 752S, | 5 |
| | 20% Danegaptide | 23.7% Danegaptide (HCl, H₂O) | |
| **6** | 30% R202S, | 28.6% R202S, | 6 |
| | 45% 755S, | 42.9% 755S, | 9 |
| | 5% PEG 3350, | 4.8% PEG 3350, | 1 |
| | 20% Danegaptide | 23.7% Danegaptide (HCl, H₂O) | |

| | | | |
|---|---|---|---|
| *) when considering Danegaptide as base-form (Mw: 291.30) **) when considering (and using) Danegaptide in the present format = "HCl salt, monohydrate" (Mw: 345.78) #) The w/w ratios between the involved excipients are identical in each of the two composition columns | | | |

| **Table 11. Manufacturing parameters used for manufacture of formulation no. 1 to 6.** | | | | | | |
|---|---|---|---|---|---|---|
| **Formulation** | **Feed zone temp (°C)** | **Mixing zone temp (°C)** | **Die Zone temp (°C)** | **Product temp (°C)** | **Torque (Nm)** | **Die pressure (PSI)** |
| 1 | 75 | 100 | 90 | 85 | 1.44 | 25 |
| 2 | 83 | 110 | 100 | 84 | 1.62 | 22 |
| 3 | 95.5 | 130 | 120 | 113 | 1.80 | 23 |
| 4 | 91 | 125 | 115 | 106 | 1.80 | 30 |
| 5 | 75 | 100 | 90 | 85 | 1.62 | 27 |
| 6 | 83 | 110 | 100 | 70 | 1.80 | 26 |

### Potency of prototype formulations no. 1 to 6.

Five rods of each of the 6 prototypes were weighed in a 10 ml volumetric flask. A volume of 2 ml of acetonitrile was added and the formulations were dissolved to form a slightly opal solution. The flasks were taken to volume with water. After 2 hours, the solutions were filtered and diluted 1:1 in mobile phase and analyzed by HPLC and the potency of the formulations were determined.

The potency is given in Table 12 below.

**Table 12. Potency of formulations no. 1 to 6**

| **Prototype Formulation** | **Potency %** |
|---|---|
| 1 | 20.8 |
| 2 | 20.5 |
| 3 | 18.7 |
| 4 | 20.4 |
| 5 | 20.5 |
| 6 | 19.5 |

### Dissolution testing of prototype formulations

Fifteen dose units of each prototype was weighed and were wrapped into a permeable filter bag consisting of empty triangular tea bags made of polymeric material, with a mesh size of less than 0.5 µm. The bags were each inserted into glass vials and 10 ml 0.9% NaCl solution media were added to each vial. The vials were placed in a water bath at 37 °C. At regular intervals the media was drained and 10 ml new media was added and the vials were returned to the water bath. Sampling times were frequent in the beginning and were gradually prolonged. One day delivery periods were also tested several times during the dissolution period. The vials were not stirred, however, the small vibrations from the water bath were believed to mechanically promote the dissolution process to some extent. The drained medium was subjected to HPLC analysis for a potency determination. In addition, an informative impurity profile was recorded. In relation to this, the pH value of each collected media sample was determined. When the dissolution profile became flat, the experiment was stopped and only formulations with a slow release was followed for the full nearly half year period.

To visually follow the dissolution process, few rods of each product were added to a petri dish and a few ml of salt solution (0.9% NaCl) was added and subsequently stored under the same conditions as the dissolution study vials (i.e., 37 °C). A photo was recorded at regular intervals for documentation.

### HPLC testing of prototypes and their solutions

The HPLC system consisted of a YMC Pack Pro C18, 150x4.6 mm, 3 µm HPLC column. The mobile phase was delivered isocratic with a composition of Milli Q water/ 1M ammonium formate buffer/methanol/ acetonitrile at 935/5/40/20. The column was kept at 40 °C and at a flow of 1.0 ml/min. 20 µl was injected and the eluate tested at 225 nm UV detection. Run time was 15 min.

The Danegaptide content was quantified to a Danegaptide reference substance. Impurities were also reported as area % of the active. The impurities were a mixture of contaminants from the materials and of degradation products. The profiles were not evaluated in detail but degradation of Danegaptide during the period probably only happened to a smaller extent.

### Results and Discussion

The results of the dissolution characteristics are given below for the 6 individual prototypes

### Formulation 1

### Appearance during the dissolution process

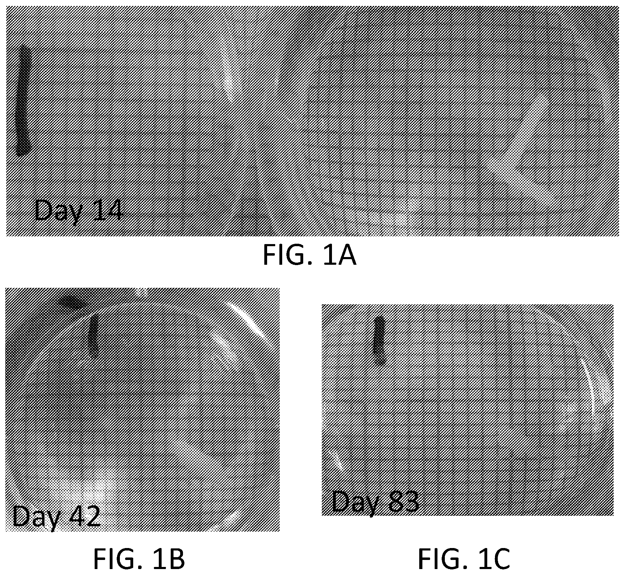

The appearance of the extruded rods of formulation 1 over time was studied visually on the days of sample pull. Suitable photos were taken and kept as raw data. After 40 days the rods started rapidly to disintegrate, becoming a gel lump after 83 days. Figs. 1A- 1C illustrate the development after 14 days up to 83 days.

### Dissolution in saline over time as percent of original content

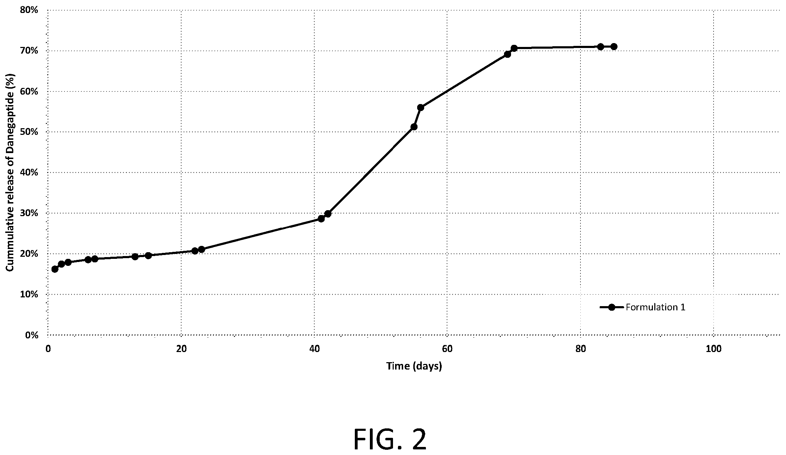

The dissolved amount of Danegaptide in percent of total amount in formulation is given in Table 13 and graphically shown in Fig. 2.

**Table 13. Amount dissolved of formulation 1 in the dissolution process, Day 1-101.**

| | | | Day | Total amount (mg) | Total amount (%) |
|---|---|---|---|---|---|
| Sample | Date | pH | | | |
| Day 1 | 24-nov | 3.77 | 1 | 0.851 | 16.2% |
| Day 2 | 25-nov | 5.81 | 2 | 0.918 | 17.5% |
| Day 3 | 26-nov | 6.08 | 3 | 0.939 | 17.9% |
| Day 3-6 | 29-nov | 5.51 | 6 | 0.974 | 18.6% |
| Day 7 | 30-nov | 6.16 | 7 | 0.983 | 18.7% |
| Day 7-13 | 06-dec | 5.84 | 13 | 1.012 | 19.3% |
| Day 14-15 | 08-dec | 5.84 | 15 | 1.026 | 19.6% |
| Day 15-22 | 15-dec | 5.36 | 22 | 1.086 | 20.7% |
| Day 23 | 16-dec | 6.11 | 23 | 1,.06 | 21.1% |
| Day 23-41 | 03-jan | 3.91 | 41 | 1.504 | 28.7% |
| Dag 42 | 04-jan | 4.98 | 42 | 1.566 | 29.9% |
| Day 42-55 | 17-feb | 3.15 | 55 | 2.685 | 51.2% |
| Day 56 | 18-jan | 3.63 | 56 | 2.938 | 56.0% |
| Day 56-69 | 31-jan | 2.97 | 69 | 3.624 | 69.1% |
| Day 70 | 01-feb | 3.56 | 70 | 3.703 | 70.6% |
| Day 70-83 | 14-feb | 3.03 | 83 | 3.721 | 71.0% |
| Day 83-85 | 16-feb | 3.52 | 85 | 3.723 | 71.0% |

### Formulation 2

### Appearance during the dissolution process

The appearance of the extruded rods of formulation 2 over time was studied visually on the days of sample pull. Suitable photos were taken and kept as raw data.

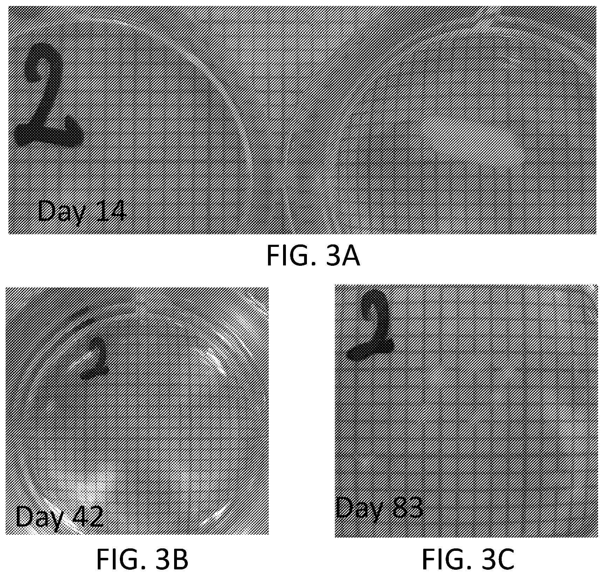

After 14 days the rods became swollen and started to glue together. After 42 days and 83 days, only a small gel lumps were left. Figs. 3A- 3C illustrate the development.

### Dissolution in saline over time as percent of original content

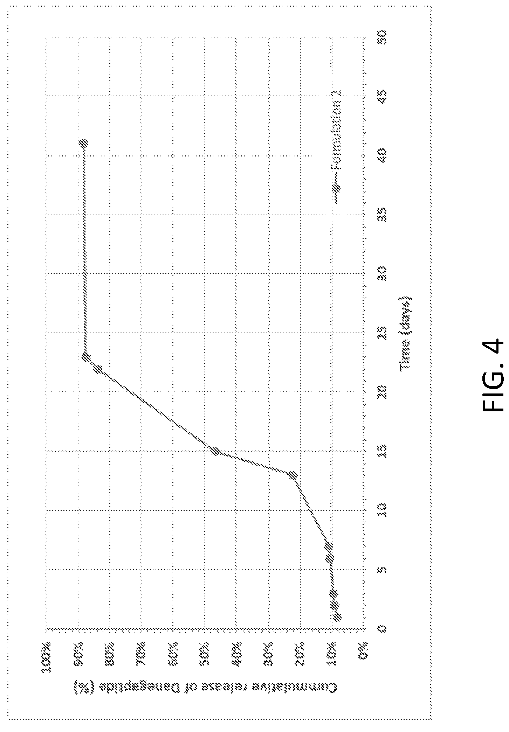

The dissolved amount of Danegaptide in percent of total amount in formulation 2 is given in Table 14 and graphically shown in Fig. 4.

**Table 14. Amount dissolved of formulation 2 in the dissolution process. Day 1-41.**

| | | | Day | Total amount (mg) | Total amount (%) |
|---|---|---|---|---|---|
| Sample | Date | pH | | | |
| Day 1 | 24-nov | 4.91 | 1 | 0.309 | 8.1% |
| Day 2 | 25-nov | 6.2 | 2 | 0.349 | 9.2% |
| Day 3 | 26-nov | 6.15 | 3 | 0.359 | 9.4% |
| Day 3-6 | 29-nov | 4.58 | 6 | 0.400 | 10.5% |
| Day 7 | 30-nov | 5.29 | 7 | 0.418 | 11.0% |
| Day 7-13 | 06-dec | 3.43 | 13 | 0.857 | 22.5% |
| Day 14-15 | 08-dec | 3.31 | 15 | 1.778 | 46.6% |
| Day 15-22 | 15-dec | 2.89 | 22 | 3.201 | 83.9% |
| Day 23 | 16-dec | 3.4 | 23 | 3.346 | 87.7% |
| Day 23-41 | 03-jan | 2.97 | 41 | 3.367 | 88.3% |

### Formulation 3

### Appearance during the dissolution process

The appearance of the extruded rods of formulation 3 over time was studied visually on the days of sample pull. Suitable photos were taken and kept as raw data.

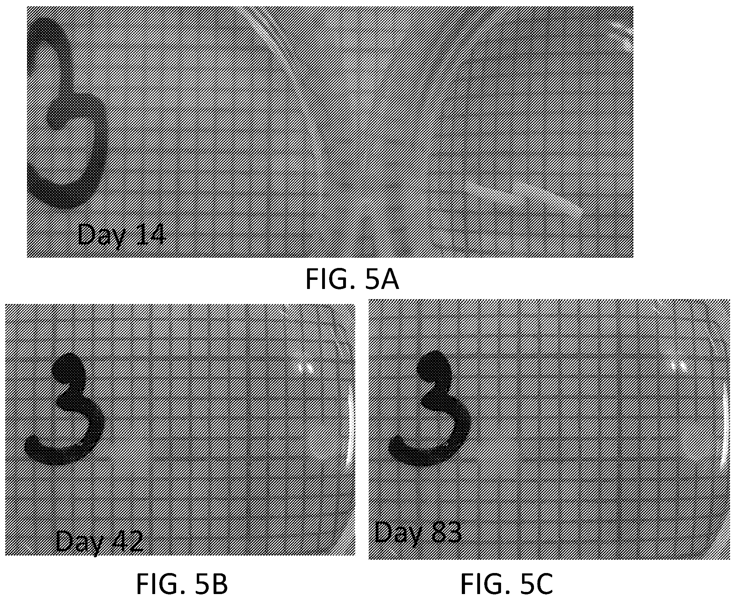

After 14 days, the rods kept their rod form, but were slightly swollen. After 42 days, only gel lumps were left and were still visible after 83 days. Figs. 5A- 5C illustrate the development.

### Dissolution of formulation 3 over time in saline

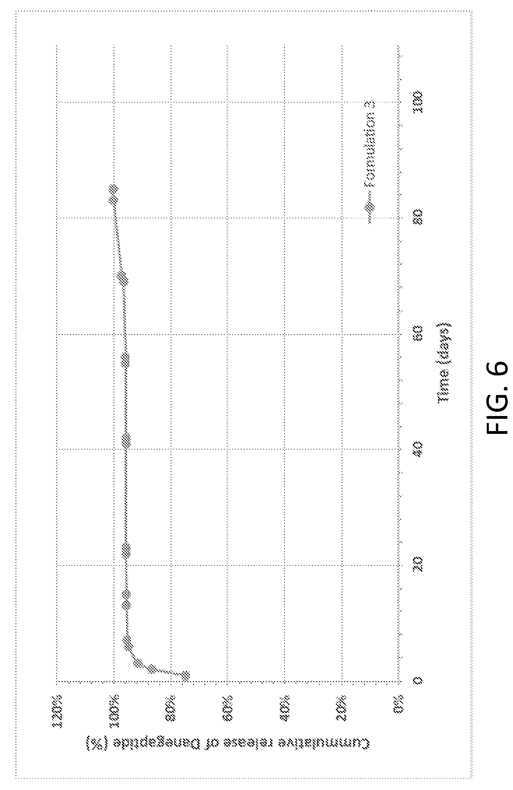

The dissolved amount of Danegaptide in percent of total amount in formulation 3 is given in Table 15 and graphically shown in Fig. 6.

**Table 15. Amount dissolved of formulation 3 in the dissolution process. Day 1-85.**

| | | | Day | Total amount (mg) | Total amount (%) |
|---|---|---|---|---|---|
| Sample | Date | pH | | | |
| Day 1 | 24-nov | 3.51 | 1 | 2.610 | 74.8% |
| Day 2 | 25-nov | 4.37 | 2 | 3.030 | 86.8% |
| Day 3 | 26-nov | 4.78 | 3 | 3,.197 | 91.6% |
| Day 3-6 | 29-nov | 5.06 | 6 | 3.317 | 95.1% |
| Day 7 | 30-nov | 6.21 | 7 | 3.329 | 95.4% |
| Day 7-13 | 06-dec | 6.72 | 13 | 3.340 | 95.7% |
| Day 13-15 | 08-dec | 6.59 | 15 | 3.342 | 95.8% |
| Day 15-22 | 15-dec | 6.66 | 22 | 3.343 | 95.8% |
| Day 23 | 16-dec | 6.44 | 23 | 3.343 | 95.8% |
| Day 23-41 | 03-jan | 6.21 | 41 | 3.345 | 95.9% |
| Day 42 | 04-jan | 6.16 | 42 | 3.346 | 95.9% |
| Day 42-55 | 17-feb | 5.91 | 55 | 3.350 | 96.0% |
| Day 56 | 18-jan | 6.24 | 56 | 3.351 | 96.0% |
| Day 56-69 | 31-jan | 3.73 | 69 | 3.377 | 96.8% |
| Day 70 | 01-feb | 4.09 | 70 | 3.399 | 97.4% |
| Day 70-83 | 14-feb | 3.16 | 83 | 3.498 | 100.2% |
| Day 83-85 | 16-feb | 3.56 | 85 | 3.505 | 100.4% |

### Formulation 4

### Appearance during the dissolution process

The appearance of the extruded rods of formulation 4 over time was studied visually on the days of sample pull. Suitable photos were taken and kept as raw data.

After 14 days the rods kept their rod form but became slightly swollen and deformed. After 42 days the rods kept their appearance, however, after 83 days only a white round lump was left and was gradually disappearing by 108 days.

### Dissolution of formulation 4 over time in saline

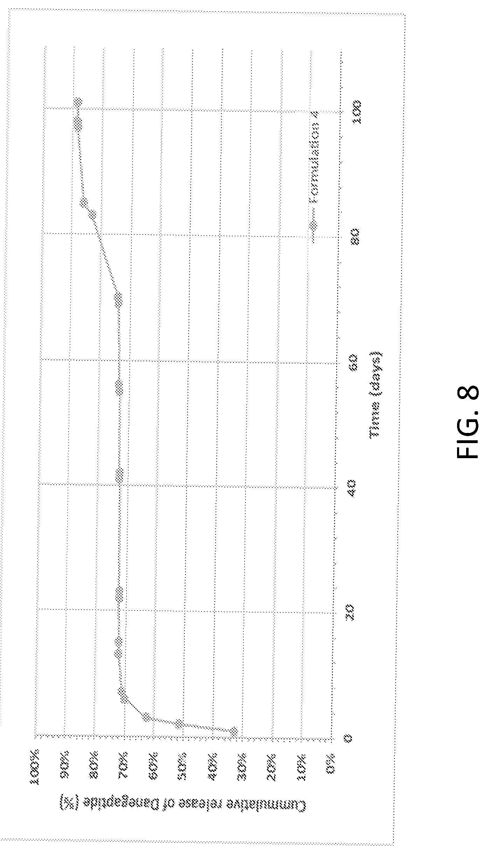

The dissolved amount of Danegaptide in percent of total amount in formulation 4 is given in Table 16 and graphically shown in Fig. 8.

**Table 16. Amount dissolved of formulation 4 in the dissolution process. Day 1-85.**

| | | | Day | Total amount (mg) | Total amount (%) |
|---|---|---|---|---|---|
| Sample | Date | pH | | | |
| Day 1 | 24-nov | 3.71 | 1 | 1.398 | 33.0% |
| Day 2 | 25-nov | 4.01 | 2 | 2.180 | 51.4% |
| Day 3 | 26-nov | 4.29 | 3 | 2.654 | 62.6% |
| Day 3-6 | 29-nov | 4.67 | 6 | 2.971 | 70.1% |
| Day 7 | 30-nov | 6.26 | 7 | 3.013 | 71.1% |
| Day 7-13 | 06-dec | 5.99 | 13 | 3.060 | 72.2% |
| Day 14-15 | 08-dec | 6.29 | 15 | 3.062 | 72.3% |
| Day 15-22 | 15-dec | 6.47 | 22 | 3.066 | 72.4% |
| Day 23 | 16-dec | 6.40 | 23 | 3.067 | 72.4% |
| Day 23-41 | 03-jan | 6.27 | 41 | 3.083 | 72.8% |
| Day 42 | 04-jan | 6.32 | 42 | 3.084 | 72.8% |
| Day 42-55 | 17-feb | 6.20 | 55 | 3.099 | 73.1% |
| Day 56 | 18-jan | 6.78 | 56 | 3.111 | 73.4% |
| Day 56-69 | 31-jan | 5.09 | 69 | 3.130 | 73.9% |
| Day 70 | 01-feb | 5.91 | 70 | 3.142 | 74.1% |
| Day 70-83 | 14-feb | 3.46 | 83 | 3.525 | 83.2% |
| Day 84-85 | 16-feb | 3.79 | 85 | 3.652 | 86.2% |

### Formulation 5

### Appearance during the dissolution process:

The appearance of the extruded rods of formulation 5 over time was studied visually on the days of sample pull. Suitable photos were taken and kept as raw data.

After 14 days the rods kept their rod form but became slightly swollen and deformed. After 42 days the rods kept their appearance, however, after 83 days only a white round lump was left and was gradually disappearing by 108 days.

### Dissolution of formulation 5 over time in saline

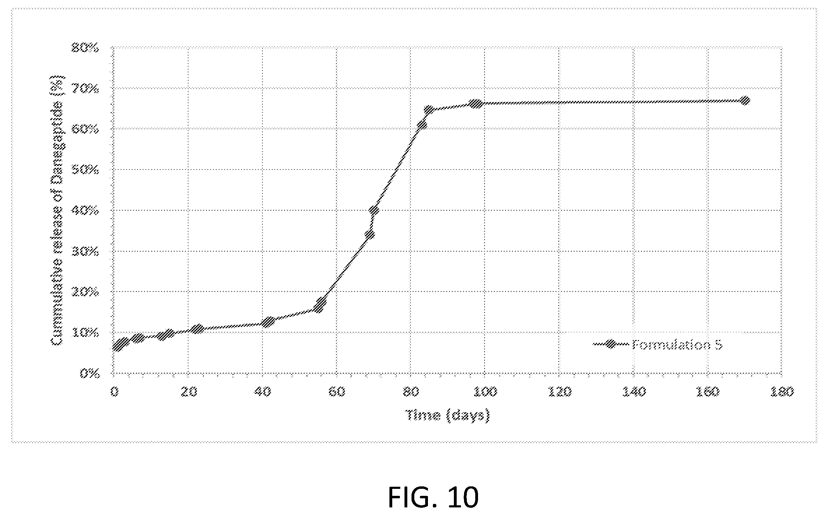

The dissolved amount of Danegaptide in percent of total amount in formulation 5 is given in Table 17 and graphically shown in Fig. 10.

**Table 17. Amount Danegaptide dissolved from formulation 5 over time. Day 1-170.**

| | | | Day | Total amount (mg) | Total amount (%) |
|---|---|---|---|---|---|
| Sample | Date | pH | | | |
| Day 1 | 24-nov | 5.09 | 1 | 0.234 | 6.5% |
| Day 2 | 25-nov | 6.34 | 2 | 0.264 | 7.3% |
| Day 3 | 26-nov | 5.81 | 3 | 0.281 | 7.8% |
| Day 3-6 | 29-nov | 6.25 | 6 | 0.308 | 8.6% |
| Day 7 | 30-nov | 6.03 | 7 | 0.316 | 8.8% |
| Day 7-13 | 06-dec | 6.01 | 13 | 0.329 | 9.2% |
| Day 14-15 | 08-dec | 6.64 | 15 | 0.354 | 9.8% |
| Day 15-22 | 15-dec | 6.06 | 22 | 0.386 | 10.7% |
| Day 23 | 16-dec | 6.79 | 23 | 0.392 | 10.9% |
| Day 23-41 | 03-jan | 5.53 | 41 | 0.442 | 12.3% |
| Day 42 | 04-jan | 6.08 | 42 | 0.463 | 12.9% |
| Day 42-55 | 17-feb | 4.72 | 55 | 0.570 | 15.9% |
| Day 56 | 18-jan | 5.33 | 56 | 0.632 | 17.6% |
| Day 56-69 | 31-jan | 3.88 | 69 | 1.220 | 33.9% |
| Day 70 | 01-feb | 4.4 | 70 | 1.436 | 40.0% |
| Day 70-83 | 14-feb | 3.5 | 83 | 2.194 | 61.1% |
| Day 83-85 | 16-feb | 3.97 | 85 | 2.324 | 64.7% |
| Day 85-97 | 28-feb | 3.48 | 97 | 2.380 | 66.2% |
| Day 98 | 01-mar | 4.06 | 98 | 2.384 | 66.3% |
| Day 99-170 | 12-maj | 4.27 | 170 | 2.407 | 67.0% |

### Formulation 6

### Appearance during the dissolution process

The appearance of the extruded rods of formulation 6 over time was studied visually on the days of sample pull. Suitable photos were taken and kept as raw data.

After 14 days the rods kept their rod form but became slightly swollen and deformed. After 42 days the rods kept their appearance, however, after 83 days only a white round lump was left and was gradually disappearing by 108 days.

### Dissolution of formulation 6 over time in saline

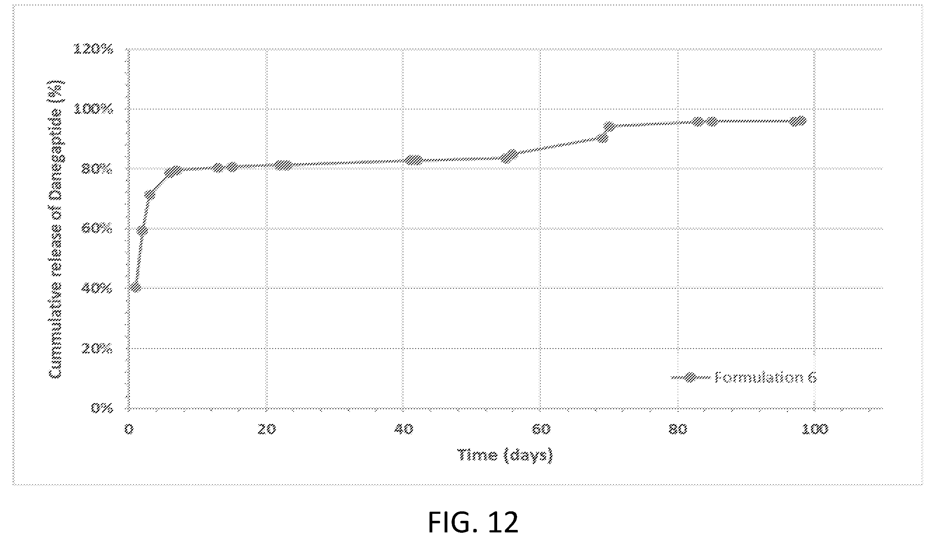

The dissolved amount of Danegaptide in percent of total amount in formulation 6 is given in Table 18 and graphically shown in Fig. 12.

**Table 18 Amount dissolved of formulation 6 in the dissolution process. Day 1-98.**

| | | | Day | Total amount (mg) | Total amount (%) |
|---|---|---|---|---|---|
| Sample | Date | pH | | | |
| Day 1 | 24-nov | 3.87 | 1 | 1.553 | 40.4% |
| Day 2 | 25-nov | 4.16 | 2 | 2.285 | 59.5% |
| Day 3 | 26-nov | 4.42 | 3 | 2.734 | 71.2% |
| Day 3-6 | 29-nov | 4.58 | 6 | 3.024 | 78.7% |
| Day 7 | 30-nov | 5.94 | 7 | 3.055 | 79.5% |
| Day 7-13 | 06-dec | 4.9 | 13 | 3.089 | 80.4% |
| Day 14-15 | 08-dec | 5.3 | 15 | 3.096 | 80.6% |
| Day 15-22 | 15-dec | 4.64 | 22 | 3.120 | 81.2% |
| Day 23 | 16-dec | 5.93 | 23 | 3.123 | 81.3% |
| Day 23-41 | 03-jan | 4.29 | 41 | 3.180 | 82.8% |
| Day 42 | 04-jan | 5.82 | 42 | 3.183 | 82.8% |
| Day 42-55 | 17-feb | 4.18 | 55 | 3.207 | 83.5% |
| Day 56 | 18-jan | 4.92 | 56 | 3.260 | 84.9% |
| Day 56-69 | 31-jan | 3.65 | 69 | 3.469 | 90.3% |
| Day 70 | 01-feb | 3.96 | 70 | 3.621 | 94.3% |
| Day 70-83 | 14-feb | 3.4 | 83 | 3.678 | 95.7% |
| Day 84-85 | 16-feb | 3.84 | 85 | 3.685 | 95.9% |
| Day 85-97 | 28-feb | 3.57 | 97 | 3.688 | 96.0% |
| Day 98 | 01-mar | 4.29 | 98 | 3.689 | 96.0% |

### Conclusion

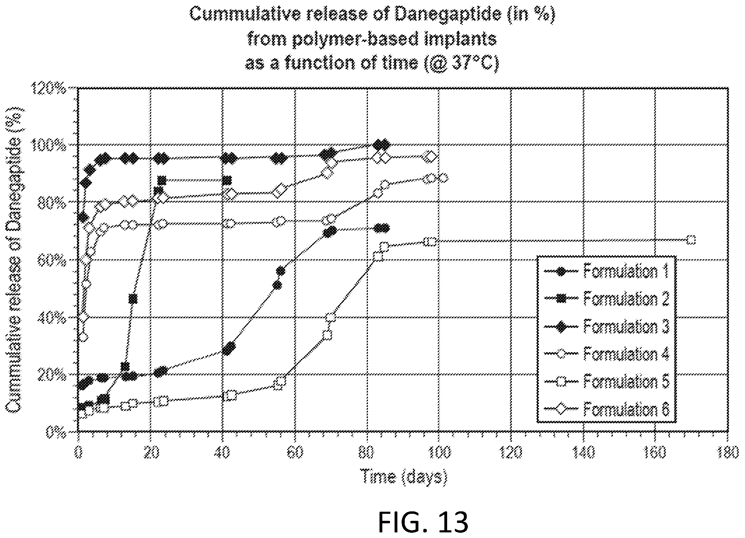

The dissolution characteristics for the 6 prototype formulations are given in Fig. 13. It is concluded that the 6 different melt extrusion formulation numbers 1 to 6 show very different dissolution pattern, and thus the formulations are sensitive to the formulation composition. Formulations 3, 4 and 6 rapidly gave up the Danegaptide content, whereas formulation 1 and formulation 5 showed a dissolution pattern releasing the Danegaptide over several months and are thus good candidates for an ophthalmic formulation.

In some embodiments, there is a tendency that slow releasing formulations do not completely liberate all Danegaptide as given by the determined potency. While not wishing to be bound by a particular theory, this could be due to some strong binding of Danegaptide residues to the remaining excipients or could be due to slow degradation of Danegaptide over the prolonged storage of the dosage form at 37 °C. HPLC chromatograms did not indicate a significant degradation pattern.

## Claims

1. A biodegradable intraocular implant comprising:
a hydrophilic compound of formula (I), 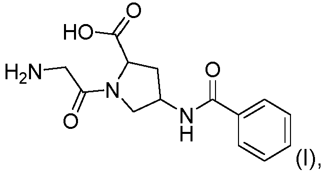
or a hydrophilic pharmaceutically acceptable salt thereof;
and an extended-release or sustained release biodegradable polymer matrix;
wherein the biodegradable polymer matrix comprises a biodegradable polymer selected from the group consisting of a polylactic acid (PLA) polymer, a polyglycolic acid (PGA) polymer, polylactide-co-glycolide (PLGA) polymer, and copolymers thereof;
wherein the pharmaceutically acceptable salt is selected from the group consisting of: a hydrochloride salt, a maleic acid salt, an acetic acid salt, a tartaric acid salt, and a pamoic acid salt; and
wherein the biodegradable polymer matrix comprises a mixture of two or more biodegradable polymers comprising a first biodegradable polymer and a different second biodegradable polymer.

2. The implant of claim 1, wherein the first biodegradable polymer is a polylactic acid polymer, preferably Poly(D,L-lactide), optionally wherein the Poly(D,L-lactide) is selected from the list consisting of:
Poly(D,L-lactide), Acid end group (R 202 H);
Poly(D,L-lactide), Ester end group (R 202 S);
Poly(D,L-lactide), Acid end group (R 203 H);
Poly(D,L-lactide), Ester end group (R 203 S); and
Poly(D,L-lactide), Ester end group (R 205 H).

3. The implant of claims 1 or 2, wherein the second biodegradable polymer is a polylactide-co-glycolide polymer, preferably Poly(D,L-lactide-co-glycolide), optionally
(i) Poly(D,L-lactide-co-glycolide) 75:25 which comprises an average molecular weight of 4,000-15,000 g/mol; or
(ii) RG752H or RG752S.

4. The implant of any of claims 1 to 3, wherein the biodegradable polymer matrix further comprises the polymer Poly(D,L-lactide), Ester end group (R 203 S).

5. The implant of any one of claims 1 to 4, wherein the compound is:

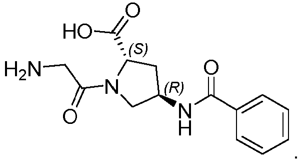

6. The implant of any one of claims 1 to 5, wherein the compound is dispersed within the biodegradable polymer matrix.

7. The implant of any of claims 1 to 6, wherein the first biodegradable polymer has an inherent viscosity of from about 0.1 dl/g to about 0.7 dl/g and/or wherein the second biodegradable polymer has an inherent viscosity of from about 0.1 dl/g to about 0.7 dl/g.

8. The implant of any one of claims 1 to 7, wherein the matrix further comprises at least one PLGA or PLA polymer selected from the group consisting of:
Poly(D,L-lactide-co-glycolide) lactide:glycolide (50:50) with an average molecular weight (avg. MW) of 30,000-60,000 g/mol;
Poly(D,L-lactide-co-glycolide) lactide:glycolide 50:50 with avg. MW of 45,000 g/mol;
Poly(D,L-lactide-co-glycolide) lactide:glycolide 50:50, acid and hydroxy terminated with avg. MW of 25,000 g/mol;
Poly(D,L-lactide-co-glycolide) lactide:glycolide (50:50), ester terminated with avg. MW of 100,000 g/mol;
Poly(D,L-Lactide-co-Glycolide) lactide:glycolide 50:50, ester terminated with avg. MW of 7,000-17,000 g/mol;
Poly(D,L-lactide-co-glycolide) acid terminated with avg. MW of 7,000-17,000 g/mol;
Poly(D,L-lactide-co-glycolide) lactide:glycolide 50:50, ester terminated with avg. MW of 24,000-38,000 g/mol;
Poly(D,L-lactide-co-glycolide) acid terminated, lactide:glycolide 50:50 with avg. MW of 24,000-38,000 g/mol;
Poly(D,L-lactide-co-glycolide) lactide:glycolide 50:50, ester terminated with avg. MW of 38,000-54,000 g/mol;
Poly(D,L-lactide-co-glycolide) acid terminated, lactide:glycolide 50:50 with avg. MW of 38,000-54,000 g/mol;
Poly(D,L-lactide-co-glycolide) ester terminated with avg. MW of 54,000-69,000 g/mol;
Poly(D,L-lactide-co-glycolide) lactide:glycolide 65:35 with avg. MW of 40,000-75,000 g/mol;
Poly(L-lactide-co-glycolide) lactide:glycolide 65:35, viscosity 0.6 dL/g;
Poly(D,L-lactide-co-glycolide) acid terminated with avg. MW of 24,000-38,000 g/mol;
Poly(D,L-lactide-co-glycolide) lactide:glycolide (75:25) with avg. MW of 66,000-107,000 g/mol;
Poly(D,L-lactide-co-glycolide) acid terminated lactide:glycolide 75:25 with avg. MW of 6,000-10,000 g/mol;
Poly(D,L-lactide-co-glycolide) ester terminated, lactide:glycolide 75:25;
Poly(D,L-lactide-co-glycolide) ester terminated, lactide:glycolide 75:25 with avg. MW of 4,000-15,000 g/mol;
Poly(D,L-lactide-co-glycolide) acid terminated, lactide:glycolide 75:25 with avg. MW of 4,000-15,000 g/mol;
Poly(D,L-lactide-co-glycolide) ester terminated, Lactide: Glycolide 75:25 with avg. MW of 66,000 -107,000 g/mol;
Poly(D,L-lactide-co-glycolide) acid terminated (RG 753 H) with avg. MW of 66,000 -107,000 g/mol;
Poly(D,L-lactide-co-glycolide) ester terminated (RG 755 S);
Poly(D,L-lactide-co-glycolide) ester terminated, lactide:glycolide 75:25 with avg. MW of 76,000-115,000 g/mol;
Poly(D,L-lactide-co-glycolide) ester terminated, Lactide: Glycolide 75:25 (RG 757 S);
Poly(D,L-lactide-co-glycolide) ester terminated with avg. MW of 50,000-75,000 g/mol;
Poly(D,L-lactide-co-glycolide)-COOH, lactide:glycolide 85:15 with avg. MW of 17,000 g/mol;
Poly(D,L-lactide-co-glycolide) ester terminated lactide:glycolide 80:20 with avg. MW of 200,000;
Poly(L-lactide-co-glycolide) ester terminated, lactide:glycolide 85:15 (LG 855 S);
Poly(L-lactide-co-glycolide) ester terminated, lactide:glycolide 85:15 (LG 857 S);
Poly(D,L-lactide-co-glycolide) ester terminated, lactide:glycolide 85:15 (RG 858 S) with avg. MW of 190,000-240,000 g/mol;
Poly(D,L-lactide), Acid end group (R 202 H);
Poly(D,L-lactide), Ester end group (R 202 S);
Poly(D,L-lactide), Acid end group (R 203 H);
Poly(D,L-lactide), Ester end group (R 203 S); and
Poly(D,L-lactide), Ester end group (R 205 H).

9. The implant of any one of claims 1 to 8, wherein
(i) the implant comprises from about 2 to about 35 w/w% of the compound; and/or
(ii) the implant comprises from about 2 to about 25 w/w% of the compound, and the biodegradable polymer matrix comprises a combination of two different polylactide polymers, preferably wherein the combination of two different polylactide polymers comprises two different PLGAs, two different PLAs, or a PLGA and a PLA; and/or
(iii) the matrix comprises a mixture of biodegradable polymers, and at least one of the biodegradable polymers is a polylactide having a molecular weight of from about 6 kDa to about 120 kDa; and/or
(iv) wherein the matrix comprises a second polymer of a polylactide having a molecular weight of from about 6 kDa to about 82 kDa.

10. The implant of any one of claims 1 to 9, wherein the implant comprises from about 1 µg to about 75 µg, about 5 µg to about 50 µg or about 5 µg, about 6 µg, about 7 µg, about 8 µg, about 9 µg, about 10 µg, about 11 µg, about 12 µg, about 13 µg, about 14 µg, about 15 µg, about 16 µg, about 17 µg, about 18 µg, about 19 µg, about 20 µg, about 21 µg, about 22 µg, about 23 µg, about 24 µg, about 25 µg, about 26 µg, about 27 µg, about 28 µg, about 29 µg, about 30 µg, about 31 µg, about 32 µg, about 33 µg, about 34 µg, about 35, about 36 µg, about 37 µg, about 38 µg, about 39 µg, or about 40 µg of the compound or a pharmaceutically acceptable salt thereof.

11. The implant of any one of claims 1 to 10, wherein the implant is configured to be placed in the vitreous of the eye or other posterior segments of the eye, optionally wherein.
(i) the compound is released at a rate effective to sustain release of the compound for more than one week from the time the implant is placed in the vitreous of the eye;
(ii) the compound is released at a rate effective to sustain release of the compound for more than one month from the time the implant is placed in the vitreous of the eye or other posterior segments of the eye; and/or.
wherein upon placement in the vitreous of the eye or other posterior segments of the eye, the implant maintains a concentration of at least 50 nM of the compound in the retinal tissue, or wherein upon placement in the vitreous of the eye or other posterior segments of the eye, the implant maintains a concentration of from 50 nM to 125 nM of the compound in the retinal tissue once a steady state equilibrium has been reached.

12. The implant of claim 11, wherein upon placement in the vitreous of the eye or other posterior segments of the eye, the implant is configured to release from about 0.1 µg per day to about 2 µg per day of the compound or a pharmaceutically acceptable salt thereof.

13. The implant of claims 11 or 12, wherein upon placement in the vitreous of the eye or other posterior segments of the eye, the implant is configured to provide an initial burst release of the compound or a pharmaceutically acceptable salt thereof followed by a lower steady state concentration of the compound or a pharmaceutically acceptable salt thereof, preferably wherein the initial burst release provides a concentration of from about 10 to 50 ng/mL of the compound or a pharmaceutically acceptable salt thereof in the ocular compartment, and optionally wherein
(i) the lower steady state concentration of the compound or a pharmaceutically acceptable salt thereof is from about 50 nM to about 125 nM steady state concentration; and/or
(ii) wherein the initial burst release provides a dose of from about 150 to about 300 ng of the compound or a pharmaceutically acceptable salt thereof over two days from the time the implant is placed in the vitreous of the eye.

14. The implant of any one of claims 11 to 13, wherein upon placement the vitreous of the eye or other posterior segments of the eye, the implant is configured to release the compound or a pharmaceutically acceptable salt thereof for at a period of time of least one month to three months.

15. The implant of any one of claims 1 to 14, wherein
the implant comprises between about 11% and 29% w/w of the compound or a pharmaceutically acceptable salt thereof, between about 0% and about 7.5% w/w of a PEG polymer, between about 15% and about 25% w/w of R203S or a substantially similar polymer, between about 10% and about 20% w/w of R202H or a substantially similar polymer, and between about 35% and about 45% of RG752S or a substantially similar polymer; and/or
wherein the implant comprises between about 11% and 29% w/w of the compound or a pharmaceutically acceptable salt thereof, between about 0% and about 7.5% w/w of a PEG polymer, between about 25% and about 35% w/w of R202S or a substantially similar polymer, and between about 45% and about 55% of RG752S or a substantially similar polymer.

## Patentansprüche

1. Biologisch abbaubares intraokulares Implantat, umfassend:
eine hydrophile Verbindung der Formel (I), 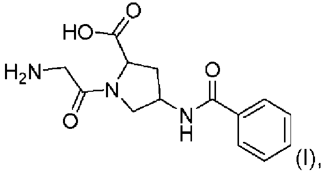
oder ein hydrophiles, pharmazeutisch annehmbares Salz davon;
und eine biologisch abbaubare Polymermatrix mit verzögerter Freisetzung oder anhaltender Freisetzung;
wobei die biologisch abbaubare Polymermatrix ein biologisch abbaubares Polymer umfasst, das aus der Gruppe ausgewählt ist, die aus einem Polymilchsäure-(PLA)-Polymer, einem Polyglykolsäure-(PGA)-Polymer, Polylactid-co-Glycolid-(PLGA)-Polymer und Copolymeren davon besteht;
wobei das pharmazeutisch annehmbare Salz aus der Gruppe ausgewählt ist, die aus Folgendem besteht: einem Hydrochloridsalz, einem Maleinsäuresalz, einem Essigsäuresalz, einem Weinsäuresalz und einem Pamoasäuresalz; und
wobei die biologisch abbaubare Polymermatrix eine Mischung aus zwei oder mehr biologisch abbaubaren Polymeren umfasst, die ein erstes biologisch abbaubares Polymer und ein anderes, zweites biologisch abbaubares Polymer umfasst.

2. Implantat nach Anspruch 1, wobei das erste biologisch abbaubare Polymer ein Polymilchsäurepolymer ist, bevorzugt Poly(D,L-Lactid), wobei das Poly(D,L-Lactid) wahlweise aus der Liste ausgewählt ist, die aus Folgendem besteht:
Poly(D,L-Lactid), Säureendgruppe (R 202 H);
Poly(D,L-Lactid), Esterendgruppe (R 202 S);
Poly(D,L-Lactid), Säureendgruppe (R 203 H);
Poly(D,L-Lactid), Esterendgruppe (R 203 S); und
Poly(D,L-Lactid), Esterendgruppe (R 205 H).

3. Implantat nach den Ansprüchen 1 oder 2, wobei das zweite biologisch abbaubare Polymer ein Polylactid-co-Glycolid-Polymer ist, bevorzugt Poly(D,L-Lactid-co-Glycolid), wahlweise
(i) Poly(D,L-Lactid-co-Glycolid) im Verhältnis 75:25, das ein durchschnittliches Molekulargewicht von 4.000-15.000 g/mol umfasst; oder
(ii) RG752H oder RG752S.

4. Implantat nach einem der Ansprüche 1 bis 3, wobei die biologisch abbaubare Polymermatrix weiter das Polymer Poly(D,L-Lactid), Esterendgruppe (R 203 S), umfasst.

5. Implantat nach einem der Ansprüche 1 bis 4, wobei die Verbindung wie folgt ist:

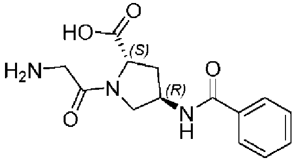

6. Implantat nach einem der Ansprüche 1 bis 5, wobei die Verbindung in der biologisch abbaubaren Polymermatrix dispergiert ist.

7. Implantat nach einem der Ansprüche 1 bis 6, wobei das erste biologisch abbaubare Polymer eine inhärente Viskosität von etwa 0,1 dl/g bis etwa 0,7 dl/g aufweist und/oder wobei das zweite biologisch abbaubare Polymer eine inhärente Viskosität von etwa 0,1 dl/g bis etwa 0,7 dl/g aufweist.

8. Implantat nach einem der Ansprüche 1 bis 7, wobei die Matrix weiter mindestens ein PLGA- oder PLA-Polymer umfasst, das aus der Gruppe ausgewählt ist, die aus Folgendem besteht:
Poly(D,L-Lactid-co-Glycolid) Lactid:Glycolid (50:50) mit einem durchschnittlichen Molekulargewicht (durchschnittliches MW) von 30.000-60.000 g/mol;
Poly(D,L-Lactid-co-Glycolid) Lactid:Glycolid 50:50 mit durchschnittlichem MW von 45.000 g/mol;
Poly(D,L-Lactid-co-Glycolid) Lactid:Glycolid 50:50, säure- und hydroxyterminiert, mit durchschnittlichem MW von 25.000 g/mol;
Poly(D,L-Lactid-co-Glycolid) Lactid:Glycolid (50:50), esterterminiert, mit durchschnittlichem MW von 100.000 g/mol;
Poly(D,L-Lactid-co-Glycolid) Lactid:Glycolid 50:50, esterterminiert, mit durchschnittlichem MW von 7.000-17.000 g/mol;
Poly(D,L-Lactid-co-Glycolid), säureterminiert, mit durchschnittlichem MW von 7.000-17.000 g/mol;
Poly(D,L-Lactid-co-Glycolid) Lactid:Glycolid 50:50, esterterminiert, mit durchschnittlichem MW von 24.000-38.000 g/mol;
Poly(D,L-Lactid-co-Glycolid), säureterminiert, Lactid:Glycolid 50:50 mit durchschnittlichem MW von 24.000-38.000 g/mol;
Poly(D,L-Lactid-co-Glycolid) Lactid:Glycolid 50:50, esterterminiert, mit durchschnittlichem MW von 38.000-54.000 g/mol;
Poly(D,L-Lactid-co-Glycolid), säureterminiert, Lactid:Glycolid 50:50 mit durchschnittlichem MW von 38.000-54.000 g/mol;
Poly(D,L-Lactid-co-Glycolid), esterterminiert, mit durchschnittlichem MW von 54.000-69.000 g/mol;
Poly(D,L-Lactid-co-Glycolid) Lactid:Glycolid 65:35 mit durchschnittlichem MW von 40.000-75.000 g/mol;
Poly(L-Lactid-co-Glycolid) Lactid:Glycolid 65:35, Viskosität 0,6 dL/g;
Poly(D,L-Lactid-co-Glycolid), säureterminiert, mit durchschnittlichem MW von 24.000-38.000 g/mol;
Poly(D,L-Lactid-co-Glycolid) Lactid:Glycolid (75:25) mit durchschnittlichem MW von 66.000-107.000 g/mol;
Poly(D,L-Lactid-co-Glycolid), säureterminiert, Lactid:Glycolid 75:25 mit durchschnittlichem MW von 6.000-10.000 g/mol;
Poly(D,L-Lactid-co-Glycolid), esterterminiert, Lactid:Glycolid 75:25;
Poly(D,L-Lactid-co-Glycolid), esterterminiert, Lactid:Glycolid 75:25 mit durchschnittlichem MW von 4.000-15.000 g/mol;
Poly(D,L-Lactid-co-Glycolid), säureterminiert, Lactid:Glycolid 75:25 mit durchschnittlichem MW von 4.000-15.000 g/mol;
Poly(D,L-Lactid-co-Glycolid), esterterminiert, Lactid: Glycolid 75:25 mit durchschnittlichem MW von 66.000-107.000 g/mol;
Poly(D,L-Lactid-co-Glycolid), säureterminiert (RG 753 H), mit durchschnittlichem MW von 66.000 - 107.000 g/mol;
Poly(D,L-Lactid-co-Glycolid), esterterminiert (RG 755 S);
Poly(D,L-Lactid-co-Glycolid), esterterminiert, Lactid:Glycolid 75:25 mit durchschnittlichem MW von 76.000-115.000 g/mol;
Poly(D,L-Lactid-co-Glycolid), esterterminiert, Lactid: Glycolid 75:25 (RG 757 S);
Poly(D,L-Lactid-co-Glycolid), esterterminiert, mit durchschnittlichem MW von 50.000-75.000 g/mol;
Poly(D,L-Lactid-co-Glycolid)-COOH, Lactid:Glycolid 85:15 mit durchschnittlichem MW von 17.000 g/mol;
Poly(D,L-Lactid-co-Glycolid), esterterminiert, Lactid:Glycolid 80:20 mit durchschnittlichem MW von 200.000;
Poly(L-Lactid-co-Glycolid), esterterminiert, Lactid:Glycolid 85:15 (LG 855 S);
Poly(L-Lactid-co-Glycolid), esterterminiert, Lactid:Glycolid 85:15 (LG 857 S);
Poly(D,L-Lactid-co-Glycolid), esterterminiert, Lactid:Glycolid 85:15 (RG 858 S) mit durchschnittlichem MW von 190.000-240.000 g/mol;
Poly(D,L-Lactid), Säureendgruppe (R 202 H);
Poly(D,L-Lactid), Esterendgruppe (R 202 S);
Poly(D,L-Lactid), Säureendgruppe (R 203 H);
Poly(D,L-Lactid), Esterendgruppe (R 203 S); und
Poly(D,L-Lactid), Esterendgruppe (R 205 H).

9. Implantat nach einem der Ansprüche 1 bis 8, wobei
(i) das Implantat etwa 2 bis etwa 35 Gew.-% der Verbindung umfasst; und/oder
(ii) das Implantat etwa 2 bis etwa 25 Gew.-% der Verbindung umfasst, und die biologisch abbaubare Polymermatrix eine Kombination aus zwei verschiedenen Polymilchsäurepolymeren umfasst, wobei die Kombination aus zwei verschiedenen Polymilchsäurepolymeren bevorzugt zwei verschiedene PLGAs, zwei verschiedene PLAs oder eine PLGA und eine PLA umfasst; und/oder
(iii) die Matrix eine Mischung aus biologisch abbaubaren Polymeren umfasst, und mindestens eines der biologisch abbaubaren Polymere ein Polylactid ist, das ein Molekulargewicht von etwa 6 kDa bis etwa 120 kDa aufweist; und/oder
(iv) wobei die Matrix ein zweites Polymer eines Polylactids umfasst, das ein Molekulargewicht von etwa 6 kDa bis etwa 82 kDa aufweist.

10. Implantat nach einem der Ansprüche 1 bis 9, wobei das Implantat etwa 1 µg bis etwa 75 µg, etwa 5 µg bis etwa 50 µg oder etwa 5 µg, etwa 6 µg, etwa 7 µg, etwa 8 µg, etwa 9 µg, etwa 10 µg, etwa 11 µg, etwa 12 µg, etwa 13 µg, etwa 14 µg, etwa 15 µg, etwa 16 µg, etwa 17 µg, etwa 18 µg, etwa 19 µg, etwa 20 µg, etwa 21 µg, etwa 22 µg, etwa 23 µg, etwa 24 µg, etwa 25 µg, etwa 26 µg, etwa 27 µg, etwa 28 µg, etwa 29 µg, etwa 30 µg, etwa 31 µg, etwa 32 µg, etwa 33 µg, etwa 34 µg, etwa 35, etwa 36 µg, etwa 37 µg, etwa 38 µg, etwa 39 µg oder etwa 40 µg der Verbindung oder eines pharmazeutisch annehmbaren Salzes davon umfasst.

11. Implantat nach einem der Ansprüche 1 bis 10, wobei das Implantat ausgebildet ist, im Glaskörper des Auges oder in anderen hinteren Segmenten des Auges platziert zu werden, wobei wahlweise.
(i) die Verbindung mit einer Rate freigesetzt wird, so dass eine Freisetzung der Verbindung für mehr als eine Woche ab dem Zeitpunkt der Platzierung des Implantats im Glaskörper des Auges anhält;
(ii) die Verbindung mit einer Rate freigesetzt wird, so dass eine Freisetzung der Verbindung für mehr als einen Monat ab dem Zeitpunkt der Platzierung des Implantats im Glaskörper des Auges oder in anderen hinteren Segmenten des Auges anhält; und/oder.
wobei das Implantat nach der Platzierung im Glaskörper des Auges oder in anderen hinteren Segmenten des Auges eine Konzentration von mindestens 50 nM der Verbindung im Netzhautgewebe aufrechterhält, oder wobei das Implantat nach der Platzierung im Glaskörper des Auges oder in anderen hinteren Segmenten des Auges eine Konzentration von 50 nM bis 125 nM der Verbindung im Netzhautgewebe aufrechterhält, sobald ein Fließgleichgewicht erreicht ist.

12. Implantat nach Anspruch 11, wobei das Implantat nach der Platzierung im Glaskörper des Auges oder in anderen hinteren Segmenten des Auges ausgebildet ist, um etwa 0,1 µg pro Tag bis etwa 2 µg pro Tag der Verbindung oder eines pharmazeutisch annehmbaren Salzes davon freizusetzen.

13. Implantat nach den Ansprüchen 11 oder 12, wobei das Implantat nach der Platzierung im Glaskörper des Auges oder in anderen hinteren Segmenten des Auges ausgebildet ist, eine anfängliche schnelle Freisetzung der Verbindung oder eines pharmazeutisch annehmbaren Salzes davon bereitzustellen, gefolgt von einer niedrigeren Steady-State-Konzentration der Verbindung oder eines pharmazeutisch annehmbaren Salzes davon,
wobei die anfängliche schnelle Freisetzung bevorzugt eine Konzentration von etwa 10 bis 50 ng/ml der Verbindung oder eines pharmazeutisch annehmbaren Salzes davon im okulären Kompartiment bereitstellt, und wobei wahlweise
(i) die niedrigere Steady-State-Konzentration der Verbindung oder eines pharmazeutisch annehmbaren Salzes davon bei einer Steady-State-Konzentration von etwa 50 nM bis etwa 125 nM liegt; und/oder
(ii) wobei die anfängliche schnelle Freisetzung eine Dosis von etwa 150 bis etwa 300 ng der Verbindung oder eines pharmazeutisch annehmbaren Salzes davon über zwei Tage ab dem Zeitpunkt bereitstellt, an dem das Implantat im Glaskörper des Auges platziert wird.

14. Implantat nach einem der Ansprüche 11 bis 13, wobei das Implantat nach der Platzierung im Glaskörper des Auges oder in anderen hinteren Segmenten des Auges ausgebildet ist, die Verbindung oder ein pharmazeutisch annehmbares Salz davon für einen Zeitraum von mindestens einem Monat bis zu drei Monaten freizusetzen.

15. Implantat nach einem der Ansprüche 1 bis 14, wobei
das Implantat zwischen etwa 11 Gew.-% und 29 Gew.-% der Verbindung oder eines pharmazeutisch annehmbaren Salzes davon, zwischen etwa 0 Gew.-% und etwa 7,5 Gew.-% eines PEG-Polymers, zwischen etwa 15 Gew.-% und etwa 25 Gew.-% R203S oder eines im Wesentlichen ähnlichen Polymers, zwischen etwa 10 Gew.-% und etwa 20 Gew.-% R202H oder eines im Wesentlichen ähnlichen Polymers sowie zwischen etwa 35 % und etwa 45 % RG752S oder eines im Wesentlichen ähnlichen Polymers umfasst; und/oder
wobei das Implantat zwischen etwa 11 Gew.-% und 29 Gew.-% der Verbindung oder eines pharmazeutisch annehmbaren Salzes davon, zwischen etwa 0 Gew.-% und etwa 7,5 Gew.-% eines PEG-Polymers, zwischen etwa 25 Gew.-% und etwa 35 Gew.-% R202S oder eines im Wesentlichen ähnlichen Polymers sowie zwischen etwa 45 % und etwa 55 % RG752S oder eines im Wesentlichen ähnlichen Polymers umfasst.

## Revendications

1. Implant intraoculaire biodégradable comprenant :
un composé hydrophile de formule (I), 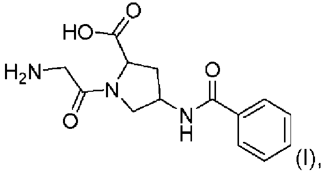
ou un sel hydrophile pharmaceutiquement acceptable de celui-ci ;
et une matrice polymère biodégradable à libération prolongée ou à libération soutenue ;
dans lequel la matrice polymère biodégradable comprend un polymère biodégradable sélectionné dans le groupe consistant en un polymère d'acide polylactique (PLA), un polymère d'acide polyglycolique (PGA), un polymère de polylactide-co-glycolide (PLGA), et les copolymères de ceux-ci ;
dans lequel le sel pharmaceutiquement acceptable est sélectionné dans le groupe consistant en : un sel d'hydrochlorure, un sel d'acide maléique, un sel d'acide acétique, un sel d'acide tartrique, et un sel d'acide pamoïque ; et
dans lequel la matrice polymère biodégradable comprend un mélange de deux polymères biodégradables ou plus comprenant un premier polymère biodégradable et un deuxième polymère biodégradable différent.

2. Implant selon la revendication 1, dans lequel le premier polymère biodégradable est un polymère d'acide polylactique, préférablement poly(D,L-lactide), facultativement dans lequel le poly(D,L-lactide) est sélectionné dans la liste consistant en :
poly(D,L-lactide), groupe terminal acide (R 202 H) ;
poly(D,L-lactide), groupe terminal ester (R 202 S) ;
poly(D,L-lactide), groupe terminal acide (R 203 H) ;
poly(D,L-lactide), groupe terminal ester (R 203 S) ; et
poly(D,L-lactide), groupe terminal ester (R 205 H).

3. Implant selon les revendications 1 ou 2, dans lequel le deuxième polymère biodégradable est un polymère polylactide-co-glycolide, préférablement poly(D,L-lactide-co-glycolide), facultativement
(i) poly(D,L-lactide-co-glycolide) 75:25 qui comprend un poids moléculaire moyen de 4000 à 15 000 g/mol ; ou
(ii) RG752H ou RG752S.

4. Implant selon l'une quelconque des revendications 1 à 3, dans lequel la matrice polymère biodégradable comprend en outre le polymère poly(D,L-lactide), groupe terminal ester (R 203 S).

5. Implant selon l'une quelconque des revendications 1 à 4, dans lequel le composé est :

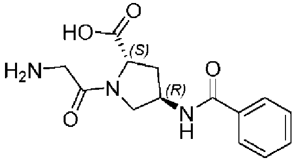

6. Implant selon l'une quelconque des revendications 1 à 5, dans lequel le composé est dispersé dans la matrice polymère biodégradable.

7. Implant selon l'une quelconque des revendications 1 à 6, dans lequel le premier polymère biodégradable présente une viscosité intrinsèque d'environ 0,1 dl/g à environ 0,7 dl/g et/ou dans lequel le deuxième polymère biodégradable présente une viscosité intrinsèque d'environ 0,1 dl/g à environ 0,7 dl/g.

8. Implant selon l'une quelconque des revendications 1 à 7, dans lequel la matrice comprend en outre au moins un polymère PLGA ou PLA sélectionné dans le groupe consistant en :
poly(D,L-lactide-co-glycolide) lactide:glycolide (50:50) avec un poids moléculaire moyen (PM moy.) de 30 000 à 60 000 g/mol ;
poly(D,L-lactide-co-glycolide) lactide:glycolide 50:50 avec un PM moy. de 45 000 g/mol ;
poly(D,L-lactide-co-glycolide) lactide:glycolide 50:50, terminé par un acide et hydroxy avec un PM moy. de 25 000 g/mol ;
poly(D,L-lactide-co-glycolide) lactide:glycolide (50:50), terminé par un ester avec un PM moy. de 100 000 g/mol ;
poly(D,L-lactide-co-glycolide) lactide:glycolide 50:50, terminé par un ester avec un PM moy. de 7000 à 17 000 g/mol ;
poly(D,L-lactide-co-glycolide) terminé par un acide avec un PM moy. de 7000 à 17 000 g/mol ; poly(D,L-lactide-co-glycolide) lactide:glycolide 50:50, terminé par un ester avec un PM moy. de 24 000 à 38 000 g/mol ;
poly(D,L-lactide-co-glycolide) terminé par un acide, lactide:glycolide 50:50 avec un PM moy. de 24 000 à 38 000 g/mol ;
poly(D,L-lactide-co-glycolide) lactide:glycolide 50:50, terminé par un ester avec un PM moy. de 38 000 à 54 000 g/mol ;
poly(D,L-lactide-co-glycolide) terminé par un acide, lactide:glycolide 50:50 avec un PM moy. de 38 000 à 54 000 g/mol ;
poly(D,L-lactide-co-glycolide) terminé par un ester avec un PM moy. de 54 000 à 69 000 g/mol ;
poly(D,L-lactide-co-glycolide) lactide:glycolide 65:35 avec un PM moy. de 40 000 à 75 000 g/mol ;
poly(L-lactide-co-glycolide) lactide:glycolide 65:35, viscosité 0,6 dl/g ;
poly(D,L-lactide-co-glycolide) terminé par un acide avec un PM moy. de 24 000 à 38 000 g/mol ;
poly(D,L-lactide-co-glycolide) lactide:glycolide (75:25) avec un PM moy. de 66 000 à 107 000 g/mol ;
poly(D,L-lactide-co-glycolide) terminé par un acide lactide:glycolide 75:25 avec un PM moy. de 6000 à 10 000 g/mol ;
poly(D,L-lactide-co-glycolide) terminé par un ester, lactide:glycolide 75:25 ;
poly(D,L-lactide-co-glycolide) terminé par un ester, lactide:glycolide 75:25 avec un PM moy. de 4000 à 15 000 g/mol ;
poly(D,L-lactide-co-glycolide) terminé par un acide, lactide:glycolide 75:25 avec un PM moy. de 4000 à 15 000 g/mol ;
poly(D, L-lactide-co-glycolide) terminé par un ester, lactide: glycolide 75:25 avec un PM moy. de 66 000 à 107 000 g/mol ;
poly(D,L-lactide-co-glycolide) terminé par un acide (RG 753 H) avec un PM moy. de 66 000 à 107 000 g/mol ;
poly(D,L-lactide-co-glycolide) terminé par un ester (RG 755 S) ;
poly(D,L-lactide-co-glycolide) terminé par un ester, lactide:glycolide 75:25 avec un PM moy. de 76 000 à 115 000 g/mol ;
poly(D,L-lactide-co-glycolide) terminé par un ester, lactide: glycolide 75:25 (RG 757 S) ;
poly(D,L-lactide-co-glycolide) terminé par un ester avec un PM moy. de 50 000 à 75 000 g/mol ;
poly(D,L-lactide-co-glycolide)-COOH, lactide:glycolide 85:15 avec un PM moy. de 17 000 g/mol ;
poly(D,L-lactide-co-glycolide) terminé par un ester, lactide:glycolide 80:20 avec un PM moy. de 200 000 ;
poly(L-lactide-co-glycolide) terminé par un ester, lactide:glycolide 85:15 (LG 855 S) ;
poly(L-lactide-co-glycolide) terminé par un ester, lactide:glycolide 85:15 (LG 857 S) ;
poly(D,L-lactide-co-glycolide) terminé par un ester, lactide:glycolide 85:15 (RG 858 S) avec un PM moy.
de 190 000 à 240 000 g/mol ;
poly(D,L-lactide), groupe terminal acide (R 202 H) ;
poly(D,L-lactide), groupe terminal ester (R 202 S) ;
poly(D,L-lactide), groupe terminal acide (R 203 H) ;
poly(D,L-lactide), groupe terminal ester (R 203 S) ; et
poly(D,L-lactide), groupe terminal ester (R 205 H).

9. Implant selon l'une quelconque des revendications 1 à 8, dans lequel
(i) l'implant comprend d'environ 2 à environ 35 % p/p du composé ; et/ou
(ii) l'implant comprend d'environ 2 à environ 25 % p/p du composé, et la matrice polymère biodégradable comprend une combinaison de deux polymères polylactides différents, préférablement dans lequel la combinaison de deux polymères polylactides différents comprend deux PLGA différents, deux PLA différents, ou un PLGA et un PLA ; et/ou
(iii) la matrice comprend un mélange de polymères biodégradables, et au moins un des polymères biodégradables est un polylactide présentant un poids moléculaire d'environ 6 kDa à environ 120 kDa ; et/ou
(iv) dans lequel la matrice comprend un deuxième polymère d'un polylactide présentant un poids moléculaire d'environ 6 kDa à environ 82 kDa.

10. Implant selon l'une quelconque des revendications 1 à 9, dans lequel l'implant comprend d'environ 1 µg à environ 75 µg, d'environ 5 µg à environ 50 µg ou environ 5 µg, environ 6 µg, environ 7 µg, environ 8 µg, environ 9 µg, environ 10 µg, environ 11 µg, environ 12 µg, environ 13 µg, environ 14 µg, environ 15 µg, environ 16 µg, environ 17 µg, environ 18 µg, environ 19 µg, environ 20 µg, environ 21 µg, environ 22 µg, environ 23 µg, environ 24 µg, environ 25 µg, environ 26 µg, environ 27 µg, environ 28 µg, environ 29 µg, environ 30 µg, environ 31 µg, environ 32 µg, environ 33 µg, environ 34 µg, environ 35, environ 36 µg, environ 37 µg, environ 38 µg, environ 39 µg, ou environ 40 µg du composé ou un sel pharmaceutiquement acceptable de celui-ci.

11. Implant selon l'une quelconque des revendications 1 à 10, dans lequel l'implant est configuré pour être placé dans le vitré de l'œil ou dans d'autres segments postérieurs de l'œil, facultativement dans lequel
(i) le composé est libéré à un rythme efficace pour assurer une libération du composé pendant plus d'une semaine à partir du moment où l'implant est placé dans le vitré de l'œil ;
(ii) le composé est libéré à un rythme efficace pour assurer une libération du composé pendant plus d'un mois à partir du moment où l'implant est placé dans le vitré de l'œil ou dans d'autres segments postérieurs de l'œil ; et/ou
dans lequel, une fois placé dans le vitré de l'œil ou dans d'autres segments postérieurs de l'œil, l'implant maintient une concentration d'au moins 50 nM du composé dans le tissu rétinien, ou dans lequel, une fois placé dans le vitré de l'œil ou dans d'autres segments postérieurs de l'œil, l'implant maintient une concentration de 50 nM à 125 nM du composé dans le tissu rétinien une fois qu'un état d'équilibre stable a été atteint.

12. Implant selon la revendication 11, dans lequel, une fois placé dans le vitré de l'œil ou dans d'autres segments postérieurs de l'œil, l'implant est configuré pour libérer d'environ 0,1 µg par jour à environ 2 µg par jour du composé ou d'un sel pharmaceutiquement acceptable de celui-ci.

13. Implant selon les revendications 11 ou 12, dans lequel, une fois placé dans le vitré de l'œil ou dans d'autres segments postérieurs de l'œil, l'implant est configuré pour fournir une libération initiale en rafale du composé ou d'un sel pharmaceutiquement acceptable de celui-ci, suivie d'une concentration à l'état d'équilibre inférieure du composé ou d'un sel pharmaceutiquement acceptable de celui-ci, préférablement dans lequel la libération initiale en rafale fournit une concentration d'environ 10 à 50 ng/mL du composé ou d'un sel pharmaceutiquement acceptable de celui-ci dans le compartiment oculaire, et facultativement dans lequel
(i) la concentration à l'état d'équilibre inférieure du composé ou d'un sel pharmaceutiquement acceptable de celui-ci est d'environ 50 nM à environ 125 nM à l'état d'équilibre ; et/ou
(ii) dans lequel la libération initiale en rafale fournit une dose d'environ 150 à environ 300 ng du composé ou d'un sel pharmaceutiquement acceptable de celui-ci sur deux jours à partir du moment où l'implant est placé dans le vitré de l'œil.

14. Implant selon l'une quelconque des revendications 11 à 13, dans lequel, une fois placé dans le vitré de l'œil ou d'autres segments postérieurs de l'œil, l'implant est configuré pour libérer le composé ou un sel pharmaceutiquement acceptable de celui-ci pendant une période d'au moins un mois à trois mois.

15. Implant selon l'une quelconque des revendications 1 à 14, dans lequel
l'implant comprend entre environ 11 % et 29 % p/p du composé ou d'un sel pharmaceutiquement acceptable de celui-ci, entre environ 0 % et environ 7,5 % p/p d'un polymère PEG, entre environ 15 % et environ 25 % p/p de R203S ou d'un polymère sensiblement similaire, entre environ 10 % et environ 20 % p/p de R202H ou d'un polymère sensiblement similaire, et entre environ 35 % et environ 45 % de RG752S ou d'un polymère sensiblement similaire ; et/ou
dans lequel l'implant comprend entre environ 11 % et 29 % p/p du composé ou d'un sel pharmaceutiquement acceptable de celui-ci, entre environ 0 % et environ 7,5 % p/p d'un polymère PEG, entre environ 25 % et environ 35 % p/p de R202S ou d'un polymère sensiblement similaire, et entre environ 45 % et environ 55 % de RG752S ou d'un polymère sensiblement similaire.
